# EUROPEAN PATENT SPECIFICATION

(11) **EP 0 137 280 B1**
(45) Date of publication and mention of the grant of the patent: **11.03.1992**
(21) Application number: 84110305.4
(22) Date of filing: 29.08.1984
(51) Int. Cl.: C12N 15/56, C12N 9/42, C12R 1/885

(54) **Recombinant fungal cellobiohydrolases**
Rekombinante Cellobiohydrolasen aus Pilzen
Cellobiohydrolases recombinantes fongueuses

(30) Priority: 31.08.1983 US 528216; 16.07.1984 US 630974
(43) Date of publication of application: 17.04.1985
(73) Proprietor: CETUS ONCOLOGY CORPORATION, Emeryville California 94608 (US)
(72) Inventor: Shoemaker, Sharon Payne, Fairfield California 94533 (US); Gelfand, David Harrow, Oakland California 94608 (US); Innis, Michael Alan, Oakland California 94602 (US); Kwok, Shirley Yee, San Ramon California 94583 (US); Ladner, Martha Baillie, Richmond California 94804 (US); Schweickart, Vicky, Albany California 94706 (US)
(74) Representative: VOSSIUS & PARTNER

(56) References cited:
- EP-A- 0 011 767
- ABSTRACTS OF THE ANNUAL MEETING OF THE AMERICAN SOCIETY FOR MICROBIOLOGY, 1981, 81st Annual Meeting, March 1-6, 1981 (Dallas, Texas) S.K. PICA TAGGIO et al. "The Cloning of Trichoderma, reesei Genomic DNA in Escherichia coli HB 101" page 116, column 2, Abstract H 19
- GENE, vol. 17, no. 1, January 1982 (Amsterdam) D.J. WHITTLE et al. "Molecular cloning of Cellulomonas fimi cellulasegene in Escherichia coli" pages 139-145
- Proc. Bioenergy RRD 135-39 (1981)
- L.G. Fägerstram, Doctoral Thesis University of Uppsala, Sweden, (1981)

## Description

### Technical Field

The present invention relates to the field of cellulose degradation and modification and, more specifically, to the particular enzymes useful therein. Specifically, the invention relates to the cloning and expression of the genes encoding the various enzymes involved in cellulolysis, which include endoglucanases and cellobiohydrolases derived from cellulolytic fungi.

### Background Art

The world's most abundant renewable resource is cellulose, a polymer of glucose, which is a major structural component of wood and herbaceous materials. As a result, cellulose is a significant part of so- called waste products, such as municipal solid waste, used paper products, and by-products of agricultural crops. It would be advantageous to harvest the glucose from these sources in an efficient manner, either for use directly as food, or as a starting material for other useful materials such as ethanol. Limited techniques are available for converting the cellulose starting material to a glucose end product. Various non-enzymatic processes such as acid hydrolysis, have been attempted, but these lack sufficient specificity and purity of product to be economically competitive.

An important alternative is the use of cellulolytic enzymes derived from natural sources, or direct use of the microorganisms which harbor these enzymes. Because the level of enzymatic activity available is controlled by factors which are not readily manipulatable, these methods have also suffered from economic drawbacks.

Accordingly, there is an interest in developing improved strains of cellulase-producing microorganisms, and in introducing cellulase genes in recombinant form into microbial organisms which otherwise are incapable of utilizing cellulose as a carbon source. In particular, it would be desirable economically to introduce cellulolytic enzyme genes into fermentation organisms, such as fermentation yeasts, which may not naturally have the capacity to hydrolyze cellulose to utilizable substrates such as glucose, but which may have superior growth and production properties.

The typical cellulase system contains three major types of enzymes which act cooperatively in degrading cellulose (Gritzal, M., et al., Hydrolysis of Cellulose: Mech. of Enzymatic and Acid Catalyses, Brown, R. D., et al., ed. (1979) Am. Chem. Soc., Washington, D.C., p. 237; Mandels M., et al., Process Biochem. (May, 1978), p. 7). These enzyme types are cellobiohydrolases, endoglucanases, and glucosidases.

Cellobiohydrolases (CBH's), attack cellulose from the non-reducing ends of the cellulose polymers and yield cellobiose (a glucose dimer) as a major product. Two types of CBH enzymes are known: cellobiohydrolase-I (CBHI) which generates cellobiose exclusively, and cellobiohydrolase-II (CBHII) which generates a mixture of cellobiose and glucose. CBH enzymes have not been found in cellulolytic, non-filamentous bacteria, and thus appear to be limited to fungal and filamentous bacterial sources. They are highly specific with respect to substrate, and have high substrate affinity. (Derivatized forms of cellulose are not cleaved extensively by CBH).

Endoglucanases (EG's), hydrolyze internal glycosidic linkages within the cellulose chain. They are found in bacteria as well as fungi, and have a broader substrate specificity than CBH enzymes. They attack {3-glycosidic linkages in a semi-random manner, and will utilize as substrates derivatized cellulose (such as carboxymethyl cellulose) and will hydrolyze 1,3, β,D- and 1,6-β,D- as well as 1,4,β,D- linkages. There are several known forms of EG enzymes which have been derived from bacteria and fungi. Endoglucanase-I (EGI) is produced in relatively high levels (5-10% of extracellular protein) in the fungus Trichoderma reesei; a second EG activity in this fungus, endoglucanase-II (EGII), has been shown to atta̅c̅k̅ l̅i̅n̅e̅a̅r̅ 1̅,̅4̅-̅β̅,̅D̅-mannan to generate primarily mannobiose.

{3-glucosidases, like the CBH's attack the non-reducing end of glycosides, but act only on solubilized cello-oligosaccharides (chain length less than 6), to generate glucose units. The {3-glucosidases thus degrade the cellobiose product of CBH's and EG's to glucose. The presence of {3-glucosidases in a total cellulase system is significant not only because it provides the final end product, but because some CBH's and EG's are inhibited by cellobiose. Providing an additional enzyme to deplete the concentration of this product thus aids conversion of cellulose to glucose.

The above distinct reaction specificities of the cellulase system components suggest that the enzymes can be used individually or in selected combinations to achieve controlled and limited hydrolysis of cellulosic materials. The purpose of limited hydrolysis would be to alter the structural and/or functional characteristics of cellulosic materials in desired ways. Exemplary structural changes include those affecting chain length, viscosity and cellulose binding. Exemplary functional charges include those affecting texture, flavor, and water binding to the material. In addition, the endoglucanases, because of their broader substrate specificity, act on other linked polymers. One example of this is the action of EGI on xylan. Another example is the action of EGII on linear 1,4-{3,D-mannan. In experiments conducted in support of the present invention, it was shown that EGII converts {3-mannan to generate primarily mannobiose. This may be significant to the coffee industry, as (3-mannan is a major polysaccharide coating the outside of coffee beans, and inhibits the extraction of flavor components and imparts a bitter flavor in the manufacture of coffee, particularly freeze-dried coffee.

The enzymes of the cellulase system have been widely studied and, indeed, attempts have been made to clone and express the coding sequences. See, for example, Whittle, D. J., et al., Gene (1982) 17:139; Cornet, P., et al., FEBS Micro. Biol. Letters (1983) 16:137; Tuse, D., Genetic Technol. News (1981) November, p. 6; Abstract No. H19 ASM Annual Meeting, March 1981; Symposium ASM Annual Meeting, March 1983; Wilson, D. B., et al., Bio/Technology (1983) 1:594; Teeri, T., et al., Bio/Technology (1983) 1:696; and Collmer, A., et al., Bio/Technology (1983) 1:594-601. None of the foregoing attempts, although using various bacterial and fungal sources, including T.reesei, as the source for the coding sequences, have reported the expression of secreted, glycosylated and biologically active fungal cellulases in yeast. Indeed, the only cellulase genes reported above to be expressed in a heterologous host are those encoding bacterial, not fungal, endoglucanase.

### Disclosure of the Invention

This invention provides recombinant forms of the cellulase enzymes CBHI and EGI which are related to those derived from fungal sources. The coding sequences from the various cellulase enzymes of fungal sources are retrievable in uninterrupted form by the general procedures disclosed herein. By introducing such sequences in suitable expression vectors, and introducing the recombinant expression vectors obtained into suitable host organisms, large or increased amounts of these enzymes can be produced either individually or in combination. Production of large amounts of such enzymes permits industrial application of their activity to hydrolyze cellulose by carrying out the reaction in vitro using these enzymes either in solubilized or in immobilized form. The enzymes thus produced are advantageously secreted into the medium.

According to one aspect of the invention, the genes encoding the cellulose enzymes CBHI and EGI are isolated from a suitable fungal source, such as T. reesei, and their sequences determined. Using these sequences and the information they provide, it is possible to construct intron-free coding sequences which encode the mature enzyme in reading frame with the leader sequences which are responsible for secretion from the native host. The coding sequence is placed in a novel expression vector which allows gene expression of the heterologous sequence in foreign hosts such as yeast. The host which is transformed with the expression vector is capable of producing and secreting the cellulase in mature, glycosylated, functionally active form.

According to another feature of the invention, the recombinant vectors used in transforming host organisms contain cellulase signal sequences which are shown to function in yeast for the efficient processing and secretion of cellulases from yeast. These sequences could also be used for the secretion of other proteins from yeast, preferably for the secretion of proteins that are normally secreted by their native host. Examples of such proteins include amylases, proteases, interferons, lymphokines, insulin, and hormones.

In still another aspect of the invention, an organism which produces a number of different cellulase enzymes in defined ratios may be manipulated to inactivate or modify one or more of the cellulase enzymes either to enhance the enzyme in a desired fashion or to prevent production of the enzyme. For example, in a fungal organism which produces both CBHI and CBHII enzymes, the CBHII gene may be inactivated to yield an organism which produces only the CBHI cellobiohydrolase. The method for inactivating a selected cellulase gene generally includes inactivating (e.g., by a large-segment deletion) the recombinant cellulase gene constructed according to the invention, and transforming a cellulase-producing organism with the inactivated gene. Recombination between the inactivated and genomic gene leads to inactivation of the selected genomic gene. The feasibility of this approach applied to filamentous fungal organisms has been shown in Yelton, M. M., et al., Proc. Nat. Acad. Sci., USA, (1984) 80:1470.

These and other objects and aspects of the invention will become more apparent when the following detailed description is read in conjuction with the accompanying drawings.

### Brief Description of the Drawings

Figure 1 shows gel electrophoretic patterns of poly A RNA isolated from T. reesei grown either in the presence (lane 1) or absence (lane 2) of a cellulase-inducing carbon source.

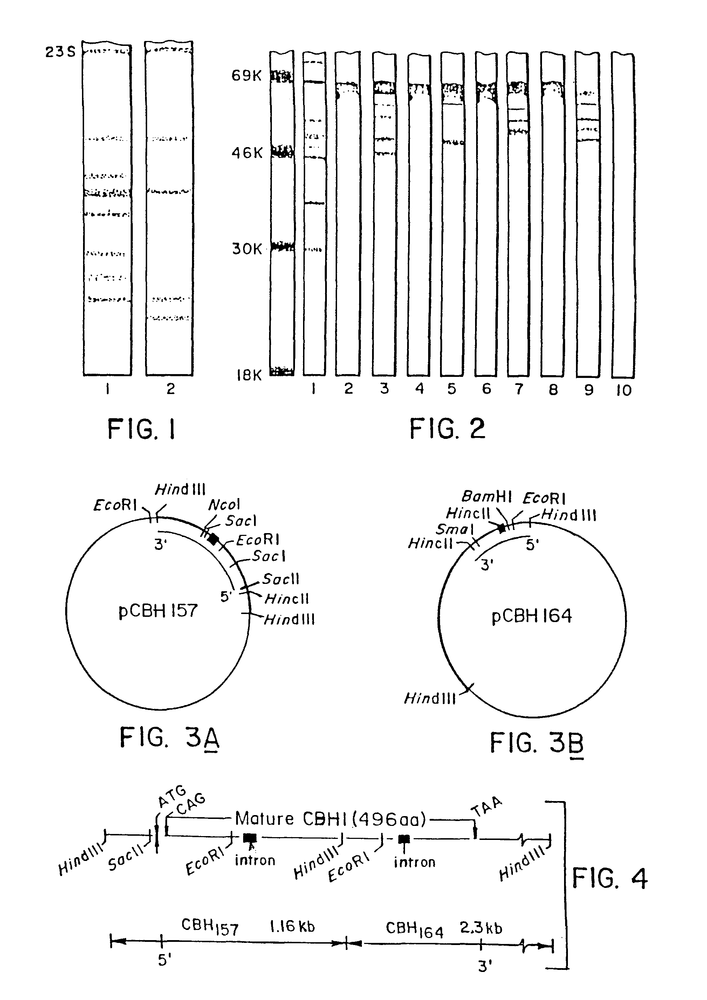

Figure 2 shows gel electrophoretic patterns of polypeptides produced in a cell-free protein synthesizing system supplied with: (a) total poly A RNA obtained from T. reesei grown under cellulase-induction conditions (lanes 1 and 2); (b) three different size fractions of the total induced poly A RNA (lanes 3-8); or (c) a size fraction of poly A RNA obtained from T. reesei cells grown under cellulase non-induction conditions (lanes 9 and 10), where the odd-numbered gel patterns show total polypeptide synthesized under the direction of the various RNA samples, and the associated even-numbered gels show the corresponding peptides which immunoprecipitate in the presence of anti-CBHI antibody.

Figure 3A is a map of a vector designated pCBH157 which contains a 1.16 kilobase Hindlll fragment from T. reesei strain L27 genomic DNA.
Figure 3B is a map of a vector designated pCBH164 containing a 2.3 kilobase Hindlll fragment adjacent the above 1.16 kilobase fragment from the same genomic DNA.
Figure 4 is a map of the two adjacent Hindlll genomic digest fragments from T. reesei strain L27 which, together, contain the CBHI gene.
Figure 5 is a map of an E. coli/S. cerevisiae shuttle vector used in one embodiment of the invention.
Figure 6 illustrates steps in the construction of an intron-free 5'-end portion of a CBHI gene, according to one embodiment of the invention.
Figure 7 illustrates steps in the construction of a 3'-end portion of an intron-free CBHI gene, according to the embodiment of the invention.
Figure 8 illustrates the construction of a vector designated ptrpCBH5 from pDG151 (Figure 5) and pCBH5 (Figure 6).
Figure 9 illustrates the construction, from ptrpCBH5 (Figure 8) and pCBH3 (Figure 7), of a vector designated ptrpCBH8 which contains a full length, intron-free CBHI gene.
Figure 10 illustrates an alternative construction, from vectors ptrpCBH5 and pCBH3, of a vector designated ptrpCBH81 containing a full-length, intron-free CBHI gene.
Figure 11 shows the construction of ptrpCBH82 a vector suitable for expression of CBHI in E. coli.
Figure 12 shows the construction of peno500.202 a vector suitable for expression of CBHI in yeast.
Figure 13 shows the results of Western blot analysis of native and recombinant CBHI.
Figure 14 illustrates in frame A, a restriction map of the EGI-encoding portion of pUC8-h; in frames B-D, the series of steps resulting in the construction of an intron-free coding sequence for EGI; and in frames E and F, the steps in providing Hindlll sites immediately upstream and downstream of the EGI coding sequence.
Figure 15 shows the results of a hybridization experiment testing the ability of a 4 kb Hindlll fragment from T. reesei to isolate mRNA capable of directing EGI synthesis.
Figure 16 shows the ability of colonies transformed with pJV160 to hydrolyze CMC.
Figure 17 is an autoradiogram of Western blots performed on culture supernatants from yeast transformed with pJV160 and with a control vector.

### Modes of Carrying Out the Invention

### A. Definitions

"Cellobiohydrolase" or "exo-cellobiohydrolase" or "CBH", as the terms are used herein, refers to a protein capable of cleaving cellulose into cellobiose units from the non-reducing ends of cellulose polymer chains. CBH enzymes are known to occur in filamentous bacterial and fungal sources, but have yet to be demonstrated in non-filamentous bacteria. At least two forms of CBH are known in Trichoderma -- CBHI which generates only cellobiose residues, and CBHII which generates a mixture of cellibiose and glucose.

The CBHI protein illustrated herein has a sequence of 496 amino acids and is preceded by a 17 amino acid leader sequence. The mature CBHI, produced by removal of the leader sequence, has an approximate molecular weight of 66,000 daltons. When produced in native form the protein shows some degree of glycosylation predominantly through linkage to the OH of serine and/or threonine (Gum, E.K., et al., Biochem. Biophys. Acta (1976) 446:371

"Endoglucanase" refers to an enzyme which is capable of cleaving cellulose at internal glycosidic sites. A number of forms of endoglucanase are known including both EGI and EGII from fungal sources, such as T. reesei and other suitable fungal sources.

The EGI illustrated herein has a sequence of 437 amino acids in what is considered to be the mature protein, and is preceded by a 22 amino acid leader sequence. The secreted form of the protein, based on its 437 amino acid composition, has a molecular weight of about 46,000 daltons. The native protein is known to contain some degree of glycosylation when produced by the native host. (Shoemaker, S.P., et al., Biochem. Biophys. Acta (1978) 523:147).

"{3-glucosidases" are similarly defined. These enzymes comprise part of the cellulase system of fungi, as well as of bacterial systems. They are known to exist in at least two physical forms, Q-glucosidase-I, and {3-glucosidase-1I in T. reesei. These enzymes complete the cellulose digestion by generating glucose units from soluble oligosaccharide.

The following applies to all three types of the cellulases defined above:
It is clear that all proteins are potentially either acidic or basic salts according to the pH at which they are prepared. All ionization states of the protein are included in the definition. Further, it is understood that certain modifications are made to the molecule by virtue of post-translational processing, such as glycosylation, phosphorylation, acetylation, or the like, which are dependent on the nature of the host system. Included in the definitions of the cellulases are both processed and unprocessed forms of the polypeptides. Similarly, modifications to the structure may occur through inadvertent or intentional chemical modification of amino acid side chains such as, for example, oxidation of sulfhydryl groups. Proteins so modified are further included in the definition so long as these modifications do not destroy enzyme activity. Finally, it is expected that minor alterations in the precise amino acid sequences may be made without destroying the activity of the enzymes. Such modifications may include amino acid substitutions as well as additions or deletions of one or a few amino acids in the sequence, and these modifications, too are included in the definition, provided enzyme activity is retained.

In particular, two cellulase enzymes have "substantially equivalent" amino acid sequences when an approximate one-to-one correspondence between the sequences exists; however, minor changes in one or several amino acids which do not result in loss of its CBH or EG functionality are considered to generate peptides remaining within this definition.

A particular cellulase "derived from" a particular source refers to amino acid sequences which are produced natively by that source.

In the illustrations below, the entire genomic sequences associated with EGI or CBHI derived from T. reesei L27 are used as the coding sequences. These coding sequences contain the pre-sequences encoding the leader peptides. In these illustrations, the pre-sequences are retained.

The target gene will be referred to as the EGI or CBHI gene in the illustrations below. The DNA sequences of the invention are those which encode active protein, regardless of any additional (or deleted) nucleotides beyond those of the mature form.

"Treatment" of a suitable cellulase substrate, such as cellulose, encompasses hydrolytic enzyme reactions designed either to produce selected substrate modifications, or to effect hydrolytic breakdown of the substrate, including complete hydrolysis of cellulose to glucose, or limited, controlled hydrolysis/modification.

"Control sequences" refer to DNA sequences which regulate the expression of a particular coding sequence in a particular host. Depending on the nature of the host these may include promoters, operators, ribosome binding sites, terminators, enhancers, and whatever other sequences are necessary to effect some level of expression. "Suitable" control sequences refer to those sequences which are compatible with the particular host concerned.

The term "leader sequence" or "signal sequence" is defined herein as a sequence of amino acids which is responsible for initiating export of a protein chain. A leader sequence, once having initiated export of a growing protein chain, is cleaved from the mature protein at a specific site. The term also includes leader peptides. One preferred leader sequence herein is the deduced leader sequence for CBHI derived from T. reesei indicated in Table I. Another is that for EGI from T. reesei shown in Table III.

"Operably linked" refers to juxtaposition of gene elements and for control sequences in such a manner that the activity of the components of the system is retained. Specifically, "coding sequences operably linked to control sequences" refers to a configuration wherein the coding sequences are expressed in the appropriate host. The control sequences provided may be maximized as to their efficiency, but the phrase "operably linked to suitable control sequences" simply implies that sufficient control sequence efficacy is available to provide some useful level of expression.

"Cell", "cell line", "cell culture", and "host cell" are used interchangeably and meant to denote the most general case unless otherwise clear from the context.

A vector which is a "derivative" of another vector means that the parent vector was employed in the series of steps leading to the synthesis of the "derivative" vector.

### B. General Description

### B.1. Production of Recombinant Cellulases

In general terms, the production of a recombinant form of cellulase enzyme in the approach illustrated below involves the following steps:
1) obtaining a coding sequence uninterrupted by introns for the protein preceded by a leader sequence in excisable or otherwise recoverable form;
2) placing the cellulase coding sequence, either before or after removal of intron(s) according to step 1, in operable linkage with suitable control sequences of a replicable expression vector;
3) transforming a suitable host with the thus formed expression vector;
4) culturing the transformed host under favorable conditions to effect the production of the recombinant cellulase enzyme; and,
5) optionally recovering the cellulase from the medium or from the cells. Recovery of the enzyme may, in fact, not be necessary in order to utilize its activity for cellulose degradation. Indeed, in one embodiment of the method of the invention, which concerns use of the recombinant cellulase for cellulose degradation, cellulose may be added to the cultured cells in situ, and the growing or resting cell culture used to effect cellulose degradation directly.

Each of the foregoing steps can be done in a variety of ways. For example, the desired coding sequences can be obtained by preparing suitable cDNA from cellular messenger and manipulating the cDNA to obtain the complete sequence. Alternatively, genomic fragments may be manipulated, according to a number of procedures such as those described below, to remove identified intron regions. If a portion of a sequence is sufficiently short to come within the purview of known chemical synthetic techniques, such sequence can be prepared from the individual nucleotide starting materials through chemical synthesis. Indeed, the length of sequence obtainable by this method is entirely dependent on the current status of the oligonucleotide synthesis art.

A particularly useful way to retrieve the coding sequences corresponding to the cellulase enzymes from a genomic library is illustrated herein. The genomic library, prepared in phage from the nuclear DNA of a high producer for the cellulase complex, such as induced T. reesei L27, contains coding sequences for all the desired enzymes. For each enzyme desired, a purified native form is used to obtain antibodies. The antibodies are used to identify an enriched mRNA fraction capable of synthesizing the appropriately immunoprecipitating protein in an in vitro translation system. The identified mRNA is used to generate a cDNA probe which, in turn, is used to identify the desired gene or gene portions from the library. Further modifications in the probe-identified gene may be made. For convenience, suitable restriction sites can, if not natively available, be added to the ends of the coding sequence so as to provide an excisable gene segment which can then be inserted adjacent suitable control sequences in an expression vector. Alternatively, control sequences may be introduced into vectors already containing the gene or portions thereof. The control sequences, expression vectors, and transformation methods are dependent on the type of host cell used to express the gene. Generally, procaryotic or yeast cells are presently useful as hosts. The appropriate control sequences and techniques will be dependent on the choice of host as is outlined below.

### B.1.a. Control Sequences

Procaryotes most frequently are represented by various strains of E. coli. However, other microbial strains may also be used, such as bacilli, for example Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid derived from an E. coli species by Bolivar, et al., Gene (1977) 2:95. pBR322 contains genes for ampicillin and tetracycline resistance, and these markers can be either retained or destroyed in constructing the desired vector. Commonly used procaryotic control sequences which are defined herein to include promoters for transcription initiation, optionally with an operator, along with ribosome binding site sequences, include such commonly used promoters as the beta- lactamase (penicillinase) and lactose (lac) promoter systems (Chang, et al., Nature (1977) 198:1056 and the tryptophan (trp) promoter system (Goeddel, et al., Nucleic Acids Res. (1980) 8:4057. The lambda-derived P_{L} promoter and N-gene ribosome binding site (Shimatake, et al., Nature (1981) 292:128), which has been made useful as a portable control cassette, as set forth in copending Application Serial No. 578,133, filed February 8, 1984, and assigned to the same assignee, is another example. However, any available promoter system compatible with procaryotes can be used.

In addition to bacteria, eucaryotic microbes, such as yeast, may also be used as hosts. Laboratory strains of Saccharomyces cerevisiae, Baker's yeast, are most used, although a number of other strains are commonly available. While vectors employing the 2 micron origin of replication are illustrated, (Broach, J. R., Meth. Enz. (1983) 101:307), other plasmid vectors suitable for yeast expression are known (see, for example, Stinchcomb, et al., Nature (1979) 282:39, Tschempe, et al., Gene (1980) 10:157 and Clarke, L., et al., Meth. Enz. (1983) 101:300). Control sequences for yeast vectors include promoters for the synthesis of glycolytic enzymes (Hess, et al., J. Adv. Enzyme Reg. (1968) 7:149; Holland, et al., Biochemistry (1978) 17:4900). Additional promoters known in the art include the promoter for 3-phosphoglycerate kinase (Hitzeman, et al., J. Biol. Chem. (1980) 255:2073), and those for other glycolytic enzymes, such as glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, ibid).

Evidence suggests that terminator sequences are desirable at the 3' end of the coding sequences. Such terminators are found in the 3' untranslated region following the coding sequences in yeast-derived genes. Many of the vectors illustrated contain control sequences derived from the enolase-I gene containing plasmid peno46 (Holland, M. J., et al., J. Biol. Chem. (1981) 256:1385) or the LEU2 gene obtained from YEpl3 (Broach, J., et al., Gene (1979) 8:121), however any vector containing a yeast compatible promoter, origin of replication and other control sequences is suitable.

It is also, of course, possible to express genes encoding polypeptides in eucaryotic host cell cultures derived from multicellular organisms. See, for example, Tissue Cultures, Academic Press, Cruz and Patterson, editors (1973). Useful host cell lines include VERO and HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) Fiers, et al., Nature (1978) 273:113), or other viral promoters such as those derived from polyoma, Adenovirus 2, bovine papiloma virus, or avian sarcoma viruses. General aspects of mammalian cell host system transformations have been described by Axel in U.S. Patent No. 4,399,216 issued August 16, 1983. It now appears also that "enhancer" regions are important in optimizing expression; these are, generally, sequences found upstream of the promoter region. Origins of replication may be obtained, if needed, from viral sources. However, gene integration into the chromosome is a common mechanism for DNA replication in eucaryotes, and hence independently replicating vectors are not required. Plant cells are also now available as hosts, and control sequences compatible with plant cells such as the nopaline synthase promoter and polyadenylation signal sequences (Depicker, A., et al., J. Mol. Appl. Gen. (1982) 1:561) are available.

### B.1.b. Transformations

Depending on the host cell used, transformation is done using standard techniques appropriate to such cells. The calcium treatment employing calcium chloride, as described by Cohen, S. N., Proc. Natl. Acad. Sci. (USA) (1972) 69:2110 is used for procaryotes or other cells which contain substantial cell wall barriers. Transformations into yeast were carried out according to the method of Van Solingen, P., et al., J. Bact. - (1977) 130:946 and Hsiao, C. L., et al., Proc. Natl. Acad. Sci. (USA) (1979) 76:3829. Briefly, yeast cultures grown to mid-log phase in YEPD rich medium (yeast extract, peptone and 4% glucose) were washed and protoplasted with zymolyase 5000 (Miles Laboratory) in sorbitol phosphate buffer. Protoplasts were washed, allowed to stand at room temperature for one hour in 67% YEPD containing 1 M sorbitol, then pelleted and suspended in Tris-sorbitol-calcium chloride buffer to 2 x 10⁹ protoplasts/ml. Protoplasts were mixed with 5-10 I.Lg of DNA for transformation in a 100 u.1 reaction mix, then 1 ml of 44% PEG was added and the mixture allowed to stand for 40 minutes at room temperature. Alternatively, the procedure of Klebe, et al., (Gene (1983) 25:333), can be used.

### B.1.c. Vector Construction

Construction of suitable vectors containing the desired coding and control sequences employs standard ligation and restriction techniques which are well understood in the art. Isolated plasmids, DNA sequences, or synthesized oligodeoxyribonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 u.1 of buffer solution; in the examples herein, typically, an excess of restriction enzyme is used to insure complete digestion of the DNA substrate. Incubation times of about one hour to two hours at about 37 ° C are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min. at 20 to 25°C in 50 mM Tris-CI, pH 7.6, 50 mM NaC1, 6 mM MgCl₂, 6 mM DTT and 5-10 µM dNTPs. The Klenow fragment fills in at 5' sticky ends but chews back protruding 3' single strands, even though the four dNTPs are present. If desired, selective repair can be performed by supplying only a selected one or more dNTPs within the limitations dictated by the nature of the sticky ends. After treatment with Klenow, the mixture is extracted with phenol/chloroform and ethanol precipitated followed by running over a Sephadex G-50 spin column. Treatment under appropriate conditions with S1 nuclease results in hydrolysis of any single-stranded portion.

Synthetic oligonucleotides may be prepared by the triester method of Matteucci, et al. (J. Am. Chem. Soc. (1981) 103:3185-3191), or the diethylphosphoramidite method of Caruthers, described in U.S. Patent No. 4,415,732, issued November 15, 1983.

Phosphorylation of single strands prior to annealing or for radio-labeling is achieved using an excess polynucleotide kinase, e.g., approximately 10 units of kinase to 1 nmole substrate in the presence of 50 mM Tris, pH 7.6, 10 mM MgCl₂, 5 mM dithiothreitol, 1-2 mM ATP, 1.7 pmoles y32P-ATP (2.9 mCi/mmole), 0.1 mM spermidine, and 0.1 mM EDTA.

Ligations are performed typically in 15-30 ti1 volumes under the following standard conditions and temperatures: 20 mM Tris-C1 pH 7.5, 10 mM MgCl₂, 10 mM DTT, 33 µg/m1 BSA, 10 mM-50 mM NaC1, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/m1 total DNA concentrations (5-100 nM total end concentration). Intermolecular blunt end ligations (usually employing a 10-30 fold molar excess of linkers) are performed typically at about 1 µM total ends concentration.

In vector constructions employing "vector fragments", the vector fragment is commonly treated with bacterial alkaline phosphatase (BAP) in order to remove the 5' phosphate and prevent religation of the vector. BAP digestions are conducted typically at about pH 8, in approximately 150 mM Tris, in the presence of Na and Mg +2, using about 1 unit of BAP per µg of vector at 60 for about one hour. In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

### B.1.d. Verification of Construction

In the constructions set forth below, correct ligations for plasmid construction are confirmed by first transforming E. coli strain MM294 obtained from E. coli Genetic Stock Center, CGSC #6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al., Proc. Natl. Acad. Sci. (USA) (1969) 62:1159, optionally following chloramphenicol amplification (Clewell, D. B., J. Bacteriol. (1972) 110:667). The isolated DNA is analyzed by restriction and/or sequenced by the dideoxy method of Sanger, F. et al., Proc. Natl. Acad. Sci. (USA) (1977) 74:5463 as further described by Messing, et al., Nucleic Acids Res. (1981) 9:309, or by the method of Maxam, et al., Methods in Enzymology (1980) 65:499.

### B.1.e. Site Specific Mutagenesis

Oligonucleotide induced mutagenesis is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage- supporting host bacterium. Cultures of the transformed bacteria are plated in top agar, permitting plaque formation from single cells which harbor the phage.

Theoretically, 50% of the new colonies will contain the phage having, as a single strand, the mutated form; 50% will have the original sequence. The resulting plaques are hybridized with phosphorylated synthetic primer at a temperature which permits hybridization of an exact match, but at which the mismatches with the original strand are sufficient to prevent hybridization. Plaques which hybridize with the probe are then picked, cultured, and the DNA is recovered. Details of site specific mutation procedures are described below in specific examples.

### B.1.f. Hosts Exemplified

Host strains used in cloning and expression herein are as follows:
For cloning and sequencing, and for expression of construction under control of most bacterial promoters, E. coli strain MM294 (supra), Talmadge, K., et al., Gene (1980) 12:235; Meselson, M., et al., Nature (1968) 217:1110, was used as the host.

For M13 phage recombinants, E. coli strains susceptible to phage infection, such as E. coli K12 strain DG98 are employed. The DG98 strain has been deposited with ATTC and has accession number 39768 .

Expression in yeast employed laboratory strains of S. cerevisiae: C468 (a, his4-, leu2-, ma1-) strain obtainable from ATCC, Deposit 20,690, and S173-6B (trpl-, leu2-, ura3-, his4-) strain, obtainable from Prof. Fred Sherman, U. of Rochester, N.Y.

### B.2. Utility of the Recombinant Enzyme

Recombinant cellobiohydrolases and endoglucanases can, of course, be employed for approximately the same utilities, and under the same conditions as are appropriate for the native forms of the corresponding enzyme. The recombinant forms have the advantage of being obtainable singly or in controlled combination in relatively large quantity, and of being subject to suitable modification to enhance their properties by genetic manipulation of the coding sequences or by selecting hosts which have optimal post-translational processing features The recombinant enzymes, when produced in a novel host, confer in the host the ability to utilize and/or modify novel substrates.

Each of the recombinant cellulase enzymes can be employed in vitro, either in solution or in immobilized form, to carry out its role in the complete or controlled hydrolysis of cellulose, or such other modified substrates as is desired and compatible with the enzyme in question. The specific conditions of temperature, pH, ionic strength, and time of reaction will be variable with the precise enzymes employed.

The enzymes of the invention can be used either alone or in combination with each other or with other suitable catalysts. For efficient hydrolysis of an insoluble cellulose substrate, it is clearly advantageous to use a combination of at least one endoglucanase, cellobiohydrolyase, and {3-glucosidase. Again, these can be supplied in soluble or immobilized form, or can be provided by the same or different transformant host. For example, it is within the scope of the invention to transform a yeast host with expression vectors which contain each of the enzymes of the cellulase complex, or with a single expression vector which contains expression modules for each of these enzymes. It is currently known that some species of yeast endogenously produce (3-glucosidase and other strains may be found which endogenously produce other enzymes of the cellulase found which endogenously produce other enzymes of the cellulase complex. For such host strains it would be necessary only to supply the missing member(s) of the cellulase complex in the form of suitable expression vector(s).

Further, the utility of the recombinant enzymes of the invention is not limited to the production of glucose from cellulose. The CBH's are valuable research tools because of their high specificity for 1,4-;8,D-glucan linkages, and thus provide a means for ascertaining the presence or absence of such linkages in preparation of polysaccharides. Indeed, under some circumstances it might be desirable to obtain a pure preparation of cellibiose specifically, and CBHI would be singularly useful for this purpose. A method for engineering a cellulase-producing organism to produce only CBHI cellobiohydrolase has been outlined above. Finally, industrial yeast strains may be transformed with one or more of the components of the cellulase complex simply by providing the vector with a suitable dominant selectable marker. See, The Molecular Biology of Yeast, Gold Spring Harbor Meeting, August 16-21, 1983.

### B.3 Recombinant-Gene Yeast Vectors

As will be seen below, recombinant vectors constructed according to the present invention provide efficient production in yeast of secreted, glycosylated, biologically active cellulases. The novel constructional features of the vector which are important in yeast to production of heterologous recombinant cellulases include: ' (1) a yeast promoter sequence; (2) a cellulase enzyme leader sequence linked to the promoter; and (3) the coding region of the mature cellulase to be produced. Additionally, the exemplary recombinant vectors contain a yeast terminator sequence at the 3'-end of the cellulase coding region.

According to one aspect of the invention, the yeast vectors containing cellulase genes may be modified, by conventional recombinant techniques, to include: (1) the above yeast promoter; (2) the cellulase enzyme leader sequence; and (3) a suitable restriction site at the 3' end of the leader sequence or within the adjacent cellulase coding region at which a heterologous gene can be introduced. The heterologous gene may code for another cellulase, or for an unrelated protein, such as an amylase, protease, peptide hormone, or one of a large number of other peptides for which a heterologous gene, preferably in uninterrupted form, can be obtained.

Section D below illustrates an exemplary yeast vector designated peno CBH500.202, containing a yeast enolase promoter, a CBHI leader sequence, the coding sequence of mature CBHI from T. reesei, and the yeast enolase terminator. The vector can be modified according to known methods, to replace all or a portion of the CBHI coding sequence with a heterologous protein coding sequence. The resultant vector allows the efficient processing and secretion of the heterologous protein in yeast.

Section E below describes another exemplary yeast vector, designated pJV160, containing a yeast enolase promoter, an EGI leader sequence, the coding sequence of mature EGI from T. reesei, and the above enolase terminator sequence. Using analogous techniques, this vector can be modified to provide efficient processing and secretion of a heterologous-gene product in yeast.

### C. Cloning and Expression of EGI and CBHI from T. reesei Strain L27

Illustrated below in Sections D and E are embodiments of the present invention wherein the DNA sequences encoding the CBHI and EGI secreted from T. reesei strain L27, respectively, are cloned and expressed.

Briefly, and in summary, a preferred source of the two coding sequences is a strain of T. reesei, strain L27. This strain was selected as a high level producer of the cellulase complex and because portions of the EGI and CBHI sequences derived from T. reesei have been published (referenced below).

The strategy for obtaining the intronless coding sequences was as follows: poly-A RNA (mRNA) was isolated from the selected fungal source following cellulose induction, and size-fractionated using agarose gel electrophoresis. Gels containing mRNA fractions derived from induced cells, (which showed a number of bands absent or present only in relatively small amounts in uninduced cells) were sliced according to size, and each slice tested for the presence of the desired mRNA. Testing was conducted by adding eluted mRNA to an in vitro protein synthesis (translation) system, and analyzing the proteins synthesized by SDS-PAGE both by electrophoresis of total proteins and polypeptides which immunoprecipitate in the presence of anti-EGI or anti-CBHI antibodies. This procedure enabled identification in each case of an mRNA fraction enriched in the desired message.

The identified mRNA fraction was then used to obtain single stranded copy DNA (cDNA), which was radiolabelled and used as a probe of a genomic library. The cDNA was also used to obtain a cDNA library which included the fragment(s) containing coding sequences for the EGI or CBHI gene.

A genomic DNA library from the selected fungal strain was prepared in a X phage. The total nuclear DNA from the fungus was partially digested with one or more selected restriction enzymes, and inserted in the phage vector. This phage library contains coding sequences for all the enzymes of this fungal cellulase complex. The cloned fragments were probed with cDNA derived from the appropriate enriched mRNA fraction. Phages homologous to the CBHI or EGI specific probes were identified and the T. reesei DNA fragments corresponding to the coding regions were subcloned into suitable plasmid vectors. A 4 kb Hindlll genomic fragment containing the entire EGI sequence, and two contiguous Hindlll genomic fragments of 1.16 kb and 2.3 kb which together encode the entire CBHI sequence were obtained by this method.

By further restriction analysis and sequencing, the sequence of the coding portion of the selected fragments was determined. Gene sequencing led to the identification of two introns in both CBHI and EGI genes. The cellulase gene introns are removed, preferably before the gene is placed in its expression vector.

For CBHI, the two introns were removed by cDNA fragment replacement. For EGI, one intron was deleted by replacing a portion of the genomic DNA with the corresponding sequences of a cDNA clone obtained from the cDNA library; the other intron was deleted by site directed mutagenesis using a chemically synthesized primer.

The intronless CBHI coding fragments were used to construct an expression vector capable of transforming yeast to secrete functionally active CBHI. For EGI, the ends of the coding sequences were modified by site directed mutagenesis to insert desirable restriction sites, making the entire coding sequence available as a Hindlll cassete. This cassette was cloned into an expression vector operable in yeast, and used to transform a suitable yeast host, resulting in a recombinant cell capable of EGI production.

### D. Preparation of Recombinant CBHI

This section describes an intron-free cellobiohydrolase-I (CBHI) gene, expression vectors into which the gene has been introduced by recombinant DNA techniques, hosts transformed with these vectors and CBHI prepared thereby. The CBHI gene is derived from a selected fungal source which preferably is capable of producing induced levels of extracellular cellulases, including CBHI, when grown on an inducing carbon source, such as cellulose.

### D.1 Selection of a Fungal Source of CBHI

In selecting a fungal source of CBHI for use in the present invention, it is advantageous to select an organism in which CBHI and the corresponding mRNA are inducible from substantially undetectable activity levels, when grown on a non-inducing carbon source, to easily measured levels when grown on an inducing carbon source, such as cellulose. Preferred fungal sources includes species of the Trichoderma genus, and most preferably T. reesei, which has an inducible CBHI enzyme that constitutes up to about 60% of the total extracellular cellulases induced when the organism is grown in the presence of cellulose. An additional advantage of T. reesei is that the enzymes which make up the inducible cellulase system have been relatively well studied. In particular, a substantial portion of the amino acid sequence of the T. reesei CBHI enzyme has been reported (Fagerstam, L. et al., FEBS LETT. (1980) 119:97 and Fagerstam, L. G., Doctoral Thesis, University of Uppsala, Sweden (1981)).

The ability of the microbial source to produce CBHI mRNA efficiently and in large amounts may be further enhanced by colony selection techniques, preferably after exposing the microbial source to a mutagen, to identify strains which are capable of producing elevated levels of one or more cellulases and/or which are capable of producing maximally induced levels of cellulase over reduced fermentation periods. One of the inventors herein (Shoemaker) has previously described a group of T. reesei mutant strains capable of producing higher induced levels of cellulase enzymes than in parent, commercially available strains. Further, as noted below, the maximal increase in extracellular cellulases was observed after a shorter enzyme-induction period than in the parent strain. The reader is referred to Shoemaker, S. P., et al., Trends in the Biology of Fermentations for Fuels and Chemicals, Hollaender et al. (eds), Plenum Publishing Corp., N.Y., N.Y., (1981) p. 89 for a detailed discussion of methods for obtaining mutant T. reesei strains of this type. What will be described herein are comparative properties of one of the mutant strains, designated strains L27, and the parent strain, Quartermaster No. 9414 (QM 9414), which led to the selection of the L27 strain as a preferred candidate for use in the invention.

The higher levels of extracellular cellulases produced by strain L27 suggest that this strain may produce higher levels of cellulase mRNA's than does strain QM 9414. To determine relative cellulase mRNA levels in the two strains, total polyA-RNA from each of the two strains grown under cellulase-inducing conditions was isolated and added to a cell-free protein synthesizing system to direct the synthesis of cellulase polypeptides. General procedures used in performing the cell-free protein syntheses are described in paragraph 0.3 below. The polypeptides synthesized in each cell-free synthesizing experiment were immunoprecipitated with antibodies to either CBHI or EGI. The immunoprecipitates were examined conventionally by sodium dodecyl sulfate-polyacrylamide gel electrophoresis (SDS-PAGE), referenced in subparagraph C.3. As judged by the relative amounts of radioactivity in specific protein-band regions of the gel (not shown) strain L27 gave higher in vitro translation activity for both enzymes, suggesting that the strain produces higher in vivo levels of mRNA for each cellulase component studied.

In the above experiment, the total polyA-RNA was isolated from the induced cultures at the time when greatest increase in extracellular cellulases was observed. This induction time was between about 18 to 20 hours for strain L27, and between about 26 and 28 hours for strain QM 9414. Therefore, strain L27 provides the additional advantage that relatively shorter induction times are required for the production of induced cellulase mRNA's. It is also noted here that strain L27 shows induced cellulase levels when grown on either cellulose or glucose, unlike the parent strain QM 9414 in which cellulase synthesis is glucose-repressed.

### D.2 Purification of CBHI Protein and Production of Anti CBHI Antibodies

According to one feature of the invention, CBHI mRNA may be identified by its ability to direct the synthesis, in a cell-free protein synthesizing system, of a polypeptide which is immunoreactive with anti-CBHI antibody. The antibody is preferably raised against a purified CBHI enzyme obtained from the selected fungal source of CBHI mRNA.

Purified CBHI may be obtained according to standard protein purification procedures from an extracellular culture broth. Typically, the broth, or filtrate, is fractionated by one or more ion-exchange chromatographic steps, and where necessary, a molecular-sieve chromatographic step, until enzyme purity is achieved. The enzyme can be assayed during purification steps by a conventional cellulase assay method, such as will be described below, and by SDS-PAGE.

By way of example, CBHI from T. reesei strain L27 was purified from an 8-day fermentation of the strain grown on 8% Avicel (microcrystalline cellulose) obtained from FMC Corp. (Rockfield, MD). The fermentation broth was filtered through a 0.45 micron micropore filter to remove cellular material, then dialyzed and concentrated in 50 mM sodium acetate buffer, pH 5.5, using an Amicon Ultrafiltration Cell (Lexington, MA). The concentrated extracellular filtrate was fractionated by anion exchange chromatography, using DEAE-Sepharose obtained from Pharmacia Fine Chemicals (Uppsala, Sweden). The column material was eluted with 0-300 mM sodium chloride gradient in 50 mM sodium acetate buffer, pH 5.0. CBHI eluted from the column at about 200 mM NaCI.

The enzyme was further purified by ion exchange chromatography, using DEAE-Sepharose in a 50 mM sodium acetate buffer, pH 6.0. The CBHI eluted from the column also at about 200 mM NaCl in a 0-300 mM sodium chloride gradient. The presence of CBHI in the column elution fractions was identified by the presence of reducing sugar after incubating the fractions in a solution containing 1% phosphoric acid swollen cellulose (PSC) in a 50 mM sodium acetate buffer, pH 4.5 at 45 ° C for thirty minutes. The identity of the reducing sugar as cellubiose was confirmed by high performance liquid chromatography (HPLC). The reader is referred to Shoemaker, S. P., et al., Biochem. Biophys. Acta (1978) 523:147 for details of exo-cellobiohydrolase assays.

The purity of the twice-chromatographed CBHI was analyzed by SDS-PAGE, using a modified discontinuous buffer system (Laemmli, U. K., et al., J. Mol. Biol. (1973) 80: 575) in a vertical slab apparatus. The electrophoretically separated proteins were detected by staining with Comassie Blue R250. The gel showed a single stained band, indicating substantial enzyme purity. The apparent protein molecular weight was estimated, by comparing its migration rate with that of known molecular-weight proteins, to be about 66,000 daltons.

A comparison was made between the amino acid composition and sequence of the purified L27 CBHI and the amino acid composition and sequence of the CBHI enzyme obtained from T. reesei, QM 9414, which is disclosed in Fagerstam, L., et al. supra and Fagerstam, L. G. supra.

The amino acid compositions of the two enzymes were compared as follows: Purified CBHI from strain L27 was reduced with dithiothreitol (0.02 mM) in 6 mM guanidine and carboxymethylated with iodo[14C] acetic acid (0.03 M), as described in Watt, K. W., et al., Proc. Nat. Acad. Sci. USA (1978) 75: 1731. Amino acid analysis of reduced, carboxymethylated CBHI was performed by hydrolyzing 40 /1.g of the protein with 0.1% phenol in 5.7 N HCI at 108° for 24, 48 and 72 hours in vacuo. The amino acids in the hydrolysate were quantified with a Spinco model 121 MB Amino Acid Analyzer (Beckman Instruments, Inc., Palo Alto, CA). Tryptophan was determined from a separate acid hydrolysate with 10% mercaptoacetic acid in 5.7 N HCI. The amino acid composition obtained for strain L27 CBHI was nearly identical to that of the published amino acid composition of CBHI from T. reesei, QM 9414.

The N-terminal portion of the strain L27 enzyme, containing the first 37 N-terminal amino acids in the mature CBHI enzyme, was sequenced, and the amino acid sequence compared with the corresponding region of the already-sequenced CBHI protein from T. reesei QM 9414. Automated amino acid sequencing of 40-50 nmole of purified strain L27 CBHI was conducted with a spinning cup sequenator (Model 890c modified with a cold trap, Beckman Instruments, Inc., Palo Alto, CA). T. reesei strain L27 CBHI is blocked at the amino terminus with a pyroglutamyl residue. The residue is removed by treating the protein with calf liver pyroglutamate amino peptidase (Boehringer Biochemicals), as described in Podell, D. N., et al., Biochem. Biophys. Res. Commun. (1978) 81: 176. After peptidase treatment, the enzyme was filtered, lyophilized, reduced with dithiothreitol, and carboxymethylated with iodo[¹⁴C] acetic acid and used directly for sequence analysis. Temporary reduction of background in sequence analysis was achieved by blocking all newly generated amino termini when a proline residue was determined to be the amino terminus. Identification of the degradation products from the sequenator was achieved on the amino acid analyzer in a fully automated HPLC system (Waters Associates).

The partial sequence as determined by this method is identical in 36 of the 37 amino acids to the published sequence of the first 37 amino acids of mature CBHI from T. reesei, QM 9414, confirming, along with the amino acid composition data, that the strain L27 CBHI is identical or nearly identical to the QM 9414 CBHI.

The purified protein is used to raise anti-CBHI antibodies by conventional methods. The antibody obtained is tested for its ability to neutralize exo-cellobiohydrolase enzyme activity, and for its ability to immunoprecipitate CBHI specifically from a mixture of induced cellulase enzymes.

To prepare antibodies against purified CBHI from T. reesei strain L27, the purified enzyme was mixed with Freunds adjuvant, and 0.2-0.4 ml of the mixture was injected intramuscularly into New Zealand white rabbits. Injections were repeated every ten days until antibody titers were detectable by the double immunodiffusion method of Ouchterlony, O., Arkiv Kemi. (1949) 1:43.

The anti-CBHI antibody produced was capable of neutralizing the exo-cellobiohydrolase enzyme activity of purified CBHI. Ouchterlony immunodiffusion analysis showed that the antibody prepared against the above CBHI preparation was immunoreactive against both CBHI and EGI enzymes.

### D.3 Preparation of mRNA Enriched in CBHI mRNA

A preferred fungal source of CBHI, such as T. reesei strain L27, when grown under cellulase-induction conditions, contains a CBHI mRNA which is essentially undetectable in uninduced culture. The mRNA is capable of directing the synthesis, in a cell-free protein synthesizing system, of a polypeptide which is immunologically reactive with antibodies prepared against the CBHI protein purified from the sane microbial source. To obtain an RNA fraction enriched for CBHI mRNA, total polyA-RNA isolated from the induced culture is size fractionated, for example, by gel-electrophoresis. PolyA-RNA from induced and uninduced cultures of the microbial source may be fractionated in parallel, allowing a comparison of the gel- electrophoretic patterns of the two polyA-RNA preparations.

In order to identify the mRNA fraction most enriched in CBHI mRNA, various size fractions of the fractionated polyA-RNA are examined for their ability to direct the synthesis, in a cell-free synthesizing system, of a polypeptide immunoprecipitable with anti-CBHI antiserum. By way of illustration, procedures for preparing an mRNA fraction enriched to CBHI mRNA from T. reesei strain L27 will now be described.

Mid-log T. reesei strain L27 cultures grown on either Avicel (for 18 hours) or glycerol (for 48 hours) were harvested and frozen in liquid nitrogen. Cultures grown on glycerol showed no measurable extracellular CBHI activity during the 48 hour growth period. Total cellular RNA was isolated from these cultures using a modification of the method described in Chirgwin, J. M., et al., Biochem. (1979) 18: 5294. Briefly, frozen cells were homogenized in 4.7 M guanidine thiocyanate and 12 mM adenosine:VOS0₄ complex (an RNase inhibitor). The RNA was pelleted through a CsCI cushion by ultracentrifugation, and the polyA-RNA was isolated by oligo dT chromatography. The polyA RNA's from the induced and uninduced cultures were fractionated on methyl mercury agarose gels, according to known procedures, using standard Sea Kem agarose obtained from Marine Colloids (New York, NY). Figure 1 shows the electrophoretic patterns of poly ARNA derived from T. reesei strain L27 grown on Avicel (lane 1) or on glycerol (lane 2). Comparison of the two gels shows several RNA bands which are unique to or present in higher concentrations in induced cultures. The RNA size region corresponding to 23S RNA is shown at the left in the figure.

The gel containing induced RNA was sliced at 2 mm intervals starting at the 235 RNA region of the gel and proceeding toward lower molecular weight RNA's (toward the bottom of the gel in the figure). To release RNA from the gel slices, each slice was melted at 95°C for 5 minutes in a 0.01 M Tris-HCI buffer, pH 7.5, containing 0.1 M NaCI, 0.01 M EDTA, and 0.2% SDS. The agarose was pelleted by centrifugation. After phenol/chloroform extraction of the supernatant, the RNA was precipitated with ethanol and redissolved in water.

A small amount of each RNA fraction obtained from the gel slices was added to a cell-free protein synthesizing system, specifically a reticulocyte lysate kit obtained from New England Nuclear Company (Boston, MA) and sold as reticulocyte lysate/methionine L-[³⁵ S]-translation system. Pelham, R. B., et al., Europ. J. Biochem. (1979) 67:247 describes a typical reticulocyte lysate system. After a defined reaction period, aliquots of the lysate were removed and analyzed by SDS-PAGE. The lysate material analyzed was either total lysate or the immunoprecipitate produced by adding anti-CBHI antibody to the lysate. The immunoreactive products were precipitated essentially according to conventional methods.

Selected gel electrophoretic patterns obtained are shown in Figure 2. The molecular weights shown in the figure, in thousands of daltons, are indicated by the positions of marker proteins in the lane at the left in the figure. The electrophoretic pattern of total lysate proteins produced by supplying the translation system with total "induced" polyA-RNA is shown in lane 1. Lane 2 shows an analogous pattern using a hydrolyzed immunoprecipitate of these peptides with anti-CBHI. As seen, the lane 2 pattern consists of a single, relatively broad band in the molecular weight range of about 66,000 daltons, the estimated apparent molecular weight of CBHI.

As indicated above, antibody prepared against T. reesei CBHI cross reacts with T. reesei EGI, raising the possibility that at least a portion of the broad 66,000 molecular weight band seen in lane 2 is due to endogluconase polypeptide synthesis. The fact that purified EGI, when analyzed by SDS-PAGE, gives a protein band which is distinct from and has a lower molecular weight than CBHI, argues against this possibility. Also, it is expected that CBHI polypeptide synthesis would account for a large portion of the total peptide synthesis occurring in the translation system, since the CBHI enzyme makes up about 60% of the total cellulases induced in the fungal culture at the time the polyA-RNA was isolated. By contrast, EGI makes up only about 5% of the total cellulases.

The size fractionated RNA obtained from successive gel slices was used to direct the synthesis of radioactive polypeptides in the translation system. Gel slice #9 --the slice located approximately 18 millimeters from the point of initial cutting of the gel at a 235 RNA band-- produced the total translation system-produced polypeptide pattern seen in lane 3, having a concentration of proteins in the 46,000-69,000 dalton molecular weight range. The analogous pattern given by the immunoprecipitate produced by adding anti-CBHI antibody is seen in lane 4, which shows a single broad protein band in the CBHI molecular weight range.

The RNA from gel slice #10, containing the next smaller size range of fractionated polyA-RNA, directed the synthesis, in the translation system, of the polypeptides seen in lane 5. Similar analysis at a hydrolyzate of the immunoprecipitate is shown in lane 6. The relatively high density of immunoprecipitated material in the CBHI molecular weight range indicates that gel slice #10 is more highly enriched for CBHI mRNA than is gel slice #9.

The analogous polypeptide patterns produced by RNA from gel slice #11 are shown in lanes 7 and 8, lane 7 showing the total lysate polypeptides, and lane 8, polypeptides which immunoprecipitate in the presence of anti-CBHI antibody. As seen, the density of the 66,000 dalton molecular weight band in lane 8 is comparable to that in lane 4, and appreciably less than that seen in lane 6. From this comparison, gel slice #10 was identified as the gel slice maximally enriched in CBHI mRNA, and was therefore used as the source of CBHI mRNA in the procedures described below for producing a CBHI probe and CBHI cDNA's.

To confirm that the relatively broad band in the 66,000 dalton molecular weight range seen in lane 6 represents the product of induced CBHI mRNA, the translation system was primed with a comparable-size mRNA fraction of polyA-RNA from an uninduced T. reesei culture (grown in the presence of glycerol). After fractionating the uninduced polyA-RNA by gel electrophoresis, the RNA which eluted from the 10th gel slice down from the 23S region of the gel was added to the translation system. Total translation polypeptides and immunoprecipitated peptides from the translation system are seen in lanes 9 and 10, respectively. As seen, lane 9 contains very little newly synthesized protein in the 66,000 molecular weight range, and lane 10 shows essentially no newly synthesized protein which immunoprecipitated in the presence of anti-CBHI antibody. The results confirm that most, if not all of the newly synthesized polypeptide which is immunologically reactive with anti-CBHI, is produced under the direction of induced CBHI mRNA.

### D.4 Preparation of a CBHI cDNA Probe

The enriched CBHI mRNA fraction from above provides a template for synthesizing radiolabeled, single-stranded CBHI cDNA for use as a probe in identifying genomic digest fragments of the selected microbial organism which contain regions of the CBHI gene.

Methods for producing radiolabeled, single-stranded cDNA from mRNA are well known. The general method described in Payvar, F., et al., J. Biol. Chem. (1979) 254: 7636 was followed to produce cDNA from T. reesei strain L27 CBHI enriched mRNA. Briefly, the enriched mRNA was pretreated with 10 mM methyl mercury hydroxide to denature the RNA, and then introduced into a reaction mixture containing radiolabelled nucleotides, oligo dT as a primer and 2 mM adenosine:VOS0₄ as an RNAase inhibitor. Following cDNA synthesis, the polyA-RNA was destroyed by treatment with NaOH, and the cDNA was size analyzed by gel electrophoresis to confirm the synthesis of full-length cDNA. A typical gel electrophoresis pattern of the cDNA fraction showed a prominent major band in the 1.6 to 1.8 kilobase size regions, corresponding approximately to the size range of the enriched mRNA fraction used to produce the cDNA.

A CBHI probe may also be produced, in accordance with known methods, by radiolabeling CBHI mRNA fragments. Alternatively, a synthetic polynucleotide constructed to have a codon sequence corresponding to the known amino acid sequence of a short segment of the mature CBHI protein may also provide a suitable CBHI probe for use in the invention.

### D.5 Preparation of Genomic DNA Clones Containing CBHI Gene Regions

Total genomic DNA isolated from the selected microbial source is treated with one or more selected endonucleases to generate genomic fragments which can be cloned and identified as containing CBHI gene regions by their ability to hybridize the above cDNA CBHI probe. The clones identified as containing CBHI gene regions provide the gene material used in determining the nucleotide sequence of the genomic DNA, and clones containing selected regions of the gene may be used in constructing an intron-free CBHI gene according to methods detailed below.

A complete library of T. reesei L27 DNA was constructed in lambda L47.1. The lambda phage vector was prepared by isolating phage DNA and purifying the cloning arms away from the stuffer fragment as follows: starting with whole phage DNA, cos sites were ligated together under "sticky end" conditions. The efficiency of ligation was determined to be about 80-90% by agarose gel analysis. Ligated phage DNA was cut with BamHl and Sall. The BamHl digestion releases stuffer fragments from ligated arms, whereas Sall cuts the stuffer fragments twice more and insures greater ease of separation from the BamHl digested arms. The BamHl digested arms were purified from the stuffer fragments using a CsCI velocity sedimentation gradient.

T. reesei genomic DNA was partially digested with Sau3A and size fractionated by centrifugation on NaCl gradients, and the fraction containing molecules in the 15-20 kilobase range selected. Fragments of this fraction were mixed with the BamHl arms of the vector in a 1:1 molar ratio, at a total DNA concentration of 200 ug/ml and ligated overnight at 4°C with 0.2 Weiss units of T4 DNA ligase in a volume of 20 microliters. One half microgram from the ligation was packaged into lambda heads in vitro, using frozen packaging components as described in Hohn, B., Methods in Enzymology (1979) 68:299-309, and the resulting phage were plated on E. coli strain MM 294. The procedure produced a library of about 1.7 x 10⁴ phage, which gave a 0.99 + probability of any single copy gene being represented.

The T. reesei genomic library so produced was probed with radiolabeled CBHI cDNA prepared as above. Out of approximately 60,000 phages tested, 54 phages hybridized to the cDNA probe. To eliminate false positives, 23 clones which produced the strongest hybridization with the cDNA were further purified and probed with two different cDNA probes, one produced from mRNA from "induced" cultures, and the other from mRNA from "uninduced" cultures, lacking CBHI mRNA. 22 of the 23 selected genomic library clones hybridize the "induced" cDNA probe, but not the "uninduced" cDNA. Restriction analysis of 15 of the 22 selected clones revealed several duplicates, reducing the CBHI gene library to six unique clones.

One of the six clones, designated lambda CBH-4, which was presumed to contain all of the CBHI genomic coding sequence was used to generate gene subfragment clones. Experimentally, lambda CBH-4 was digested to completion with Hindlll and the digest fragments produced were ligated into Hindlll- digested E. coli plasmid pBR322. Plasmids containing digested CBHI fragments in recombinant form were selected by ampicillin resistance (carried on the pBR322 vector), and screened for tetracycline sensitivity produced by insertion of heterologous DNA leading to inactivation of the tetracycline-resistance gene. Subclones containing either a 1.16 kilobase fragment, including a 5' region of the CBHI gene, or a 2.3 kilobase fragment, including a 3' portion of the gene, were selected. These two clones were designated pCBH157 and pCBH164, respectively, and are illustrated in Figures 3A and 3B, respectively.

pCBH157 contains the coding region for the first 278 amino acids of the mature CBHI protein from T. reesei strain L27. The heavy-lined segment of pCBH157 shown in Figure 3A represents the entire 1.16 kilobase DNA fragment, the interior line segment extending along a portion of the fragment represents the 5' CBHI gene region.

The pCBH164 subclone, containing the 2.3 kilobase Hindlll fragment from lambda CBH-4, includes the region of the CBHI genomic gene which encodes the last 218 amino acids of the mature CBHI protein, from amino acids 279 to 496. The heavy-line segment in Figure 3B represents the 2.3 kilobase DNA fragment, and the interior line segment indicates the coding region of the CBHI genomic gene.

Figures 3A and 3B also indicate the positions of several restriction endonuclease sites within the CBHI gene region, as determined by conventional restriction endonuclease site analysis.

pCBH157 and pCBH164 were tested for their ability to "fish out" CBHI mRNA from a total polyA-RNA preparation obtained from a cellulase-induced T. reesei L27 culture. Each clone was bound to a nitrocellulose filter, according to published procedures (Harpold, M., H., et al., Nucleic Acids Res. (1978) 5:2039) and total polyA-RNA from an induced T. reesei L27 culture was added to the filters under conditions which allowed hybridization of complementary sequences. After stringent washes to remove unbound (nonspecific mRNA), the mRNA which specifically hybridized the filter-bound plasmid was eluted.

For each filter, bound and unbound RNA fractions were translated in the cell-free protein synthesizing system described above. Total proteins translated from the two samples were precipitated with a mixture of anti-CBHI antibodies and Staphylococcus aureus cells. The total translated proteins were analyzed on SDS-PAGE and by analysis of the immunoprecipitates as described above. Plasmids pCBH164 and pCBH157 both bound RNA capable of directing the synthesis of a protein which was immunologically reactive with anti-CBHI antibody.

### D.6 Determination of Genomic Sequences

To obtain sequence information, subclones of the genomic CBHI gene, obtained in accordance with above-described methods, are further fragmented by digestion with one or more selected endonucleases to produce subcloned fragments suitable in length for nucleotide sequencing. By way of illustration, methods used in sequencing the CBHI genomic gene from T. reesei strain L27 are described below.

Subclone pCBH157, containing a 5' portion of the gene, was digested substantially to completion with both Hindlll and EcoRl, producing two gene-region fragments of approximately 600 base pairs and 500 base pairs, as can be appreciated from Figure 3A. The two fragments were subcloned into M13 bacteriophage vectors M13mp8 and M13mp9, respectively.

The other Hindlll fragment subclone, pCBH164, was digested variously with BamHl, Hindlll, and Hincll. The fragments produced were subcloned into the vector M13mp8.

The fragments of the CBHI genomic region subcloned into the vectors M13mp8 and M13mp9 were sequenced by the dideoxy chain termination method described in Sanger, F., et al., Nat. Acad. Sci. USA - (1977) 74:5463 and Messing, J., et al., Nucleic Acids Res. (1981) 9:309. Portions of the sequence were confirmed by the Maxam-Gilbert sequencing technique (Maxam, A. M., et al., Proc. Nat. Acad. Sci. USA - (1977) 74:560). The entire gene sequence, extending from the 5' Hindlll site in the 1.16 kilobase Hindlll fragment from T. reesei strain L27 to approximately 306 nucleotides past the stop codon at the 3' end of the CBHI gene in the 2.3 kilobase fragment, is shown in Table I below. The sequence data was internally consistent with respect to the various fragments sequenced.

The nucleotide sequence obtained was compared, in a computer-programmed matching operation, with the regions of known amino acid sequence of T. reesei CBHI from Fagerstam, L., et al. supra and Fagerstam, L. G. supra. The matching operation examined the nucleotide sequence in each of the three possible reading frames for codon correspondence with the given amino acid sequence. The matching operation produced nearly complete correspondence between coding regions of the CBHI gene and the regions of known amino acid sequence of CBHI from T. reesei, QM 9414. This matching allowed the identification of the correct reading frame, the start and stop codons, and the two introns in the gene, as will be discussed. Table I also shows the amino acids corresponding to the leader sequence encoding portions of the gene. (Leader sequence amino acids are underlined.) Recognition sequences of selected restriction endonucleases referred to in the text are indicated by underlining in the table. Overlined portions of the untranslated introns indicate recognizable sequences analogous to those of introns reported in other genes.

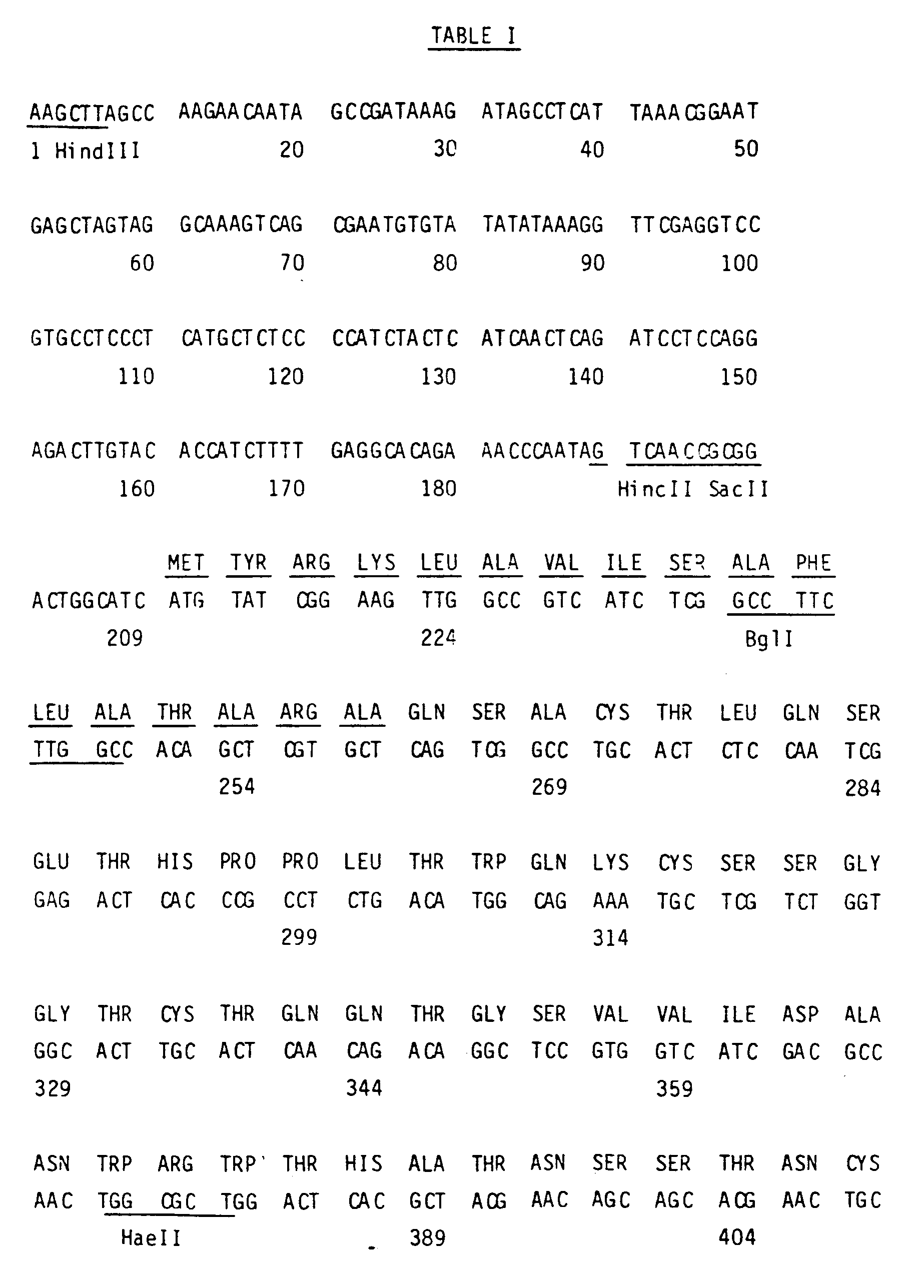

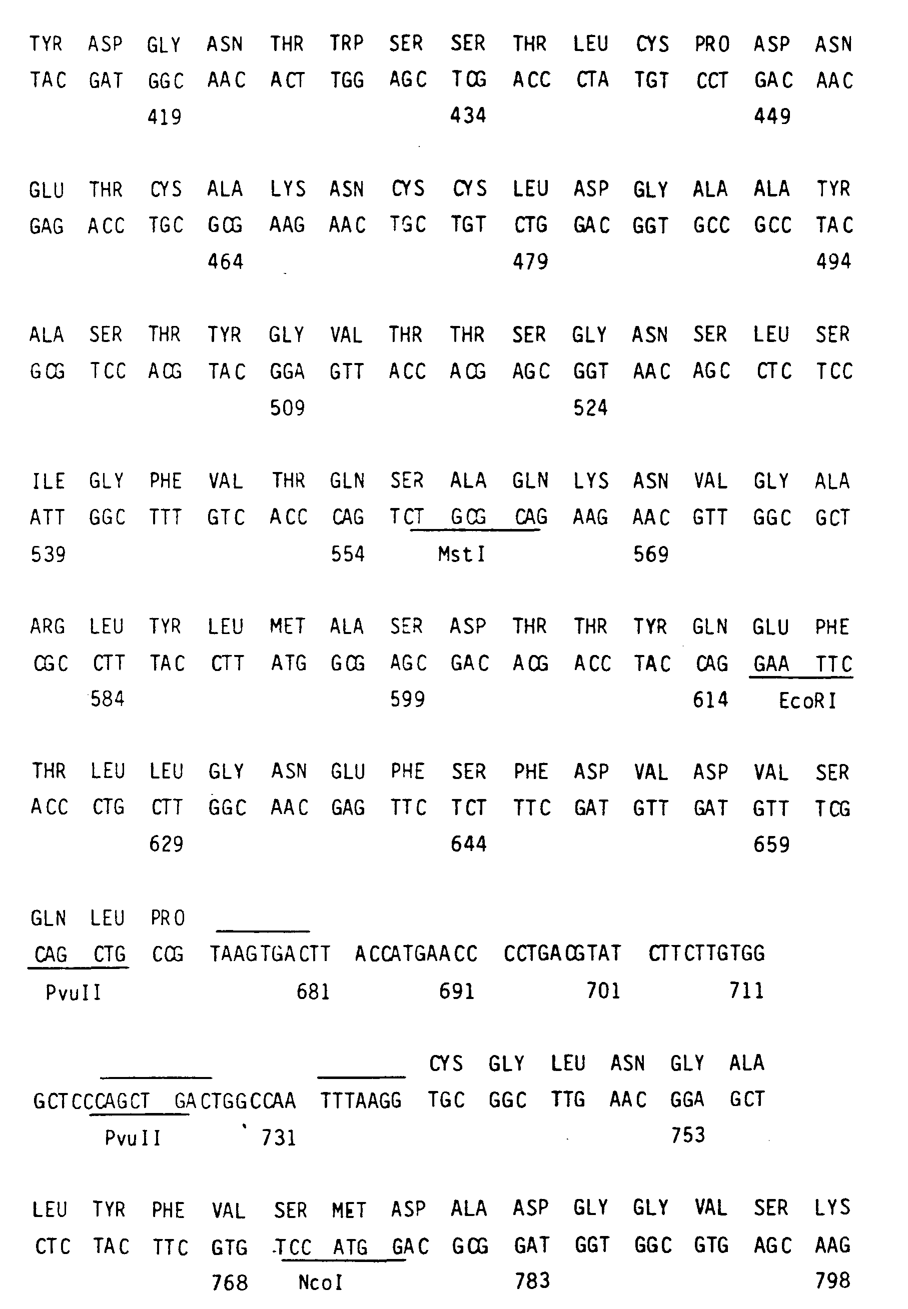

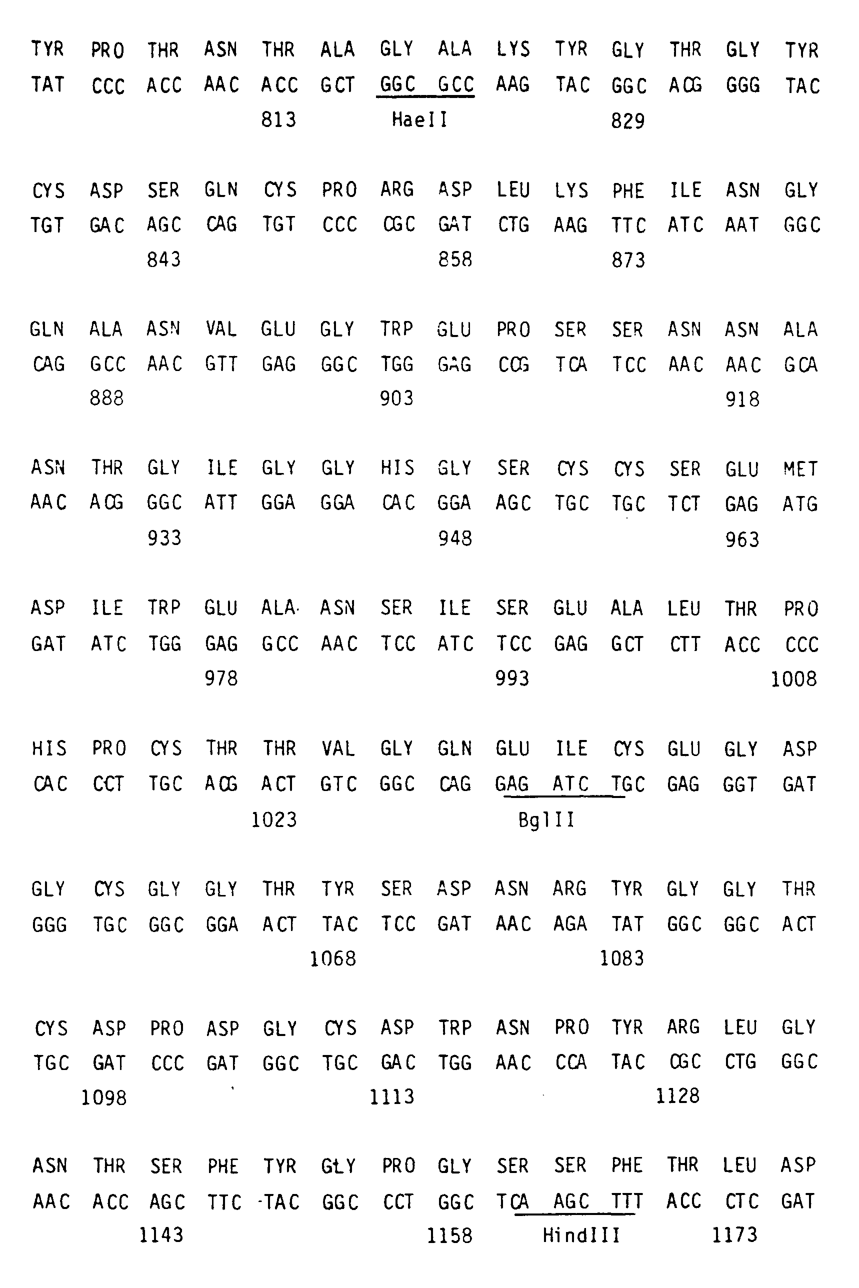

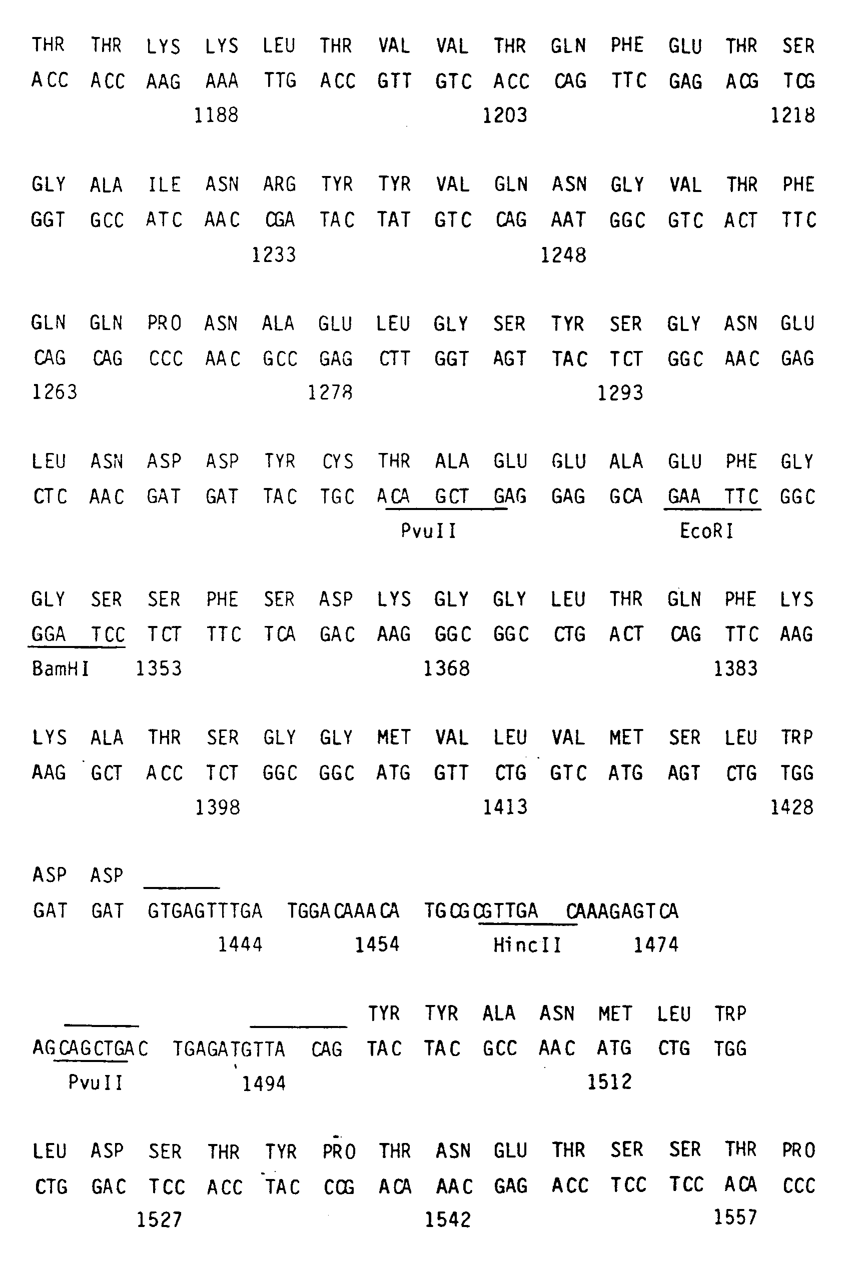

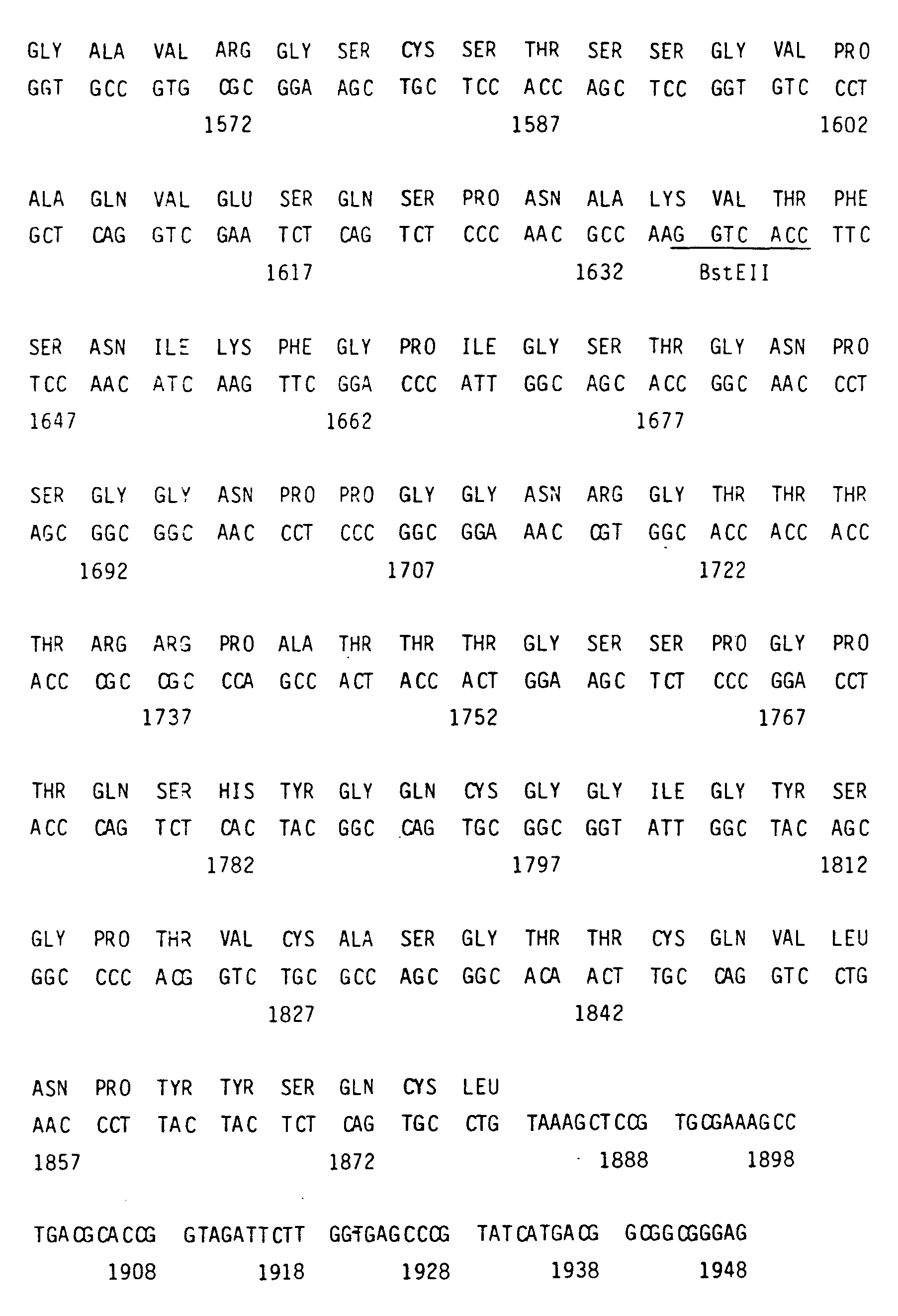

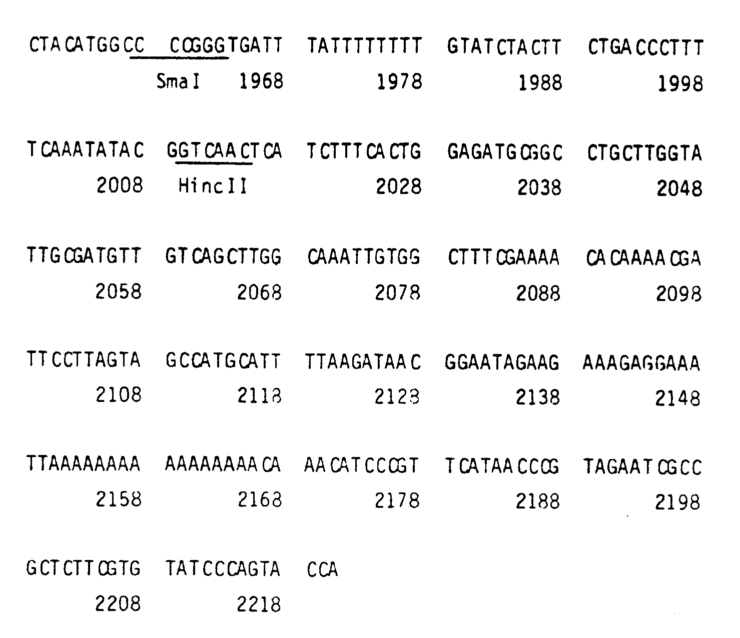

Viewing the table, it is seen that ATG start codon is located at nucleotides 210-212, numbered from the 5'-end of the inserted sequence in pCBH157. The codon at nucleotides 261-263 corresponds to the N-terminus glutamine (pyroglutamine) amino acid in the mature CBHI protein. The codons between nucleotides 210 and 260 presumably code a 17-amino acid hydrophobic peptide leader sequence whose amino acid sequence, as determined by corresponding codon sequence, is shown underlined in the table. The codons between nucleotides 261 and 671 code for the first 137 amino acids of the mature CBHI enzyme.

Between the coding regions coding for amino acids 137 and 138 in the mature CBHI protein, is a 67 base pair region which is identified herein as the first, or upstream intron in the CBHI gene. This intron contains a 7 base-pair AGCTGAC sequence (highlighted by overlining in Table I), Which is similar to a 7-mer in the intron sequence of the actin gene from yeast (Langford, C. J., et al., Cell (1983) 33:519) and in three of the introns in the glucoamylase gene from Aspergillus awamori and which is identical to an intron sequence in the glutamate dehydrogenase gene from Neurospora crassa (Kinnaird, J., et al., Gene (1982) 20: 387) and one of the introns in the A. awamori glucoamylase gene. This sequence apparently provides a signal which is necessary for splicing the intron out of the mRNA transcript. In addition, the intron contains donor and acceptor consensus sequences TAAGTG and TTTAAGG, respectively (also highlighted by overlining in Table I), which are identical to donor and acceptor site consensus sequences which have been cataloged by Mount, S. M., Nucleic Acids Res. (1982) 10:459.

Amino acid 138 (cysteine) through amino acid 369 (aspartate) are encoded by an uninterrupted protein-encoding region extending between nucleotides 739-1434. The gene region between the codons coding for amino acids 369 and 370 contain 63 non-coding nucleotides which make up a second, or downstream intron in the genomic CBHI gene. The second intron also contains the 7-base pair sequence AGCTGAC characteristic of yeast introns, and donor and acceptor consensus sequences GTGAGT, and TTACAG, respectively, which are identical to known donor and acceptor site sequences at intron-exon junctions in yeast genes (Mount, S. M., supra).

The second intron is followed by an uninterrupted 127-cocon region encoding for amino acids 370-496 of the mature CBHI protein, whose C-terminal amino acid is leucine (encoded by nucleotides 1876-1878). Nucleotides 1879-2221, which are downstream of stop codon at the 3' coding region of the gene, contain Smal and Hincll sites as shown.

The sequence of nucleotides in the protein-encoding portions of the gene in Table I forms a codon sequence which encodes the sequence of amino acids shown. Many of the codons can be varied in nucleotide sequence, generally at the third nucleotide position, without changing the amino acid specified by the codon. A variety of methods for producing single nucleotide, or point mutations in genes are known to those skilled in the art. The term "codon sequence", as defined herein, includes all nucleotide sequences which encode a particular amino acid sequence, the nucleotide sequence shown in Table I being one of the many codon sequences which encodes the amiono acid sequence there shown.

In addition to nucleotide substitutions which preserve the gene's codon sequence, the present invention contemplates nucleotide variations in the gene which lead to "neutral" or non-critical amino acid changes in the encoded CBHI enzyme, as discussed above. It is known, particularly from studies on evolutionary changes in protein amino acid sequences, that neutral amino acid changes in a protein can occur without significantly changing the protein's functional characteristics. A variety of techniques are available for producing minor codon changes experimentally, including mutagenizing a gene to produce point or deletion mutations; selectively cleaving, followed by deleting or adding selected nucleotides, and then religating the gene; and by applying conventional oligonucleotide mutagenesis techniques. A CBHI gene so mutated may encode an enzymatically active CBHI enzyme, and often one whose specific activity is substantially unchanged with respect to the unmutated CBHI enzyme. The present invention includes genes whose codon sequences encode such neutral amino acid changes in the CBHI enzyme, as well as those whose codon sequences have strict one-to-one codon correspondences with the amino acid sequence of an unmutated mature CBHI. As defined herein, a gene whose codon sequence has a "substantially" one-to-one correspondence with mature CBHI includes both those having a strict correspondence and as those having neutral or minor codon variations which encode a polypeptide with CBHI (exo-cellobiohydrolase) enzyme activity (see Shoemaker, S. P., et al., Biochem. Biophys. Acta (1978) 523:147).

Figure 4 shows a map of the genomic CBHI gene from T. reesei strain L27, constructed from the above sequence data. The map shows the protein-encoding region of the gene in relation to several restriction endonuclease sites, and in particular, in relation to the Hindlll sites defining the above-described 1.16 and 2.3 kilobase Hindlll gene fragments. As seen from the map, the upstream intron in the gene is located in the central region of the 1.16 kilobase Hindlll fragment, and the downstream intron, in the central protein-encoding portion of the 2,3 kilobase fragment.

In the example just discussed, the existence and location of the upstream and downstream introns in the T. reesei strain L27 CBHI gene were determined by comparing the codon sequence of the sequenced genomic gene with the amino acid sequence of the encoded protein, a method which can be used to define the introns with high precision. Alternatively, the lengths and positions of the gene introns can be determined by comparing the nucleotide sequence of the genomic CBHI gene with that of a full-length CBHI cDNA prepared and sequenced in accordance with the general procedures described herein. It may also be possible to identify the existence and location of genomic gene introns from the nucleotide sequence alone. As discussed above, both introns in the T. reesei strain L27 CBHI gene contain a 7 base-pair sequence characteristic of yeast introns, thus providing a means for making a preliminary identification of the introns. Similarly, the donor and acceptor end regions of the intron may be identified by characteristic consensus sequences which match one of the several consensus sequences which have been cataloged. While the invention has been illustrated with reference to the amino acid-codon matching method of intron identification, the other two just-mentioned methods for determining the existence and location of gene introns from the nucleotide sequence are also included in the method of the invention.

### D.7. Elimination of Intron Sequences From the Gene

This section describes a method for constructing an intron-free CBHI gene. The method includes, first, providing in a suitable cloning vector, a 5' genomic CBHI gene fragment which extends from the 5' gene end to a selected restriction endonuclease site downstream of the 5'-end intron in the genomic CBHI gene. Generally, the methods described above in subparagraph D.5 for producing genomic DNA digest clones containing CBHI gene regions are applicable for producing such a 5' genomic gene fragment.

In particular, one advantageous procedure illustrated herein involves a first step of treating the genomic DNA with a selected restriction endonuclease under conditions which produce one or more fragments containing the entire CBHI gene. This complete-gene fragment is then further digested to generate two or more gene subfragments, including, for example the 1.16 kilobase 5' CBHI gene fragment from T. reesei, strain L27 described above.

The second step includes providing, in recombinant form, one or more CBHI cDNA coding sequence fragments which can be spliced together or substituted for corresponding intron containing regions of genomic gene fragments, to produce uninterrupted protein-encoding gene regions. The cDNA gene fragments are constructed by conventional methods, preferably by copying an enriched CBHI mRNA to form a single-stranded cDNA, and forming a double-stranded cDNA from the single strand.

The general prodecure for providing enriched CBHI mRNA follows that described in subparagraph D.3 above. The enriched mRNA, when reacted with oligo dT in the presence of reverse transcriptase, serves as a temptate for synthesizing a cDNA strand, also according to above-described methods. The mRNA in the mRNA/cDNA hybrid is hydrolyzed by treatment with NaOH, leaving a single-stranded cDNA which can serve to prime and as a template for the synthesis of a second cDNA strand by E. coli DNA polymerase 1, according to methods described in Maniatis, et al., Molecular Cloning - a Laboratory Manual, Cold Spring Harbor Laboratory, Cold Spring Harbor, New York (1982) pp. 235-238, to give a "hairpin" of double stranded DNA. Following synthesis of the second cDNA strand, the hairpin loop may be removed by treatment with nuclease S1, and various duplex cDNA's selected.

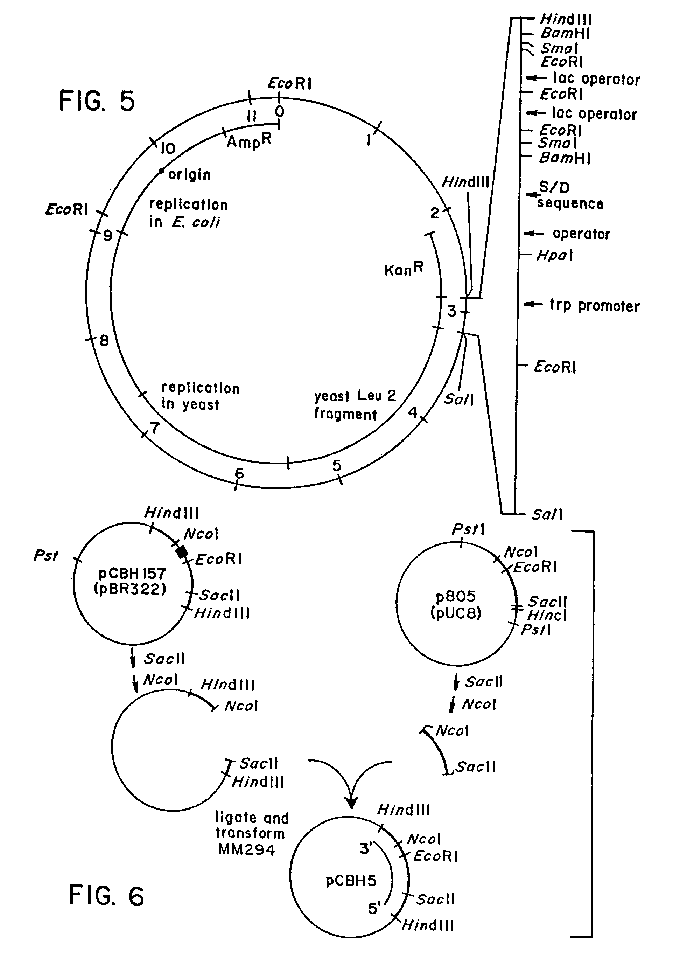

Double-stranded cDNA obtained by copying T. reesei strain L27 CBHI mRNA was formed according to above-described methods and cloned into a pUC8 vector according to standard procedures. The clones were identified by their ability to hybridize one of the above-described pCBH157 or pCBH164 clones containing regions of CBHI genomic DNA. One of the cDNA fragment clones selected, designated p805, is shown in Figure 6. The cDNA fragment in p805, which is indicated by heavy line in the figure, extends from the 5'-end of the gene downstream past an Ncol site which, in the genomic gene, is located on the downstream side of the first or upstream intron, as indicated.

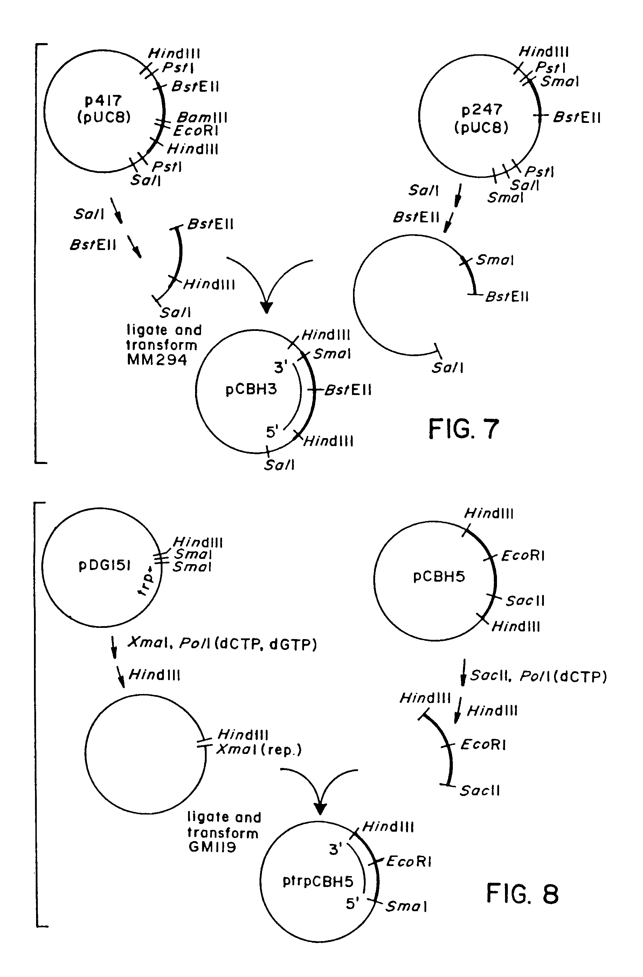

Two other cDNA clones, identified as p417 and p247, are seen in Figure 7. As shown, the cDNA fragment in p247 extends from the 3'-end of the cDNA upstream past a BstEll site. The cDNA fragment in p417 overlaps fragment 247 at the just-mentioned BstEll site, and extends upstream therefrom past the internal Hindlll site in the CBHI gene, as shown. The lengths and relative positions of the three just- described cDNA fragments were determined by conventional restriction endonuclease mapping.

To construct the intron-free CBHI gene, a portion of the genomic DNA containing the the upstream intron was excised from 5' genomic fragment and a corresponding cDNA region inserted into the excised intron-containing region. This intron-free 5' gene fragment was then joined, at a selected restriction endonuclease site, to a 3'-end cDNA fragment which extends from such site to the 3'-end of the gene. In the illustration below, the intron-free 5' gene fragment was cloned into a suitable expression vector adjacent control sequences which allow expression of the fragment when the vector is introduced into a suitable host. The remaining 3' cDNA portion of the gene was then introduced into the expression vector to produce a full-length, intron-free CBHI gene positioned and oriented in the vector for gene expression.

### D.7.a. Construction of pCBH5--Elimination of the Upstream Intron

Figure 6 illustrates plasmid pCBH157, containing the 1.16 kilobase Hindlll fragment from T. reesei strain L27 genomic DNA which is also shown in Figure 3A. From the gene nucleotide sequence data presented above, it is known that the 1.16 kilobase fragment includes a unique Sacll recognition site 11 base pairs 5' to the start of translation at the first ATG initiation signal in the gene, and a unique Ncol cleavage site 32 base pairs 3' to the upstream intron in the gene fragment, as indicated in the figure.

Plasmid p805, containing an approximately 800 base pair 5'-end cDNA fragment, also contains the just-mentioned unique Sacll and Ncol sites, as seen in Figure 6. Plasmids pCBH157 and p805 were each treated with Sacll and Ncol, releasing from plasmid pCBH157 a 573 base pair fragment containing the upstream intron, and from plasmid 805, a corresponding intron-free fragment 506 base pairs in length. The intron-free Sacll/Ncol fragment from p805 was ligated into the digested pCBH157 plasmid to produce a new plasmid, designated pCBH5, containing a chimeric genomic/cDNA gene fragment consisting of the 1.16 kilobase Hindlll fragment from which the upstream intron has been eliminated.

### D.7.b. Construction of pCBH3--Elimination of the Downstream Intron

Construction of a 3'intron-free gene fragment will now be considered. With reference to Figure 7, plasmid p247 contains a Smal site 77 base pairs distal to the TAA stop codon of the gene; a unique BstEll site approximately 138 nucleotides 3' to the downstream intron in the genomic sequence and a unique Sall site in the polylinker region of the plasmid, 5' with respect to the cDNA coding sequence.

Plasmid p417, also illustrated in Figure 7, contains the same unique BstEll site as in p247 but the CBH coding sequence and extends further in the 5' direction past the Hindlll recognition site within the CBHI coding sequence, and includes a unique Sall site about 600 base pairs upstream of the BstEll site, as shown.

To construct a composite 3' cDNA gene clone extending from the downstream end of the gene's stop codon upstream past the internal Hindlll site, plasmids p247 and p417 were each digested to completion with BstEll and then Sall. The smaller 600 base pair BstEll/Sall DNA fragment from p417 was purified and ligated with the BstEll/Sall p247 vector fragment. Plasmid pCBH3 shown in the figure was identified as the desired recombinant having a cDNA fragment extending from the 3'-end of the cDNA to the internal Hindlll site in the gene.

### D.8 Construction of Expression Vectors for Bacterial Hosts

The CBHI gene fragments from pCBH5 and pCBH3 were cloned into a selected E. coli/S. cerevisiae shuttle vector to a position and orientation adapted to allow CBHI gene expression under control of the trp promoter. The host vector is a 11.12 kilobase shuttle vector designated pDG151 deposited at ATCC on May 11, 1984 and designated accession number 39686, detailed in Figure 5. The plasmid includes a modified aminoglycoside phosphotransferase APH-I gene which encodes an enzyme which confers antibiotic resistance against kanamycin, neomycin, G418, and other antibiotics.

The sequences of this plasmid are as follows:
1. Coordinates 0-1.54 are the 1.54 kb Hindlll/EcoRl 3' untranslated terminator sequences of the EN01 gene derived from peno46 (Holland, M. J., et al., J. Biol. Chem. (1981) 256: 1385).
2. Coordinates 1.54-2.75 contain the modified APH-I gene, which is described in detail in The Molecular Biology of Yeast, Cold Spring Harbor Meeting, August 16-21, 1983.
3. Coordinates 2.75-2.85 contain a sequence comprising duplicated lac operator sequences flanked by inverted polylinker repeats. The sequence immediately preceding the ATG start codon of the modified APH-I (at 2.75) is: 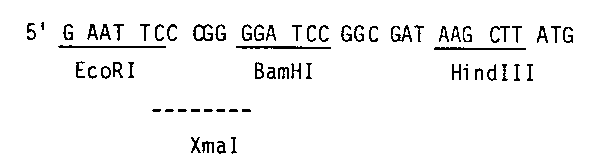
4. Coordinates 2.85-2.95 are a 107 bp 5'-EcoRl(repaired)/BamHl-3' fragment containing the trp promoter- operator.
5. Coordinates 2.95-3.04 contain a 90 bp pBR322 segment between the Sphl(repair) and Sall sites.
6. The LEU2 gene from yeast occupies coordinates 3.04-5.25. It was obtained as an Xhol/Sall digest fragment from YEp13 (Broach, J., et al., Gene (1979) 8: 121).
7. The 2 micron plasmid replicon in coordinates 5.25-8.97 is obtained by digestion of pDB248 (Beach, D., et al., Nature (1981) 290: 140) with EcoRl (repair) and Sall, and isolation of the 2.7 kb DNA fragment containing the replicon. The existence of the appropriate Sall site was not deducible from the disclosure of Beach. However, pDB248 was shown to contain a Sall site about 50 bp downstream from the indicated LEU2 region/2u. Pstl tailing site as set forth in the Beach reference.
8. Coordinates 8.97-11.12 contain a Tthllll(repair)/EcoRl digest fragment of pBR322 which supplies Amp^{R} and an E. coli origin of replication.

Figure 8 illustrates the method by which the intron-free 5'-end gene fragment from pCBH5 was inserted in pDG151. Plasmid pCBH5 was digested to completion with Sacll, and the 3' protruding two-base sticky end was repaired with E. coli DNA polymerase I (Klenow) in the presence of dCTP. Following inactivation of the Poll, the DNA substrate was digested to completion with Hindlll and the 895 base pair Sacll(repair)-/Hindlll fragment was purified. With reference to the expanded gene region shown in Figure 5, plasmid pDG151 was digested to completion with Xmal, liberating a 62 base pair DNA fragment containing the duplicated synthetic lac operator fragment between the two adjacent Xmal sites. The 5' protruding four-base cohesive ends were repaired with Poll, in the presence of dCTP and dGTP. Following inactivation of the Poll, the flush ended DNA was digested to completion with Hindlll. The repaired Xmal site in the 11.12 kilobase vector DNA fragment is 18 nucleotides 3' of the E. coli trp promoter and leader peptide ribosome binding site, and the cohesive Hindlll site immediately precedes the initiating codon of the modified APH-I gene.

The pDG151 DNA fragments (1.5 micrograms) and the purified pCBH5 DNA fragment (0.24 micrograms), were ligated under sticky-end ligation conditions at a total DNA concentration of about 60 micrograms per ml. The ligated linear DNA fragments were diluted to 20 micrograms per ml and ligation was continued under blunt-end conditions to favor intramolecular circle formation. E. coli K12 strain GM119 was transformed to ampicillin resistance with 150 ng of the ligated DNA and non-constitutive Lac + colonies were screened for the presence of the desired 11.94 kilobase plasmid, ptrpCBH5. Plasmid ptrpCBH5, shown in Figure 8, contained the desired insert with a new unique Xmal recognition site at the repaired pDG151 Xmal site. The plasmid construction shown in Figure 8 was confirmed by restriction enzyme mapping. Plasmid ptrpCBH5 in E. coli K12/GM119 has been deposited in the Cetus Master Culture Collection (CMCC) and has CMCC deposit = 1842. The plasmid has been sent to the Northern Regional Research Laboratory (NRRL), Peoria, IL for deposit, and has the deposit No NRRL #B15574.

### D.8.a. Construction of ptrpCBH8

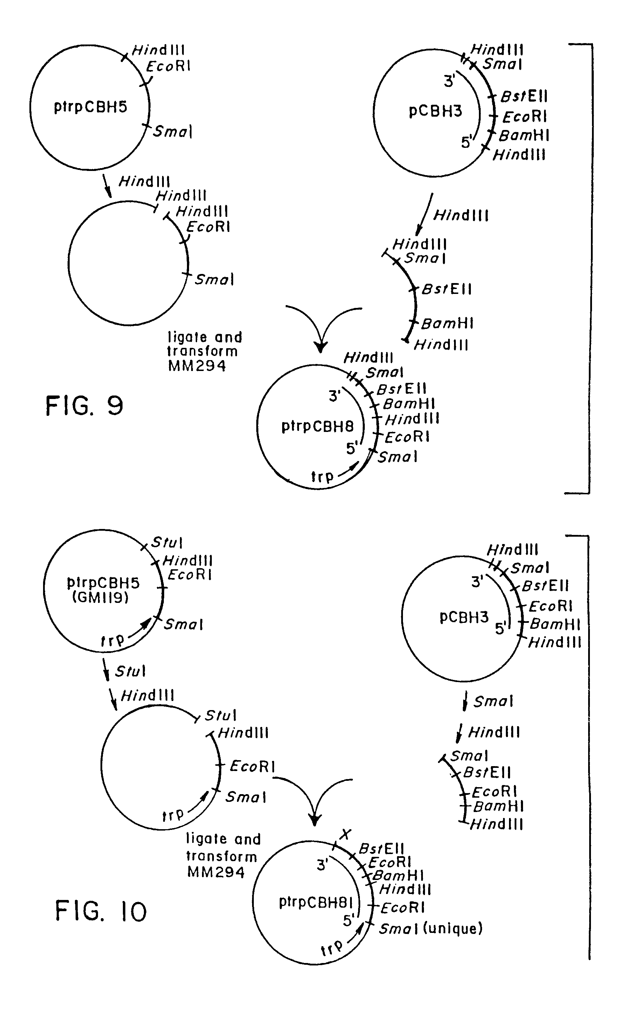

The procedure for subcloning the 3' cDNA gene fragment from pCBH3 into ptrCHB5 is illustrated in Figure 9. Plasmid pCBH3 was digested to completion with Hindlll, releasing an approximately 800 base pair 3' CBHI coding fragment which spans from the internal Hindlll site of the CBHI gene past the 3'-end of the gene adjacent the Smal site, to include a sequence from pUC8 adjacent the 3' Hindlll site. Plasmid ptrpCBH5 was also digested with Hindlll to linearize the vector at the Hindlll site immediately preceding the initiation codon at the APH-I gene. The purified pCBH3/Hindlll fragment (0.2 micrograms) was ligated under sticky end conditions with the ptrpCBH5/Hindlll fragments (1.6 micrograms). E. coli K12 strain MM294 was transformed to ampicillin resistance, and colonies were screened for the presence of the desired plasmid. The construction of the new plasmid, designated ptrpCBH8, is shown in Figure 9. Restriction mapping with Hindlll, BamHl and EcoRl confirmed the construction shown. Plasmid ptrpCBH8 contains two Xmal/Smal sites flanking the CBHI coding sequence. Digestion of ptrpCBH8 with either enzyme yields a 1.63 kilobase DNA CBHI gene fragment containing the entire CBHI coding region about 12 base pairs at the 5'-end of the coding region and about 80 base pairs at the 3'-end of the coding region. Plasmid ptrpCBH8 in E. coli K12/MM294 is identified as CMCC #1841, and has been deposited with NRRL under the No. NRRL #B15573.

### D.8.b. Construction of ptrpCBH81

The procedure for inserting the 3' CBHI cDNA fragment from pCBH3 into ptrpCBH5 is as follows: pCBH3 was treated with Smal and Hindlll to release a 734 base pair fragment containing the entire coding region of the CBHI gene downstream of the internal Hindlll site. The shuttle vector ptrpCBH5 (prepared in GM119, an E. coli K12 dam- host) was treated successively with endonucleases Stul and Hindlll to release a Hindlll/Stul vector fragment. The purified CBH3 Smal/Hindlll fragment from pCH3 was ligated under sticky-end then blunt end conditions with the ptrpCBH5 Stul/Hindlll fragments. E. coli K12/MM294 was transformed to ampicillin resistance, and colonies were screened for the presence of the desired plasmid. The construction of the new plasmid, designated ptrpCBH81 is shown in Figure 10. Plasmid ptrpCBH81 in E. coli K12/MM294 is identified as CMCC #1843, and has been deposited with NRRL under the No NRRL #B15575.

As indicated in Figure 10, the Smal/Stul fusion does not regenerate an Smal site in the new vector. The ptrpCBH81 plasmid thus has a unique Smal/Xmal site 12 base pairs preceding the initiation (methionine) codon of the CBHI intron-free gene, and 16 base pairs following the trp leader ribosome binding site.

### D.8.c Construction of ptrpCBH82

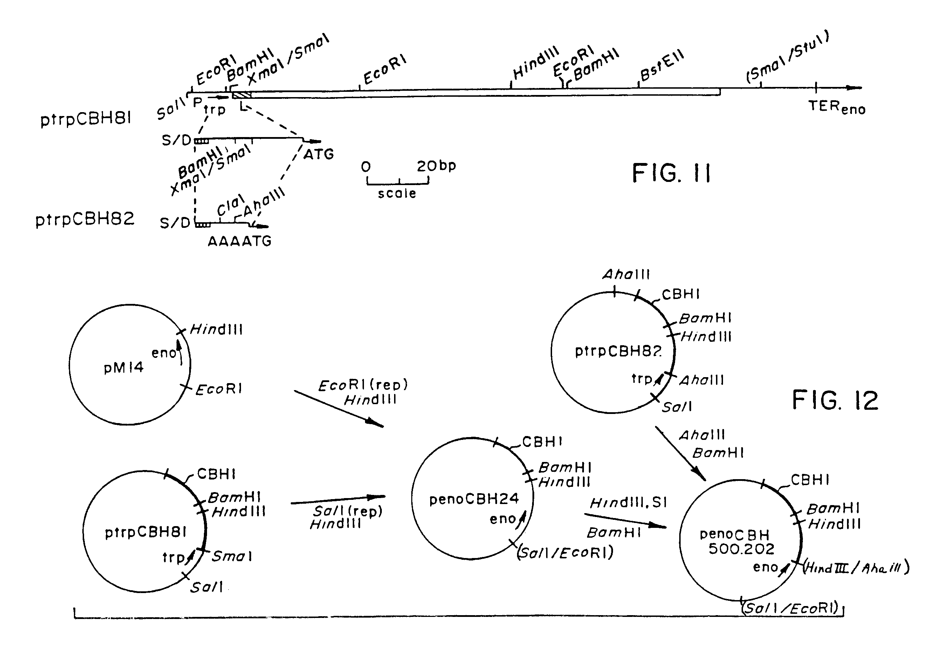

The plasmids ptrpCBH8 and ptrpCBH81 described in paragraphs D.8.a and D.8.b above were further modified to decrease the spacing between the trp ribosome binding site (RBS) and the ATG start codon of the desired CBHI encoding sequence. As detailed below, 23 nucleotides of the intervening sequence between the RBS and ATG initiating codon in a ptrpCBH8 fragment containing this sequence were replaced by a seven nucleotide segment, and the resulting modified fragment substituted for the corresponding portion of ptrpCBH81. The altered sequence further contains overlapping Clal and Ahalll recognition sites which replace BamHl and Xmal sites previously contained in this region. The replacement procedure is described below with reference to Figure 11.

### D.8.c.1. Modification of the pre-ATG sequence

A Sall/Pstl DNA fragment was excised from ptrpCBH8 by double digestion with these enzymes. This fragment extends from the Sall site on the 5' side of the pDG151 trp promoter (Figure 5) to the Pstl site adjacent the 3' side of the CBH gene (Figure 7). The fragment was isolated by agarose gel electrophoresis and recovered by electroelution.

Bacteriophage M13mp10w DNA was treated with Sall, followed by digestion with Pstl to release a Sall/Pstl phage fragment. Bacteriophage M13mp10w is a conventional phage of the type used for site mutagenesis; comparable types of phage are available from New England Biolabs (Beverly, MA). The Sall/Pstl fragment was isolated by agarose gel electrophoresis and recovered by electrolution.

The ptrpCBH8 and M13mp10w Sall/Pstl fragments prepared above were ligated under standard conditions and transfected into frozen competent E. coli K12 strain DG98. The cells were plated on media containing 5 X 10-⁴ M isopropyl thiogalactoside (IPTG) obtained from Sigma Chem. (St. Louis, MO.) and 40 µg/ml X-gal . Non a-complementing white plaques (16/405, 4%) were picked into fresh media. Mini cultures were screened for recombinant single strand phage DNA of the expected (9 kb) size. The structure of the desired recombinant phage, designated A6, was confirmed using restriction analysis.

A chemically synthesized, purified, oligodeoxyribonucleotide having the sequence: 5'-CAACCTCOGATACATTTTAAATOGATACCCTTTTTAC-3' was radiolabelled according to a modification of the technique of Maxam and Gilbert (Maxam, A., et al., Methods in Enzymology (1980) 68: 521, Academic Press). Briefly, 87 picomoles (2900 Ci/mmol) of ³²p-ATP obtained from New England Nuclear, were dried and combined with 50 picomoles of the oligomer and 12U of T4 polynucleotide kinase in a total volume of 30 µl containing 40 mM Tris-CI, pH 7.6, 10 mM MgCl₂, 5 mM DTT, 0.1 mM spermidine, and 0.1 mM EDTA. The mixture was incubated for one hour at 37 °C, another 12U of kinase was added and the reaction was continued for an additional hour. The crude mixture was purified by PAGE using a 1 mm, 16% acrylamide gel, electrophoresed at 500V for 30 min. with cooling. The band with the slowest migration was excised, crushed, and eluted twice with 0.5 ml hybridization buffer after heating at 100 ° C for 10 min. The specific activity of the solution was 5.3 x 10⁶ dpm/pm (75% incorporation).

Recombinant M13mp10w bacteriophage A6 was prepared in E. coli K12 strain DG98 and the single strand phage DNA purified. One pmole of single strand phage DNA and 10 pmoles of the above synthetic nucleotide primer (not phosphorylated) were annealed by heating for 1 min. at 67 ° C, and then 30 min. at 37°C in 15 µl 20 mM Tris-CI, pH 8, 20 mM MgC1₂, 100 mM NaCI, 20 mM 2-mercaptoethanol. The annealed DNA was incubated with DNA polymerase I (Klenow) and 500 µM dNTPs for 30 min., 0 °C and then brought to 37 °C. Aliquots (0.05 or 0.25 pmole) were removed after 5 min., 20 min., and 45 min., transformed into E. coli K12 strain MM294 and plated with E. coli K12 strain DG98.

The plates we̅r̅e̅ c̅h̅i̅l̅led at 4° C and plaques lifted with P̅a̅l̅l̅ membranes obtained from Biodyne (1-2 min. in the first filter, more than 10 min. for the second filter. The filters were denatured in 2.5 M NaCI, 0.5 M NaOH (5 min.). The denaturing medium was neutralized with 3 M sodium acetate to pH.5.5, the filters baked at 80 °C in vacuo for one hour, and then prehybridized in 6X SSC, 5X Denhardt's solution, 0.1% SDS, 50 µg/ml yeast t-RNA at 54.4°C, two hours. The filters were then probed with 6.5 x 106cpm/1.25 pmole of the phosphorylated synthetic oligonucleotide/2.5 ml/filter at 50.5 °C, overnight, washed twice in 6X SSC, 40 °C, 5 min., and once in 6X SSC, 50.5 °C, 5 min. and autoradiographed overnight at -80 ° C.

Nucleotides 1-15 of the above synthetic oligonucleotide are complementary to the 5'-end sequences of the CBHI gene, and nucleotides 23-37, to the control region between the S/D sequence and the adjacent BamHl site in ptrpCBH8. As seen in the expanded portion in Figure 11, the desired recombinant phage, in which the oligonucleotide primer has replaced an end-homologous ptrpCBH81 region, has lost BamHl and Xmal sites, and acquired Clal and Ahalll sites derived from the central portion of the oligonucleotide. In particular the modified phage contains a TTTAAA Ahalll recognition site immediately adjacent the ATG start codon of the CBH gene, as indicated in the lower expanded portion of the figure.

Candidate plaques were picked and replicative form (RF)-DNA analyzed, following infection of E. coli K12 strain DG98, for acquisiton of the new Clal and Ahalll recognition sites and loss of an Xmal recognition site. One candidate showing the appropriate analysis was designated mp10w5A.

### D.8.c.2. Replacement into ptrp CBH81

Each of the plasmids ptrpCBH81 and mp10w5A RF-DNA were digested to completion with Sall and BamHl, and the digest ligated under standard conditions. The ligation mixture was digested with Xmal to inactivate undesired ligation products and used to transform E. coli K12 MM294 to Amp^{R}. Candidate plasmids were screened by restriction analysis to release: a diagnostic 1.26 kb DNA fragment with Sall/BamHl digestion; a 1.07 kb DNA fragment by Clal/BamHl digestion; and a 1.07 kb DNA fragment by BamHl/Ahalll digestion. The correct construction, designated ptrpCBH82 in Figure 11, was confirmed by sequencing.

### 9. Construction of Yeast Expression Plasmids Encoding CBHI

Several plasmids were constructed placing the CBHI coding sequences under the control of the EN01 promoter, retaining the vector backbone features of the bacterial/yeast replicating plasmids of subparagraph D.8 derived from pDG151. The construction of one of these vectors, designated penoCBH500.202, is illustrated in Figure 12.

Plasmid pPM14 was used as a source of the EN01 promoter. pPM14 is a derivative of pBR322 containing a 722 bp EcoRl/Hindlll fragment from peno46 (Holland, M. J., et al, J. Biol. Chem. (1981) 256:1385) which encompasses a fragment running from 723 bp preceding the enolase-I translation initiation site to nucleotide -2 of the enolase ATG initiation codon. pPM14 was digested with EcoRl, treated with Klenow in the presence of dATP and dTTP, and then digested with Hindlll to provide the EcoRI(blunt)-/Hindlll EN01 promoter containing fragment.

Plasmid ptrpCBH81 was digested with Sall, treated with Klenow in the presence of all four dNTP's, and then digested to completion with Hindlll.

The fragments prepared in the previous paragraphs were ligated at an equimolar ratio under sticky end conditions, followed by blunt end ligation, and the ligation products were treated with Nrul and Smal to inactivate unwanted ligation products. The ligation mixture was used to transform E. coli K12 MM294 to Amp^{R} and the transformants were screened for the presence of the desired 11.32 kb plasmid, designated penoCBH24. The correct construction was confirmed by restriction analysis.

With continued reference to Figure 12, ptrpCBH82 was digested with Ahalll and the 2.2 kb DNA fragment which encodes CBHI was purified using agarose gel electrophoresis. The two Ahalll sites cleaved by this procedure are (1) the Ahalll site introduced adjacent the 5' end of the CBHI gene, as just described and (2) an Ahalll site contained in the pDG151 vector region which borders the 3' end of the CBHI gene in ptrpCBH81 and ptrpCBH82. This fragment was then digested with BamHl yielding two subfragments, a 1068 bp Ahalll/BamHl fragment encoding the 5' CBHI sequences and a 1.13 kb BamHl/Ahalll fragment encoding the 3' CBHI sequences, and which further contains a stretch of bacterial DNA and the 3' untranslated EN01 sequences.

Plasmid penoCBH24 was digested with Hindlll, treated with SI nuclease, and then digested with BamHl. The foregoing fragments and the vector DNA fragment from penoCBH24 were ligated at a 2:1 molar ratio under sticky end conditions, then under blunt end conditions. The ligation mixture was used to transform E. coli K12 strain MM294 to Amp^{R} and successful transformants screened for the desired 12.2 kb plasmids containing 5' CBHI sequences. Successful candidates, which were identified by release of a 2.05 kb fragment upon digestion with EcoRl, and a 200 bp fragment upon digestion with Haelll, were designated penoCBH500.X, where X is an indicator number specified below. The plasmids were further analyzed by dideoxy DNA sequence analysis using an internal octadecamer primer which is complementary to nucleotides 31-48 of the mature CBHI coding sequence.

Plasmid penoCBH500.202 contained the sequence: 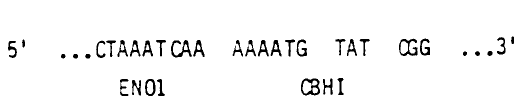 indicating a precise elimination of the Hindlll cohesive end and fusion to the Ahalll/CBHI DNA fragment.

Plasmids penoCBH500.108 and penoCBH500.150 contained identical DNA sequences: 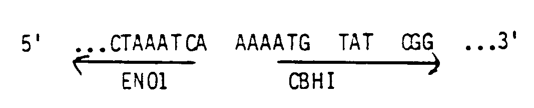 indicating removal of one A/T base pair during the SI nuclease treatment.

Plasmid penoCBH500.212 contained the sequence: 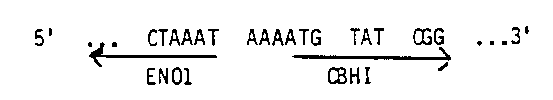 indicating removal of two A/T base pairs and one C/G base pair from the EN01 promoter fragment.

### D.10. Expression of Cloned CBHI Gene in Yeast

Yeast strain S. cerevisiae S173-6B was transformed with penoCBH500.202 and selected for LEU transformants. A successful transformant colony was cultured and grown in a 10 liter fermenter, using glucose as a carbon source on minimal medium. The culture was grown to an optical density (OD₆₈ₒ) of 10. The contents of the fermenter were filtered, and the liquid fraction was concentrated and dialyzed against water.

The approximately 10 I of medium obtained was reduced to 500 ml and further purified by applying the material to a DEAE sepharose column according to the protocol described in paragraph D.2. above. Of 500 mg protein applied to the column, about 437 mg was recovered in the non-absorbed fraction, and about 100 mg, or 18%, was eluted in a single peak corresponding to CBHI.

### 10.c. Characterization of CBHI Recombinantly Produced in Yeast

The purified, secreted CBHI (recombinant CBHI) was characterized and compared with native T. reesei CBHI which was isolated according to paragraph D.2 above. The results are summarized in Table II below.

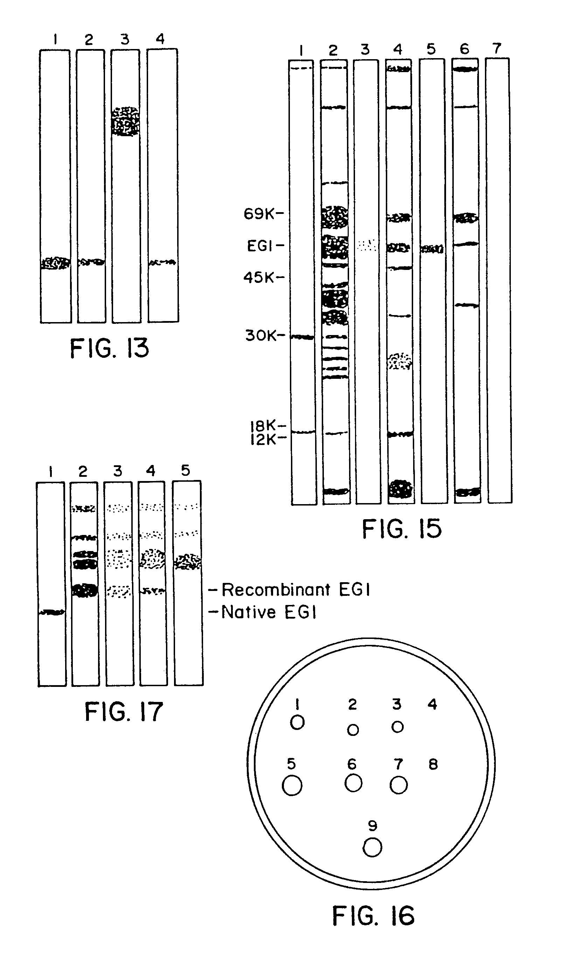

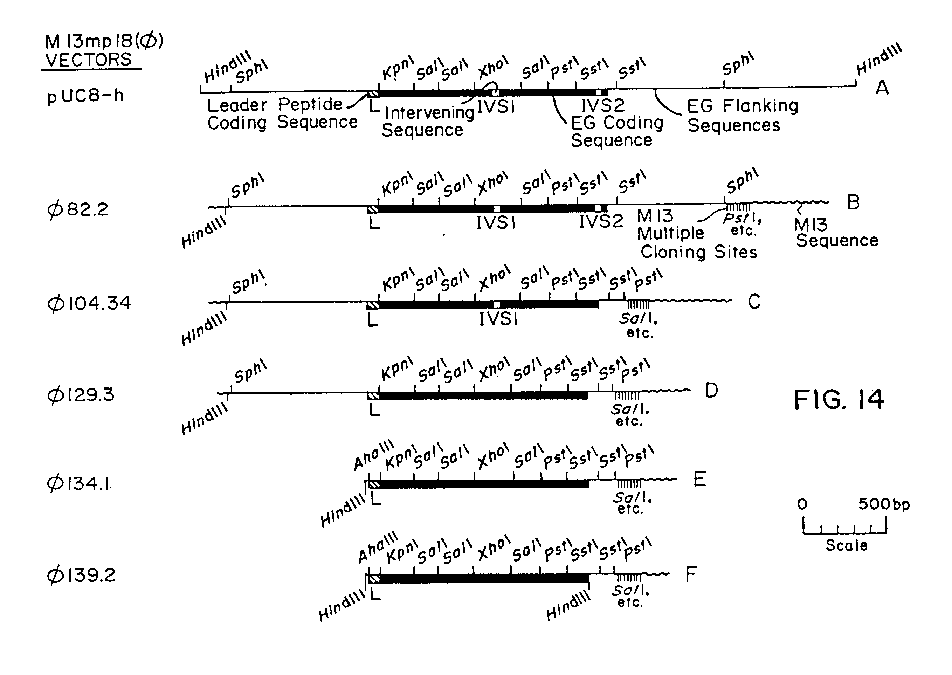

Western blot analysis of recombinant and native CBHI was performed according to the standard procedures. Lanes 1 and 3 in Figure 13 show the gel patterns of native and recombinant CBHI, respectively. As indicated in Table II, the blot analysis indicates that the apparent molecular weight of native CBHI is about 60K daltons and that of recombinant CBHI, between about 100K-200K daltons.

The two enzymes were treated with the glycosidase endo-H, which acts to deglycosylate proteins at N-linked glycosyl residues. The deglycosylated native and recombinant proteins were examined as above by Western blot analysis, with the results shown in lanes 2 and 4, respectively, of Figure 13. As seen both deglycosylated proteins have roughly the same molecular weight, indicating that recombinant CBHI, unlike native CBHI, is highly glycosylated by large N- linked residues. Native CBHI by contrast, is known to contain mainly O-linked residues, as noted in Table II. The much greater apparent molecular weight of glycosylated recombinant CBHI is attributed to the greater length of N-linked sugar residues, as compared with the 1-2 sugar residues per site with O-linked glycosylation in the native enzyme.

The CBHI activity of the purified native and recombinant enzymes was assayed conventionally for formation of cellobiose from phosphoric acid-swollen cellulose. As seen from Table II, native CBHI has a specific activity, based on a Lowry protein determination, of about 0.57 units/mg, as compared with 0.34 units/mg for recombinant CBHI. The higher (0.46 units/mg) specific activity value for recombinant CBHI was calculated on the basis of the protein amino acid composition. As seen, recombinant CBHI produced according to the present invention has nearly the same specific activity as native CBHI from T. reesei.

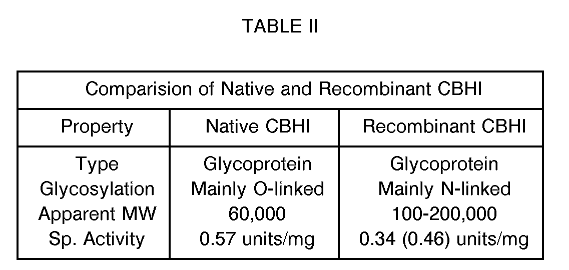

To confirm the identity of the recombinant CBHI, the amino acid sequence of an amino-terminus fragment (residues 1-37) and an internal fragment (residues 225-251) were determined, using methods described above in paragraph D.2. The two sequences were each identical in amino acid composition to corresponding fragments of native CBHI. Interestingly, the recombinant protein contained the same amino-terminal pyroglutamine as the mature native CBHI, indicating identical processing, of the recombinant enzyme involving (1) removal of the leader polypeptide and (2) cyclization of the amino-terminal glutamine residue.

In summary, a gene encoding CBHI cellulase has been cloned and expressed in yeast, and by use of suitable control sequences is adaptable for expression in a variety of hosts. The availability of recombinant CBHI offers the possibility of more efficient use of cellulose waste products and conversion of these into useful derivatives, including, ultimately, glucose and ethanol and for specific modification of cellulose materials.

### E. Preparation of Recombinant EGI

This section illustrates the construction of a recombinant EGI gene, and its expression in yeast. The exemplary EGI coding sequence was constructed as a Hindlll cassette, and ligated into a yeast expression vector in operable linkage to the yeast enolase control sequences.

### E.1. Preparation of mRNA Enriched for EGI and Synthesis of cDNA

PolyA-RNA was obtained from cellulose-induced T. reesei strain L27, and fractionated by agarose electrophoresis substantially as described in paragraph D.3 above.

Antibodies specific against EGI were prepared using purified EGI according to standard techniques. Briefly, purified EGI obtained by the method disclosed by Shoemaker, S., et al., Biotechnology (October, 1983) 687 was mixed with Freund's adjuvant and 0.2-0.4 ml of the mixture injected intramuscularly into New Zealand white rabbits, repeating the injection every 10 days until antibody titers were detectable by the double immunodiffusion method of Ouchterlony, O., Arkiv Kemi (1949) 1:43.

To verify the correct mRNA fraction, a small amount of each RNA fraction was added to the cell-free protein-synthesizing system sold as a reticulocyte lysate/methionine L-(35S)-translation system by New England Nuclear Company, Boston, MA. The presence or absence of EGI synthesized by the system was verified by SDS-PAGE of total protein synthesized and of protein from the translation system immunoprecipitated with, and then dissociated from, anti-EGI antibody.

### E.2. Preparation of a cDNA Probe

The mRNA fraction which, in a cell-free translation system, results in the synthesis of high levels of EGI, was selected and used to generate a single stranded cDNA, substantially as described in paragraph D.4 above. The single stranded DNA was size analyzed by gel electrophoresis; a major band corresponding to the expected cDNA size was observed.

### E.3. Preparation of a Double Stranded cDNA Library

The mRNA fraction prepared from paragraph E.1 was also used to create a cDNA library according to the method of Maniatis, et al., Molecular Cloning-A Laboratory Manual (1982) Cold Spring Harbor Laboratory, Cold Spring Harbor, NY, pp. 235-238. The thus obtained double stranded cDNA was then cloned into a pUC-8 cloning vector according to standard procedures, and probed using a 4 kb Hindlll genomic fragment (described above) known to encode EGI.

Three cDNA clones hybridizing to the probe -- pcEG10, pcEG9, and pcEG1 -- were selected for further study. They were analyzed by restriction analysis and by sequencing of the cDNA region using the dideoxy method. pcEG10 was found to encode the 3' region of the sequence, and pcEG1, to encode the 5' portion.

### E.4 Preparation and Sequencing of the EGI Gene

A genomic library from T. reesei strain L27 in lambda phage was prepared as described by Shoemaker, S., et al., Bio Technology (October, 1983) pp. 691-696, incorporated herein by reference, and as further described in paragraph D.5 above. The lambda genomic library was screened as described in Shoemaker, S., et al., (supra) but using the single stranded cDNA prepared in paragraph E.2, as probe. One fragment from a positively reacting phage was subcloned into pUC8 to give pUC8-h. This vector includes a 4 kb Hindlll fragment spanning the entire EGI gene.

The coding sequence was localized within this fragment by restriction analysis and hybridization to 5'- end synthetic oligonucleotides and 3'-end cDNA probes. The DNA was digested with Kpnl, Sstl, Xbal, and Sphl in tandem with EcoRl, and subjected to Southern blot analysis (Southern, J. Mol. Biol. (1975) 98:503), using as a probe a 16mer corresponding to amino acid residues 16-20 of the mature EGI sequence, with 4 nucleotide ambiguities. The results indicated that a major portion of the gene was located between the Kpnl and Sstl site. A restriction map of the pUC8-h genomic DNA is shown in Figure 14, frame A.

The identity of a portion of the 4 kb Hindlll sequence with the EGI coding sequence was further confirmed by a restriction/selection experiment. The 4 kb fragment was bound to a nitrocellulose filter according to the procedure of Harpold, M. H., et al., Nucleic acids Res. (1978) 5:2039. Total mRNA from induced cultures was added to the filters and mRNA which bound under stringent conditions separated by washes from unbound mRNA. The filter-bound mRNA was then eluted. For each filter, bound and unbound mRNA fractions were translated in the cell free protein synthesizing system and the resulting synthesized proteins precipitated with a mixture of anti-EGI antibodies and Staphylococcus aureus cells. The protein from the immunoprecipitate was subjected to SDS-PAGE, and the results of the experiment are shown in Figure 15. Lane 1 in the figure shows some of the molecular weight markers which were run in parallel. Lane 2 is a gel of total polyA-RNA translation products produced in the cell-free synthesizing system. The anti-EGI immunoprecipitate of the total translation products is displayed in lane 3, showing a single band corresponding in molecular weight to EGI. The gel pattern of translation products of polyA-RNA which hybridized to the probe is seen in lane 4, which shows a major band corresponding to the molecular weight of EGI. The lane 4 material, after immunoprecipitation by anti-EGI antibody, gave the single band shown in lane 5 having the expected molecular weight for EGI. Lanes 6 and 7 show, respectively, are total and an anti-EGI immunoprecipitate of translation products directed by polyA-RNA fraction which did not hybridize the 4 kb Hindlll fragment. As seen from the figure, only the mRNA that bound to the filter containing the 4 kb Hindlll fragment was capable of directing the synthesis of protein which was immunoprecipitated by anti-EGI.

The entire coding sequence of the EGI gene was then obtained using standard restriction and sequencing techniques. The results are shown in Table III below. The sequenced fragment contains 1697 nucleotides, including 66 nucleotides for a 22 amino acid leader sequence preceding the coding sequence for the 437 amino acid residues of the mature protein, (as deduced from the known N-terminal sequence of the secreted purified EGI.) The DNA sequence also contains two introns at nucleotide positions 893 through 962 and 1553 through 1609. The intron positions were deduced by comparison of the coding sequences with those obtained by analogous sequencing of the cDNA plasmids pcEG10 and pcEG1, and by comparison with the known amino acid sequence (also shown in the table). The coding sequence corresponding to the leader peptide (amino acids 1-22) provides a leader sequence for initiating secretion of EGI, and is referred to herein as the EGI leader sequence. The amino acid sequence in Table III, in contiguous form, is the polypeptide sequence of EGI.

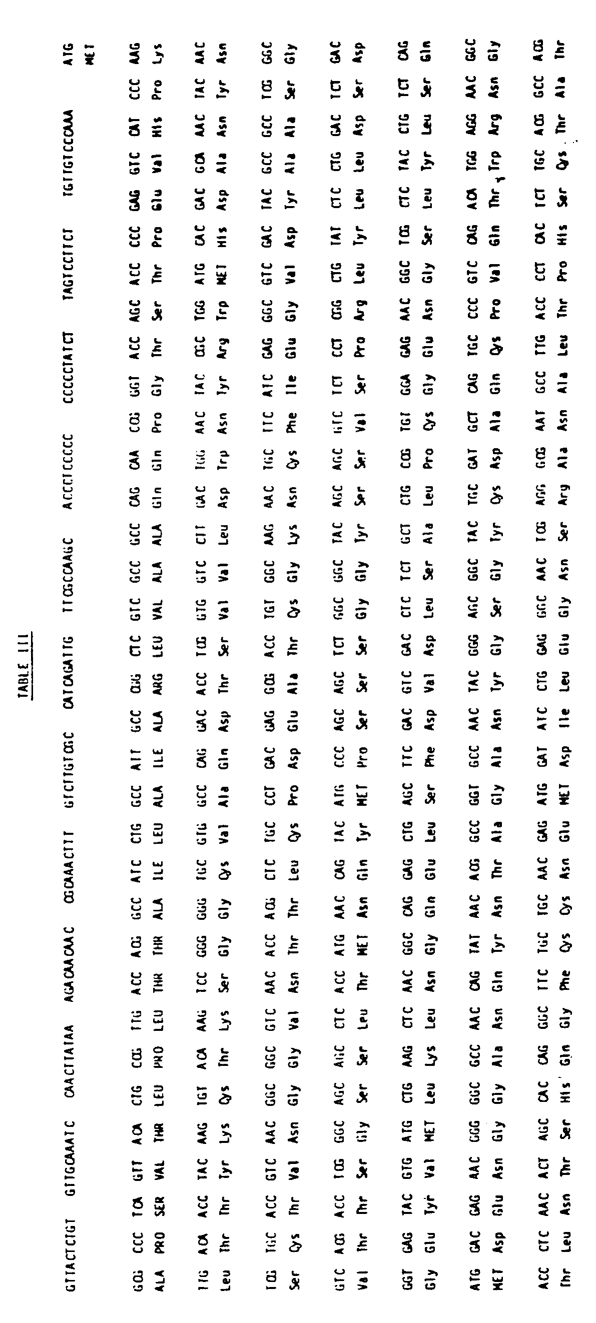

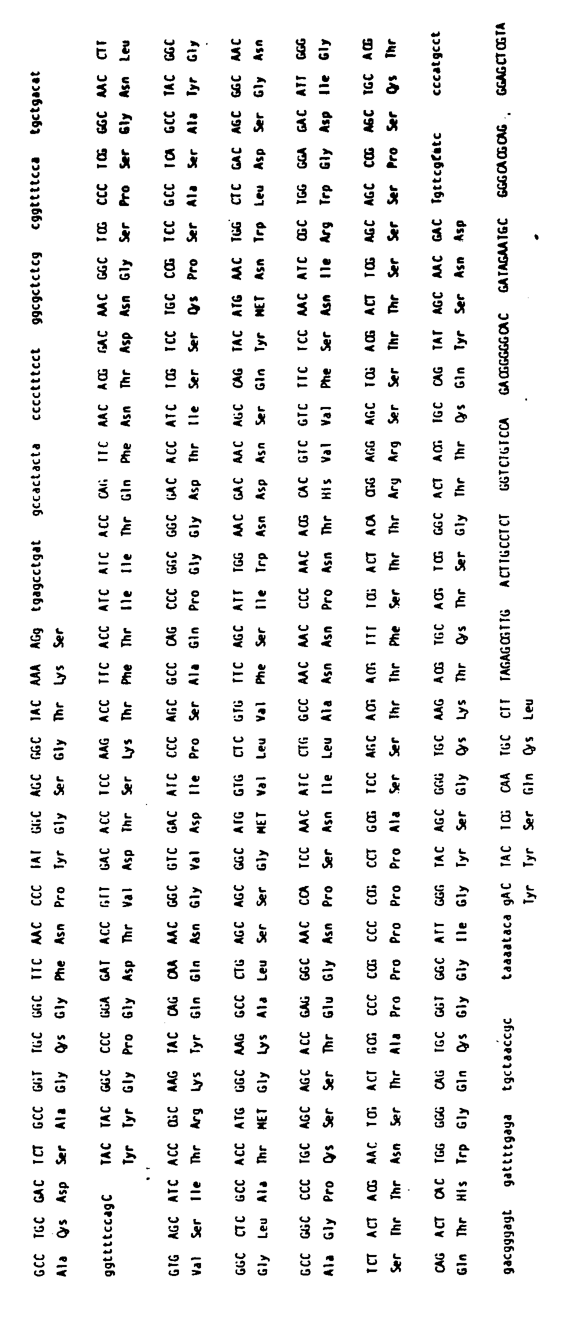

### E.5. Construction of an Intronless EGI Coding Sequence

Plasmid pUC8-h from paragraph E.4 was digested with Sphl, and the Sphl fragment was purified on agarose gel. The isolated fragment was cloned into Sphl-digested M13mp8-DNA according to standard methods. The ligated phage-DNA was transformed into competent E. coli K12 strain DG98 and cells plated in the presence of IPTG (5 x 10-⁴ M) on X-gal (40 g/ml) containing plates. Non a-complementing white plaques were screened for recombinant single strand phage DNA of the expected 10.3 kb size. The structure of the desired recombinant phage, designated JV82.2, was confirmed using restriction analysis. This construction is shown in frame B of Figure 14, where the vector is designated $82.2.

The downstream intron was excised by replacing the 3' portion of the genomic clone with a corresponding portion of a cDNA clone prepared from the enriched mRNA, and characterized by restriction analysis. Specifically, JV82.2 was digested to completion with Pstl, and the digest ligated with a Pstl digested fragment isolated from pcEG10, to obtain JV104.34,(Φ104.34) illustrated in frame C of Figure 14. The Pstl fragment from pcEG10 spans the coding region corresponding to that in the genomic clone which includes the portion containing the downstream intron. That is, JV104.34 contains the entire coding sequence of the EGI gene with the downstream intron removed.

To remove the upstream intron, site-directed mutagenesis was employed. An oligonucleotide primer complementary to the sequences framing the first upstream intron, but lacking the intron sequences was synthesized. The primer had the sequence 5'-GGCAGCGGCTACCAAAAGCTACTACGGCCCCGGAGA-3'. The recombinant M13mp19 bacteriophage JV104.34 was prepared in E. coli K12 strain DG98 and the single strand phage-DNA was purified conventionally. The single strand phage-DNA was used as a template for DNA synthesis to effect site-specific mutagenesis using the synthesized oligomer as primer, according to standard procedures. The successful bacteriophage candidate, JV129.3, (Φ129.3) obtained by screening with radiolabelled oligomer as probe, is illustrated in frame D of Figure 14. JV129.3 contains the entire uninterrupted EGI coding sequence in proper reading frame.

The intronless gene was further refined, as shown in frames E and F of Figure 14, to provide Hindlll sites immediately upstream and downstream of the coding sequence thus permitting its manipulation as a Hindlll cassette. To supply the upstream Hindlll site, the synthetic oligonucleotide 5'-CTTAGTCCTTCTTGAAGCTTTAAAATGGCGCCCT-3', which is complementary to the sequence immediately upstream of the coding sequence, but with 6 mismatches to create a Hindlll site immediately 5' of the ATG translation initiation codon was used to replace the native upstream sequences by site-specific mutagenesis as above. The resulting phage-DNA was further treated with Hindlll and religated to delete the portion of DNA between the new 5' proximal Hindlll and the upstream site shown in Figure 14, frames A-D yielding JV134.1 ($134.1 in Figure 14, frame E).

Similarly, the synthetic nucleotide 5'-AATGCCTTTAGAAGCTTGACTTGCCT-3' was used in the site-specific mutagenesis procedure to create the M13 vector containing the EGI gene as a Hindlll cassette, designated JV139.2 ($139.2 in Figure 14, frame F).

### E.6. Construction of Expression Vectors Containing the EGI Coding Sequence

An expression vector useful in yeast containing the EGI sequences under the control of the enolase-1 promoter and enolase-1 terminator was constructed by ligating the Hindlll cassette into the Hindlll site of the yeast host vector pMAC101.

pMAC101 is a 10.2 kb vector containing a Hindlll cleavage site disposed between a modified yeast enolase-1 (EN01) promoter sequence and a yeast enolase-1 (EN01) terminator sequence. The vector also contains the yeast LEU2 gene, a yeast 2 µ, origin of replication and the origin of replication and Amp^{R} portions of pBR322. pMAC101 can be obtained readily from vector pJV160, described below, by removing a Hindlll gene cassette from that vector.

To construct the expression vector for EGI in yeast, designated pJV160, pMAC101 was digested to completion with Hindlll, and ligated to the Hindlll cassette excised from the replicative form (RF) of JV139.2. The ligation mixture was used to transform yeast strain C468. The successful transformants were selected for LEU⁺, and positive candidates were assayed by their ability to clear an overlay of carboxymethylcellulose (CMC) as described below. (Of the cellulase-complex enzymes, only endoglucanases have the appropriate specificity to hydolyze carboxymethyl cellulose. Cellobiohydrolases are specific for cellulose per se and fail to hydrolyze CMC.)

### E.T. Confirmation of Expression of EGI in Yeast

After selection for LEU2 ⁺, the three successful transformants were assayed for EGI formation, substantially according to the method of Teather, R., et al., Appld. Envir. Micro. (1982) 43:777. Colonies were grown on minimal plates at 30 C, and overlayed with top agar (0.8%) containing 0.5% CMC, 15 mM NaOAc, pH 4.5 and 10 mM sodium azide. The overlay plates were then incubated at 37°C for 4 hrs. To visualize clearing of the CMC, the plates were treated for 15 min. with 1 mg/m1 Congo Red and for 5 min with 1 M NaC1 followed by 5 min. with 1 M HC1. To test the ability of the three yeast transformants to produce secreted, functionally active EGI, a yeast culture supernatant from each of the three transformants, as well as from a yeast transformed by pMAC101 (control) was prepared. The supernatant samples (either 1x or 20x concentrated) were spotted on CMC plates and assayed for EGI activity as described above. The results are shown in Figure 16. Spots 1-4 are 1x concentrates of the three EGI (1-3) and control (4) transformants, and spots 5-8 are the corresponding 20x concentrates. As seen, all of the EGI transformants, but not the control transformant, secrete active EGI. Spot 9 was produced by native EGI from T. reesei.

Verification of the expression of EGI was also made by Western blot analysis. Concentrated culture supernatants from three yeast cultures which showed EGI activity by the previous assay were run on SDS-PAGE, transferrred to nitrocellulose, and probed with anti-EGI antibody, followed by staph A protein radiolabelled with ¹²⁵₁.

Lane 1 in Figure 17 shows native, purified EGI from T. reesei. Lanes 2, 3, 4 are gel patterns of the supernatant from each of the three EGI yeast transformants. As seen, each of the EGI transformants secretes a polypeptide having a molecular weight somewhat greater than that of native EGI. This band was not seen in the supernatant from yeast transformed with pMAC101. The greater molecular weight of recombinant EGI is presumably due to differences from native EGI in post translational processing, similar to what has been described for CBHI.

Techniques used for the successful expression of EGI in yeast, described above, are applicable, by suitable modifications in vector control sequences, for achieving expression in a variety of hosts, including both bacterial and yeast hosts.

Particularly in combination with recombinant CBHI, the availability of recombinant EGI offers the possibility of more efficient use of cellulose products and conversion of these into useful derivatives, and/or selective modification of cellulosic materials. A cellulose degrading system containing CBHI, EGI and optionally, {3-glucosidase, may be constructed from individually isolated recombinant enzymes or by using a multi-host system which contains the combination of cellulases.

In addition to the ATCC and NRRL deposits identified above, the following materials have been deposited at the American Type Culture Collection, 12301 Parklawn Drive, Rockville, MD, U.S.A. (ATCC) under the terms of the Budapest Treaty on the International Recognition of the Deposit of Microorganisms for the Purposes of Patent Procedure and Regulations thereunder (Budapest Treaty) and are thus maintained and made available according to the terms of the Budapest Treaty. Availability of such strains is not to be construed as a license to practice the invention in contravention of the rights granted under the authority of any government in accordance with its patent laws.

The deposits have been assigned the indicated ATCC deposit numbers, and have also been deposited with the Master Culture Collection (CMCC) of Cetus Corporation, Emeryville, CA, U.S.A., the assignee of the present application, and assigned the indicated CMCC deposit numbers:

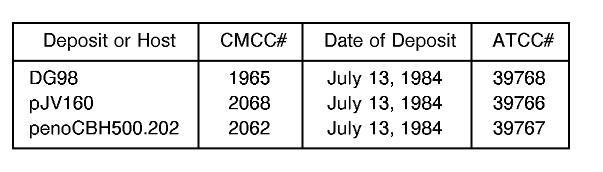

## Claims (Claims for the following Contracting State(s) : BE, CH, DE, FR, GB, IT, LI, NL, SE)

1. A DNA sequence encoding an amino acid sequence of a leader peptide-containing fungal cellulase, said amino acid sequence being
(a) the amino acid sequence 
MET TYR ARG LYS LEU ALA VAL ILE SER ALA PHE
LEU ALA THR ALA ARG ALA GLN SER ALA CYS THR
LEU GLN SER GLU THR HIS PRO PRO LEU THR TRP
GLN LYS CYS SER SER GLY GLY THR CYS THR GLN
GLN THR GLY SER VAL VAL ILE ASP ALA ASN TRP
ARG TRP THR HIS ALA THR ASN SER SER THR ASN
CYS TYR ASP GLY ASN THR TRP SER SER THR LEU
CYS PRO ASP ASN GLU THR CYS ALA LYS ASN CYS
CYS LEU ASP GLY ALA ALA TYR ALA SER THR TYR
GLY VAL THR THR SER GLY ASN SER LEU SER ILE
GLY PHE VAL THR GLN SER ALA GLN LYS ASN VAL
GLY ALA ARG LEU TYR LEU MET ALA SER ASP THR
THR TYR GLN GLU PHE THR LEU LEU GLY ASN GLU
PHE SER PHE ASP VAL ASP VAL SER GLN LEU PRO
CYS GLY LEU ASN GLY ALA LEU TYR PHE VAL SER
MET ASP ALA ASP GLY GLY VAL SER LYS TYR PRO
THR ASN THR ALA GLY ALA LYS TYR GLY THR GLY
TYR CYS ASP SER GLN CYS PRO ARG ASP LEU LYS
PHE ILE ASN GLY GLN ALA ASN VAL GLU GLY TRP
GLU PRO SER SER ASN ASN ALA ASN THR GLY ILE
GLY GLY HIS GLY SER CYS CYS SER GLU MET ASP
ILE TRP GLU ALA ASN SER ILE SER GLU ALA LEU
THR PRO HIS PRO CYS THR THR VAL GLY GLN GLU
ILE CYS GLU GLY ASP GLY CYS GLY GLY THR TYR
SER ASP ASN ARG TYR GLY GLY THR CYS ASP PRO
ASP GLY CYS ASP TRP ASN PRO TYR ARG LEU GLY
ASN THR SER PHE TYR GLY PRO GLY SER SER PHE
THR LEU ASP THR THR LYS LYS LEU THR VAL VAL
 
THR GLN PHE GLU THR SER GLY ALA ILE ASN ARG
TYR TYR VAL GLN ASN GLY VAL THR PHE GLN GLN
PRO ASN ALA GLU LEU GLY SER TYR SER GLY ASN
GLU LEU ASN ASP ASP TYR CYS THR ALA GLU GLU
ALA GLU PHE GLY GLY SER SER PHE SER ASP LYS
GLY GLY LEU THR GLN PHE LYS LYS ALA THR SER
GLY GLY MET VAL LEU VAL MET SER LEU TRP ASP
ASP TYR TYR ALA ASN MET LEU TRP LEU ASP SER
THR TYR PRO THR ASN GLU THR SER SER THR PRO
GLY ALA VAL ARG GLY SER CYS SER THR SER SER
GLY VAL PRO ALA GLN VAL GLU SER GLN SER PRO
ASN ALA LYS VAL THR PHE SER ASN ILE LYS PHE
GLY PRO ILE GLY SER THR GLY ASN PRO SER GLY
GLY ASN PRO PRO GLY GLY ASN ARG GLY THR THR
THR THR ARG ARG PRO ALA THR THR THR GLY SER
SER PRO GLY PRO THR GLN SER HIS TYR GLY GLN
CYS GLY GLY ILE GLY TYR SER GLY PRO THR VAL
CYS ALA SER GLY THR THR CYS GLN VAL LEU ASN
PRO TYR TYR SER GLN CYS LEU
said amino acid sequence representing a cellobiohydrolase I; or
(b) the amino acid sequence 
MET ALA PRO SER VAL THR LEU PRO LEU THR THR
ALA ILE LEU ALA ILE ALA ARG LEU VAL ALA ALA
Gln Gln Pro Gly Thr Ser Thr Pro Glu Val His
Pro Lys Leu Thr Thr Tyr Lys Cys Thr Lys Ser
Gly Gly Cys Val Ala Gln Asp Thr Ser Val Val
Leu Asp Trp Asn Tyr Arg Trp MET His Asp Ala
Asn Tyr Asn Ser Cys Thr Val Asn Gly Gly Val
Asn Thr Thr Leu Cys Pro Asp Glu Ala Thr Cys
Gly Lys Asn Cys Phe Ile Glu Gly Val Asp Tyr
Ala Ala Ser Gly Val Thr Thr Ser Gly Ser Ser
Leu Thr MET Asn Gln Tyr MET Pro Ser Ser Ser
Gly Gly Tyr Ser Ser Val Ser Pro Arg Leu Tyr
 
Leu Leu Asp Ser Asp Gly Glu Tyr Val MET Leu
Lys Leu Asn Gly Gln Glu Leu Ser Phe Asp Val
Asp Leu Ser Ala Leu Pro Cys Gly Glu Asn Gly
Ser Leu Tyr Leu Ser Gln MET Asp Glu Asn Gly
Gly Ala Asn Gln Tyr Asn Thr Ala Gly Ala Asn
Tyr Gly Ser Gly Tyr Cys Asp Ala Gln Cys Pro
Val Gln Thr Trp Arg Asn Gly Thr Leu Asn Thr
Ser His Gln Gly Phe Cys Cys Asn Glu MET Asp
Ile Leu Glu Gly Asn Ser Arg Ala Asn Ala Leu
Thr Pro His Ser Cys Thr Ala Thr Ala Cys Asp
Ser Ala Gly Cys Gly Phe Asn Pro Tyr Gly Ser
Gly Thr Lys Ser Tyr Tyr Gly Pro Gly Asp Thr
Val Asp Thr Ser Lys Thr Phe Thr Ile Ile Thr
Gln Phe Asn Thr Asp Asn Gly Ser Pro Ser Gly
Asn Leu Val Ser Ile Thr Arg Lys Tyr Gln Gln
Asn Gly Val Asp Ile Pro Ser Ala Gln Pro Gly
Gly Asp Thr Ile Ser Ser Cys Pro Ser Ala Ser
Ala Tyr Gly Gly Leu Ala Thr MET Gly Lys Ala
Leu Ser Ser Gly MET Val Leu Val Phe Ser Ile
Trp Asn Asp Asn Ser Gln Tyr MET Asn Trp Leu
Asp Ser Gly Asn Ala Gly Pro Cys Ser Ser Thr
Glu Gly Asn Pro Ser Asn Ile Leu Ala Asn Asn
Pro Asn Thr His Val Val Phe Ser Asn Ile Arg
Trp Gly Asp Ile Gly Ser Thr Thr Asn Ser Thr
Ala Pro Pro Pro Pro Pro Ala Ser Ser Thr Thr
Phe Ser Thr Thr Arg Arg Ser Ser Thr Thr Ser
Ser Ser Pro Ser Cys Thr Gln Thr His Trp Gly
Gln Cys Gly Gly Ile Gly Tyr Ser Gly Cys Lys
Thr Cys Thr Ser Gly Thr Thr Cys Gln Tyr Ser
Asn Asp Tyr Tyr Ser Gln Cys Leu
 said amino acid sequence representing an endoglucanase I.

2. The DNA sequence according to claim 1 (a) which is
(c) the DNA sequence contained in the following sequence data: 
AAGCTTAGCC AAGAACAATA GCCGATAAAG ATAGCCTCAT TAAACGGAAT
1 HindIII 20 30 40 50

GAGCTAGTAG GCAAAGTCAG CGAATGTGTA TATATAAAGG TTCGAGGTCC
60 70 80 90 100

GTGCCTCCCT CATGCTCTCC CCATCTACTC ATCAACTCAG ATCCTCCAGG
110 120 130 140 150

AGACTTGTAC ACCATCTTTT GAGGCACAGA AACCCAATAG TCAACCGCGG
160 170 180 HincII SacII

MET TYR ARG LYS LEU ALA VAL ILE SER ALA PHE
ACTGGCATC ATG TAT CGG AAG TTG GCC GTC ATC TCG GCC TTC
209 224 BglI

LEU ALA THR ALA ARG ALA GLN SER ALA CYS THR LEU GLN SER
TTG GCC ACA GCT CGT GCT CAG TCG GCC TGC ACT CTC CAA TCG
254 269 284

GLU THR HIS PRO PRO LEU THR TRP GLN LYS CYS SER SER GLY
GAG ACT CAC CCG CCT CTG ACA TGG CAG AAA TGC TCG TCT GGT
299 314

GLY THR CYS THR GLN GLN THR GLY SER VAL VAL ILE ASP ALA
GGC ACT TGC ACT CAA CAG ACA GGC TCC GTG GTC ATC GAC GCC
329 344 359

ASN TRP ARG TRP THR HIS ALA THR ASN SER SER THR ASN CYS
AAC TGG CGC TGG ACT CAC GCT ACG AAC AGC AGC ACG AAC TGC
HaeII 389 404
 
TYR ASP GLY ASN THR TRP SER SER THR LEU CYS PRO ASP ASN
TAC GAT GGC AAC ACT TGG AGC TCG ACC CTA TGT CCT GAC AAC
419 434 449

GLU THR CYS ALA LYS ASN CYS CYS LEU ASP GLY ALA ALA TYR
GAG ACC TGC GCG AAG AAC TGC TGT CTG GAC GGT GCC GCC TAC
464 479 494

ALA SER THR TYR GLY VAL THR THR SER GLY ASN SER LEU SER
GCG TCC ACG TAC GGA GTT ACC ACG AGC GGT AAC AGC CTC TCC
509 524

ILE GLY PHE VAL THR GLN SER ALA GLN LYS ASN VAL GLY ALA
ATT GGC TTT GTC ACC CAG TCT GCG CAG AAG AAC GTT GGC GCT
539 554 MstI 569

ARG LEU TYR LEU MET ALA SER ASP THR THR TYR GLN GLU PHE
CGC CTT TAC CTT ATG GCG AGC GAC ACG ACC TAC CAG GAA TTC
584 599 614 EcoRI

THR LEU LEU GLY ASN GLU PHE SER PHE ASP VAL ASP VAL SER
ACC CTG CTT GGC AAC GAG TTC TCT TTC GAT GTT GAT GTT TCG
629 644 659

GLN LEU PRO
CAG CTG CCG TAAGTGACTT ACCATGAACC CCTGACGTAT CTTCTTGTGG
PvuII 681 691 701 711

CYS GLY LEU ASN GLY ALA
GCTCCCAGCT GACTGGCCAA TTTAAGG TGC GGC TTG AAC GGA GCT
PvuII 731 753

LEU TYR PHE VAL SER MET ASP ALA ASP GLY GLY VAL SER LYS
CTC TAC TTC GTG TCC ATG GAC GCG GAT GGT GGC GTG AGC AAG
768 NcoI 783 798
 
TYR PRO THR ASN THR ALA GLY ALA LYS TYR GLY THR GLY TYR
TAT CCC ACC AAC ACC GCT GGC GCC AAG TAC GGC ACG GGG TAC
813 HaeII 829

CYS ASP SER GLN CYS PRO ARG ASP LEU LYS PHE ILE ASN GLY
TGT GAC AGC CAG TGT CCC CGC GAT CTG AAG TTC ATC AAT GGC
843 858 873

GLN ALA ASN VAL GLU GLY TRP GLU PRO SER SER ASN ASN ALA
CAG GCC AAC GTT GAG GGC TGG GAG CCG TCA TCC AAC AAC GCA
888 903 918

ASN THR GLY ILE GLY GLY HIS GLY SER CYS CYS SER GLU MET
AAC ACG GGC ATT GGA GGA CAC GGA AGC TGC TGC TCT GAG ATG
933 948 963

ASP ILE TRP GLU ALA ASN SER ILE SER GLU ALA LEU THR PRO
GAT ATC TGG GAG GCC AAC TCC ATC TCC GAG GCT CTT ACC CCC
978 993 1008

HIS PRO CYS THR THR VAL GLY GLN GLU ILE CYS GLU GLY ASP
CAC CCT TGC ACG ACT GTC GGC CAG GAG ATC TGC GAG GGT GAT
1023 BglII

GLY CYS GLY GLY THR TYR SER ASP ASN ARG TYR GLY GLY THR
GGG TGC GGC GGA ACT TAC TCC GAT AAC AGA TAT GGC GGC ACT
1068 1083

CYS ASP PRO ASP GLY CYS ASP TRP ASN PRO TYR ARG LEU GLY
TGC GAT CCC GAT GGC TGC GAC TGG AAC CCA TAC CGC CTG GGC
1098 1113 1128

ASN THR SER PHE TYR GLY PRO GLY SER SER PHE THR LEU ASP
AAC ACC AGC TTC TAC GGC CCT GGC TCA AGC TTT ACC CTC GAT
1143 1158 HindIII 1173
 
THR THR LYS LYS LEU THR VAL VAL THR GLN PHE GLU THR SER
ACC ACC AAG AAA TTG ACC GTT GTC ACC CAG TTC GAG ACG TCG
1188 1203 1218

GLY ALA ILE ASN ARG TYR TYR VAL GLN ASN GLY VAL THR PHE
GGT GCC ATC AAC CGA TAC TAT GTC CAG AAT GGC GTC ACT TTC
1233 1248

GLN GLN PRO ASN ALA GLU LEU GLY SER TYR SER GLY ASN GLU
CAG CAG CCC AAC GCC GAG CTT GGT AGT TAC TCT GGC AAC GAG
1263 1278 1293

LEU ASN ASP ASP TYR CYS THR ALA GLU GLU ALA GLU PHE GLY
CTC AAC GAT GAT TAC TGC ACA GCT GAG GAG GCA GAA TTC GGC
PvuII EcoRI

GLY SER SER PHE SER ASP LYS GLY GLY LEU THR GLN PHE LYS
GGA TCC TCT TTC TCA GAC AAG GGC GGC CTG ACT CAG TTC AAG
BamHI 1353 1368 1383

LYS ALA THR SER GLY GLY MET VAL LEU VAL MET SER LEU TRP
AAG GCT ACC TCT GGC GGC ATG GTT CTG GTC ATG AGT CTG TGG
1398 1413 1428

ASP ASP
GAT GAT GTGAGTTTGA TGGACAAACA TGCGCGTTGA CAAAGAGTCA
1444 1454 HincII 1474

TYR TYR ALA ASN MET LEU TRP
AGCAGCTGAC TGAGATGTTA CAG TAC TAC GCC AAC ATG CTG TGG
PvuII 1494 1512

LEU ASP SER THR TYR PRO THR ASN GLU THR SER SER THR PRO
CTG GAC TCC ACC TAC CCG ACA AAC GAG ACC TCC TCC ACA CCC
1527 1542 1557
 
GLY ALA VAL ARG GLY SER CYS SER THR SER SER GLY VAL PRO
GGT GCC GTG CGC GGA AGC TGC TCC ACC AGC TCC GGT GTC CCT
1572 1587 1602

ALA GLN VAL GLU SER GLN SER PRO ASN ALA LYS VAL THR PHE
GCT CAG GTC GAA TCT CAG TCT CCC AAC GCC AAG GTC ACC TTC
1617 1632 BstEII

SER ASN ILE LYS PHE GLY PRO ILE GLY SER THR GLY ASN PRO
TCC AAC ATC AAG TTC GGA CCC ATT GGC AGC ACC GGC AAC CCT
1647 1662 1677

SER GLY GLY ASN PRO PRO GLY GLY ASN ARG GLY THR THR THR
AGC GGC GGC AAC CCT CCC GGC GGA AAC CGT GGC ACC ACC ACC
1692 1707 1722

THR ARG ARG PRO ALA THR THR THR GLY SER SER PRO GLY PRO
ACC CGC CGC CCA GCC ACT ACC ACT GGA AGC TCT CCC GGA CCT
1737 1752 1767

THR GLN SER HIS TYR GLY GLN CYS GLY GLY ILE GLY TYR SER
ACC CAG TCT CAC TAC GGC CAG TGC GGC GGT ATT GGC TAC AGC
1782 1797 1812

GLY PRO THR VAL CYS ALA SER GLY THR THR CYS GLN VAL LEU
GGC CCC ACG GTC TGC GCC AGC GGC ACA ACT TGC CAG GTC CTG
1827 1842

ASN PRO TYR TYR SER GLN CYS LEU
AAC CCT TAC TAC TCT CAG TGC CTG TAAAGCTCCG TGCGAAAGCC
1857 1872 1888 1898

TGACGCACCG GTAGATTCTT GGTGAGCCCG TATCATGACG GCGGCGGGAG
1908 1918 1928 1938 1948
 or
(d) a DNA sequence encoding an amino acid sequence being substantially equivalent to the amino acid sequence given in claim 1 (a), wherein said amino acid sequence represents a protein capable of cleaving cellulose into cellobiose units from the non-reducing ends of cellulose polymer chains, said protein being preceded by a leader peptide, and wherein said DNA sequence is detectable with the DNA sequence of claim 1 (a). The DNA sequence according to claim 1 (b) which is
(e) the DNA sequence contained in the following sequence data: 
GTTACTCTGT GTTGCAAATC CAACTTATAA AGACAACAAC CGCAAACTTT GTCTTGTCGC CATCAGATTG TTCGCCAAGC ACCCTCCCCC CCCCCTATCT TAGTCCTTCT TGTGTCCCAA ATG
MET

GCG CCC TCA GTT ACA CTG CCG TTG ACC ACG GCC ATC CTG GCC ATT GCC CGG CTC GTC GCC GCC CAG CAA CCG GGT ACC AGC ACC CCC GAG GTC CAT CCC AAG
ALA PRO SER VAL THR LEU PRO LEU THR THR ALA ILE LEU ALA ILE ALA ARG LEU VAL ALA ALA Gln Gln Pro Gly Thr Ser Thr Pro Glu Val His Pro Lys

TTG ACA ACC TAC AAG TGT ACA AAG TCC GGG GGG TGC GTG GCC CAG GAC ACC TCG GTG GTC CTT GAC TGG AAC TAC CGC TGG ATG CAC GAC GCA AAC TAC AAC
Leu Thr Thr Tyr Lys Cys Thr Lys Ser Gly Gly Cys Val Ala Gln Asp Thr Ser Val Val Leu Asp Trp Asn Tyr Arg Trp MET His Asp Ala Asn Tyr Asn

TCG TGC ACC GTC AAC GGC GGC GTC AAC ACC ACG CTC TGC CCT GAC GAG GCG ACC TGT GGC AAG AAC TGC TTC ATC GAG GGC GTC GAC TAC GCC GCC TCG GGC
Ser Cys Thr Val Asn Gly Gly Val Asn Thr Thr Leu Cys Pro Asp Glu Ala Thr Cys Gly Lys Asn Cys Phe Ile Glu Gly Val Asp Tyr Ala Ala Ser Gly

GTC ACG ACC TCG GGC AGC AGC CTC ACC ATG AAC CAG TAC ATG CCC AGC AGC TCT GGC GGC TAC AGC AGC GTC TCT CCT CGG CTG TAT CTC CTG GAC TCT GAC
Val Thr Thr Ser Gly Ser Ser Leu Thr MET Asn Gln Tyr MET Pro Ser Ser Ser Gly Gly Tyr Ser Ser Val Ser Pro Arg Leu Tyr Leu Leu Asp Ser Asp

GGT GAG TAC GTG ATG CTG AAG CTC AAC GGC CAG GAG CTG AGC TTC GAC GTC GAC CTC TCT GCT CTG CCG TGT GGA GAG AAC GGC TCG CTC TAC CTG TCT CAG
Gly Glu Tyr Val MET Leu Lys Leu Asn Gly Gln Glu Leu Ser Phe Asp Val Asp Leu Ser Ala Leu Pro Cys Gly Glu Asn Gly Ser Leu Tyr Leu Ser Gln

ATG GAC GAG AAC GGG GGC GCC AAC CAG TAT AAC ACG GCC GGT GCC AAC TAC GGG AGC GGC TAC TGC GAT GCT CAG TGC CCC GTC CAG ACA TGG AGG AAC GGC
MET Asp Glu Asn Gly Gly Ala Asn Gln Tyr Asn Thr Ala Gly Ala Asn Tyr Gly Ser Gly Tyr Cys Asp Ala Gln Cys Pro Val Gln Thr Trp Arg Asn Gly

ACC CTC AAC ACT AGC CAC CAG GGC TTC TGC TGC AAC GAG ATG GAT ATC CTG GAG GGC AAC TCG AGG GCG AAT GCC TTG ACC CCT CAC TCT TGC ACG GCC ACG
Thr Leu Asn Thr Ser His Gln Gly Phe Cys Cys Asn Glu MET Asp Ile Leu Glu Gly Asn Ser Arg Ala Asn Ala Leu Thr Pro His Ser Cys Thr Ala Thr
 
GCC TGC GAC TCT GCC GGT TGC GGC TTC AAC CCC TAT GGC AGC GGC TAC AAA AGg tgagcctgat gccactacta ccccttcct ggcgctctcg cggtttccca tgctgacat
Ala Cys Asp Ser Ala Gly Cys Gly Phe Asn Pro Tyr Gly Ser Gly Thr Lys Ser

ggtttccagC TAC TAC GGC CCC GGA GAT ACC GTT GAC ACC TCC AAG ACC TTC ACC ATC ATC ACC CAG TTC AAC ACG GAC AAC GGC TCG CCC TCG GGC AAC CTT
Tyr Tyr Gly Pro Gly Asp Thr Val Asp Thr Ser Lys Thr Phe Thr Ile Ile Thr Gln Phe Asn Thr Asp Asn Gly Ser Pro Ser Gly Asn Leu

GTG AGC ATC ACC CGC AAG TAC CAG CAA AAC GGC GTC GAC ATC CCC AGC GCC CAG CCC GGC GGC GAC ACC ATC TCG TCC TGC CCG TCC GCC TCA GCC TAC GGC
Val Ser Ile Thr Arg Lys Tyr Gln Gln Asn Gly Val Asp Ile Pro Ser Ala Gln Pro Gly Gly Asp Thr Ile Ser Ser Cys Pro Ser Ala Ser Ala Tyr Gly

GGC CTC GCC ACC ATG GGC AAG GCC CTG AGC AGC GGC ATG GTG CTC GTG TTC AGC ATT TGG AAC GAC AAC AGC CAG TAC ATG AAC TGG CTC GAC AGC GGC AAC
Gly Leu Ala Thr MET Gly Lys Ala Leu Ser Ser Gly MET Val Leu Val Phe Ser Ile Trp Asn Asp Asn Ser Gln Tyr MET Asn Trp Leu Asp Ser Gly Asn

GCC GGC CCC TGC AGC AGC ACC GAG GGC AAC CCA TCC AAC ATC CTG GCC AAC AAC CCC AAC ACG CAC GTC GTC TTC TCC AAC ATC CGC TGG GGA GAC ATT GGG
Ala Gly Pro Cys Ser Ser Thr Glu Gly Asn Pro Ser Asn Ile Leu Ala Asn Asn Pro Asn Thr His Val Val Phe Ser Asn Ile Arg Trp Gly Asp Ile Gly

TCT ACT ACG AAC TCG ACT GCG CCC CCG CCC CCT GCG TCC AGC ACG TTT TCG ACT ACA CGG AGG AGC TCG ACG ACT TCG AGC AGC CCG AGC TGC ACG
Ser Thr Thr Asn Ser Thr Ala Pro Pro Pro Pro Ala Ser Ser Thr Phe Ser Thr Thr Arg Arg Ser Ser Thr Thr Ser Ser Ser Pro Ser Cys Thr

CAG ACT CAC TGG GGG CAG TGC GGT GGC ATT GGG TAC AGC GGG TGC AAG ACG TGC ACG TCG GGC ACT ACG TGC CAG TAT AGC AAC GAC Tgttcgtatc cccatgcct
Gln Thr His Trp Gly Gln Cys Gly Gly Ile Gly Tyr Ser Gly Cys Lys Thr Cys Thr Ser Gly Thr Thr Cys Gln Tyr Ser Asn Asp

gacggggagt gatttgaga tgctaaccgc taaaataca gAC TAC TCG CAA TGC CTT TAGAGCGTTG ACTTGCCTCT GGTCTGTCCA GACGGGGCAC GATAGAATGC GGGCACGCAG GGAGCTCGTA
Tyr Tyr Ser Gln Cys Leu
 or
(f) a DNA sequence encoding an amino acid sequence being substantially equivalent to the amino acid sequence given in claim 1 (b), wherein said amino acid sequence represents a protein which is capable of cleaving cellulose at internal glycosidic sites, said protein being preceded by a leader peptide, and wherein said DNA sequence is detectable with the DNA sequence of claim 1 (b).

4. A fragment of the DNA sequence according to any one of claims 1 to 3 which encodes the leader peptide of said fungal cellulase.

5. The DNA sequence according to any one of claims 1 to 4 which is free of introns.

6. The DNA sequence according to any one of claims 1, 2(d), 3(f) or 4 which contains introns.

7. The DNA sequence of any one of claims 1 to 6, wherein the species of fungus is a species of Trichoderma.

8. The DNA sequence of claim 7, wherein the species of fungus is T. reesei.

9. The DNA sequence of any one of claims 1 (a), 2, 4, 5 or 6, wherein the cellobiohydrolase is the T. reesei cellobiohydrolase-I.

10. An expression vector for expressing a mature fungal cellulase in yeast as a host comprising, in a 5'- to -3' direction:
(a) a yeast promoter sequence; and
(b) a DNA sequence according to any one of claims 1 to 3 or 5 to 9 operatively linked to said promoter sequence.

11. The expression vector of claim 10, which further contains a yeast terminator sequence operatively joined to the 3' end of said coding region.

12. The vector of claim 10 or 11, wherein said promoter sequence contains a yeast enolase-1 promoter.

13. The vector according to any one of claims 10 to 12 comprising additional control sequences compatible with said host operatively linked to said DNA sequence.

14. Yeast cells transformed with an expression vector according to any one of claims 10 to 13.

15. A mature, glycosylated, functionally active fungal endoglucanase I produced by Saccharomyces cerevisiae transformed with a yeast expression vector according to any one of claims 10 to 13.

16. A method of treating cellulose comprising:
(a) transforming yeast with an expression vector according to any one of claims 10 to 13 to produce the mature, glycosylated, functionally active fungal cellulase; and
(b) enzymatically reacting the cellulose with the produced cellulase.

17. The method of claim 16, wherein the transformed yeast is capable of secreting the cellulase.

18. The method of claim 16 or 17, wherein said reacting is carried out in the presence of said yeast.

19. The method of claim 16 or 17 which further comprises separating the produced cellulase from the yeast prior to said reacting.

20. The method according to any one of claims 16 to 19, wherein the produced cellulase is a cellobiohydrolase and said reacting further includes reacting the cellulose with a {3-glucosidase and/or an endoglucanase.

21. The method according to any one of claims 16 to 19, wherein the produced cellulase is an endoglucanase and said reacting further includes reacting the cellulose with a cellobiohydrolase and/or a {3-glucosidase.

22. A method of hydrolyzing cellulose which comprises treating cellulose with the yeast cells of claim 14.

23. A method for the production of a mature, functionally active fungal cellulase comprising the steps of (a) cultivating yeast cells according to claim 14 under suitable conditions so as to permit the secretion of said cellulase into the medium; and
(b) recovering said cellulase from the medium.

## Claims (Claims for the following Contracting State(s) : AT)

1. A method for preparing a DNA sequence encoding an amino acid sequence of a leader peptide-containing fungal cellulase, said amino acid sequence being
(a) the amino acid seqence 
MET TYR ARG LYS LEU ALA VAL ILE SER ALA PHE LEU ALA THR
ALA ARG ALA GLN SER ALA CYS THR LEU GLN SER GLU THR HIS
PRO PRO LEU THR TRP GLN LYS CYS SER SER GLY GLY THR CYS
THR GLN GLN THR GLY SER VAL VAL ILE ASP ALA ASN TRP ARG
TRP THR HIS ALA THR ASN SER SER THR ASN CYS TYR ASP GLY
ASN THR TRP SER SER THR LEU CYS PRO ASP ASN GLU THR CYS
ALA LYS ASN CYS CYS LEU ASP GLY ALA ALA TYR ALA SER THR
TYR GLY VAL THR THR SER GLY ASN SER LEU SER ILE GLY PHE
VAL THR GLN SER ALA GLN LYS ASN VAL GLY ALA ARG LEU TYR
LEU MET ALA SER ASP THR THR TYR GLN GLU PHE THR LEU LEU
GLY ASN GLU PHE SER PHE ASP VAL ASP VAL SER GLN LEU PRO
CYS GLY LEU ASN GLY ALA LEU TYR PHE VAL SER MET ASP ALA
ASP GLY GLY VAL SER LYS TYR PRO THR ASN THR ALA GLY ALA
LYS TYR GLY THR GLY TYR CYS ASP SER GLN CYS PRO ARG ASP
LEU LYS PHE ILE ASN GLY GLN ALA ASN VAL GLU GLY TRP GLU
PRO SER SER ASN ASN ALA ASN THR GLY ILE GLY GLY HIS GLY
SER CYS CYS SER GLU MET ASP ILE TRP GLU ALA ASN SER ILE
SER GLU ALA LEU THR PRO HIS PRO CYS THR THR VAL GLY GLN
GLU ILE CYS GLU GLY ASP GLY CYS GLY GLY THR TYR SER ASP
ASN ARG TYR GLY GLY THR CYS ASP PRO ASP GLY CYS ASP TRP
ASN PRO TYR ARG LEU GLY ASN THR SER PHE TYR GLY PRO GLY
SER SER PHE THR LEU ASP THR THR LYS LYS LEU THR VAL VAL
THR GLN PHE GLU THR SER GLY ALA ILE ASN ARG TYR TYR VAL
GLN ASN GLY VAL THR PHE GLN GLN PRO ASN ALA GLU LEU GLY
SER TYR SER GLY ASN GLU LEU ASN ASP ASP TYR CYS THR ALA
GLU GLU ALA GLU PHE GLY GLY SER SER PHE SER ASP LYS GLY
GLY LEU THR GLN PHE LYS LYS ALA THR SER GLY GLY MET VAL
LEU VAL MET SER LEU TRP ASP ASP TYR TYR ALA ASN MET LEU
TRP LEU ASP SER THR TYR PRO THR ASN GLU THR SER SER THR
PRO GLY ALA VAL ARG GLY SER CYS SER THR SER SER GLY VAL
PRO ALA GLN VAL GLU SER GLN SER PRO ASN ALA LYS VAL THR
PHE SER ASN ILE LYS PHE GLY PRO ILE GLY SER THR GLY ASN
PRO SER GLY GLY ASN PRO PRO GLY GLY ASN ARG GLY THR THR
THR THR ARG ARG PRO ALA THR THR THR GLY SER SER PRO GLY
PRO THR GLN SER HIS TYR GLY GLN CYS GLY GLY ILE GLY TYR
SER GLY PRO THR VAL CYS ALA SER GLY THR THR CYS GLN VAL
LEU ASN PRO TYR TYR SER GLN CYS LEU
 said amino acid sequence representing a cellobiohydrolyse I; or
(b) the amino acid sequence 
MET ALA PRO SER VAL THR LEU PRO LEU THR THR ALA ILE LEU
ALA ILE ALA ARG LEU VAL ALA ALA GLN GLN PRO GLY THR SER
THR PRO GLU VAL HIS PRO LYS LEU THR THR TYR LYS CYS THR
LYS SER GLY GLY CYS VAL ALA GLN ASP THR SER VAL VAL LEU
ASP TRP ASN TYR ARG TRP MET HIS ASP ALA ASN TYR ASN SER
CYS THR VAL ASN GLY GLY VAL ASN THR THR LEU CYS PRO ASP
GLU ALA THR CYS GLY LYS ASN CYS PHE ILE GLU GLY VAL ASP
TYR ALA ALA SER GLY VAL THR THR SER GLY SER SER LEU THR
MET ASN GLN TYR MET PRO SER SER SER GLY GLY TYR SER SER
VAL SER PRO ARG LEU TYR LEU LEU ASP SER ASP GLY GLU TYR
VAL MET LEU LYS LEU ASN GLY GLN GLU LEU SER PHE ASP VAL
ASP LEU SER ALA LEU PRO CYS GLY GLU ASN GLY SER LEU TYR
LEU SER GLN MET ASP GLU ASN GLY GLY ALA ASN GLN TYR ASN
THR ALA GLY ALA ASN TYR GLY SER GLY TYR CYS ASP ALA GLN
CYS PRO VAL GLN THR TRP ARG ASN GLY THR LEU ASN THR SER
HIS GLN GLY PHE CYS CYS ASN GLU MET ASP ILE LEU GLU GLY
ASN SER ARG ALA ASN ALA LEU THR PRO HIS SER CYS THR ALA
THR ALA CYS ASP SER ALA GLY CYS GLY PHE ASN PRO TYR GLY
SER GLY THR LYS SER TYR TYR GLY PRO GLY ASP THR VAL ASP
THR SER LYS THR PHE THR ILE ILE THR GLN PHE ASN THR ASP
ASN GLY SER PRO SER GLY ASN LEU VAL SER ILE THR ARG LYS
TYR GLN GLN ASN GLY VAL ASP ILE PRO SER ALA GLN PRO GLY
GLY ASP THR ILE SER SER CYS PRO SER ALA SER ALA TYR GLY
GLY LEU ALA THR MET GLY LYS ALA LEU SER SER GLY MET VAL
LEU VAL PHE SER ILE TRP ASN ASP ASN SER GLN TYR MET ASN
TRP LEU ASP SER GLY ASN ALA GLY PRO CYS SER SER THR GLU
GLY ASN PRO SER ASN ILE LEU ALA ASN ASN PRO ASN THR HIS
VAL VAL PHE SER ASN ILE ARG TRP GLY ASP ILE GLY SER THR
THR ASN SER THR ALA PRO PRO PRO PRO PRO ALA SER SER THR
THR PHE SER THR THR ARG ARG SER SER THR THR SER SER SER
PRO SER CYS THR GLN THR HIS TRP GLY GLN CYS GLY GLY ILE
GLY TYR SER GLY CYS LYS THR CYS THR SER GLY THR THR CYS
GLN TYR SER ASN ASP TYR TYR SER GLN CYS LEU
 said amino acid sequence representing an endoglucanase I, said method comprising the isolation of said DNA sequence from a library derived from a cellulase producing fungus.

2. The method according to claim 1 (a), wherein said DNA sequence is
(c) the DNA sequence contained in the following sequence data: 
AAGCTTAGCC AAGAACAATA GCCGATAAAG ATAGCCTCAT TAAACGGAAT
1 HindIII 20 30 40 50

GAGCTAGTAG GCAAAGTCAG CGAATGTGTA TATATAAAGG TTCGAGGTCC
60 70 80 90 100

GTGCCTCCCT CATGCTCTCC CCATCTACTC ATCAACTCAG ATCCTCCAGG
110 120 130 140 150

AGACTTGTAC ACCATCTTTT GAGGCACAGA AACCCAATAG TCAACCGCGG
160 170 180 HincII SacII

MET TYR ARG LYS LEU ALA VAL ILE SER ALA PHE
ACTGGCATC ATG TAT CGG AAG TTG GCC GTC ATC TCG GCC TTC
209 224 BglI

LEU ALA THR ALA ARG ALA GLN SER ALA CYS THR LEU GLN SER
TTG GCC ACA GCT CGT GCT CAG TCG GCC TGC ACT CTC CAA TCG
254 269 284

GLU THR HIS PRO PRO LEU THR TRP GLN LYS CYS SER SER GLY
GAG ACT CAC CCG CCT CTG ACA TGG CAG AAA TGC TCG TCT GGT
299 314

GLY THR CYS THR GLN GLN THR GLY SER VAL VAL ILE ASP ALA
GGC ACT TGC ACT CAA CAG ACA GGC TCC GTG GTC ATC GAC GCC
329 344 359

ASN TRP ARG TRP THR HIS ALA THR ASN SER SER THR ASN CYS
AAC TGG CGC TGG ACT CAC GCT ACG AAC AGC AGC ACG AAC TGC
HaeII 389 404
 
TYR ASP GLY ASN THR TRP SER SER THR LEU CYS PRO ASP ASN
TAC GAT GGC AAC ACT TGG AGC TCG ACC CTA TGT CCT GAC AAC
419 434 449

GLU THR CYS ALA LYS ASN CYS CYS LEU ASP GLY ALA ALA TYR
GAG ACC TGC GCG AAG AAC TGC TGT CTG GAC GGT GCC GCC TAC
464 479 494

ALA SER THR TYR GLY VAL THR THR SER GLY ASN SER LEU SER
GCG TCC ACG TAC GGA GTT ACC ACG AGC GGT AAC AGC CTC TCC
509 524

ILE GLY PHE VAL THR GLN SER ALA GLN LYS ASN VAL GLY ALA
ATT GGC TTT GTC ACC CAG TCT GCG CAG AAG AAC GTT GGC GCT
539 554 MstI 569

ARG LEU TYR LEU MET ALA SER ASP THR THR TYR GLN GLU PHE
CGC CTT TAC CTT ATG GCG AGC GAC ACG ACC TAC CAG GAA TTC
584 599 614 EcoRI

THR LEU LEU GLY ASN GLU PHE SER PHE ASP VAL ASP VAL SER
ACC CTG CTT GGC AAC GAG TTC TCT TTC GAT GTT GAT GTT TCG
629 644 659

GLN LEU PRO
CAG CTG CCG TAAGTGACTT ACCATGAACC CCTGACGTAT CTTCTTGTGG
PvuII 681 691 701 711

CYS GLY LEU ASN GLY ALA
GCTCCCAGCT GACTGGCCAA TTTAAGG TGC GGC TTG AAC GGA GCT
PvuII 731 753

LEU TYR PHE VAL SER MET ASP ALA ASP GLY GLY VAL SER LYS
CTC TAC TTC GTG TCC ATG GAC GCG GAT GGT GGC GTG AGC AAG
768 NcoI 783 798
 
TYR PRO THR ASN THR ALA GLY ALA LYS TYR GLY THR GLY TYR
TAT CCC ACC AAC ACC GCT GGC GCC AAG TAC GGC ACG GGG TAC
813 HaeII 829

CYS ASP SER GLN CYS PRO ARG ASP LEU LYS PHE ILE ASN GLY
TGT GAC AGC CAG TGT CCC CGC GAT CTG AAG TTC ATC AAT GGC
843 858 873

GLN ALA ASN VAL GLU GLY TRP GLU PRO SER SER ASN ASN ALA
CAG GCC AAC GTT GAG GGC TGG GAG CCG TCA TCC AAC AAC GCA
888 903 918

ASN THR GLY ILE GLY GLY HIS GLY SER CYS CYS SER GLU MET
AAC ACG GGC ATT GGA GGA CAC GGA AGC TGC TGC TCT GAG ATG
933 948 963

ASP ILE TRP GLU ALA ASN SER ILE SER GLU ALA LEU THR PRO
GAT ATC TGG GAG GCC AAC TCC ATC TCC GAG GCT CTT ACC CCC
978 993 1008

HIS PRO CYS THR THR VAL GLY GLN GLU ILE CYS GLU GLY ASP
CAC CCT TGC ACG ACT GTC GGC CAG GAG ATC TGC GAG GGT GAT
1023 BglII

GLY CYS GLY GLY THR TYR SER ASP ASN ARG TYR GLY GLY THR
GGG TGC GGC GGA ACT TAC TCC GAT AAC AGA TAT GGC GGC ACT
1068 1083

CYS ASP PRO ASP GLY CYS ASP TRP ASN PRO TYR ARG LEU GLY
TGC GAT CCC GAT GGC TGC GAC TGG AAC CCA TAC CGC CTG GGC
1098 1113 1128

ASN THR SER PHE TYR GLY PRO GLY SER SER PHE THR LEU ASP
AAC ACC AGC TTC TAC GGC CCT GGC TCA AGC TTT ACC CTC GAT
1143 1158 HindIII 1173
 
THR THR LYS LYS LEU THR VAL VAL THR GLN PHE GLU THR SER
ACC ACC AAG AAA TTG ACC GTT GTC ACC CAG TTC GAG ACG TCG
1188 1203 1218

GLY ALA ILE ASN ARG TYR TYR VAL GLN ASN GLY VAL THR PHE
GGT GCC ATC AAC CGA TAC TAT GTC CAG AAT GGC GTC ACT TTC
1233 1248

GLN GLN PRO ASN ALA GLU LEU GLY SER TYR SER GLY ASN GLU
CAG CAG CCC AAC GCC GAG CTT GGT AGT TAC TCT GGC AAC GAG
1263 1278 1293

LEU ASN ASP ASP TYR CYS THR ALA GLU GLU ALA GLU PHE GLY
CTC AAC GAT GAT TAC TGC ACA GCT GAG GAG GCA GAA TTC GGC
PvuII EcoRI

GLY SER SER PHE SER ASP LYS GLY GLY LEU THR GLN PHE LYS
GGA TCC TCT TTC TCA GAC AAG GGC GGC CTG ACT CAG TTC AAG
BamHI 1353 1368 1383

LYS ALA THR SER GLY GLY MET VAL LEU VAL MET SER LEU TRP
AAG GCT ACC TCT GGC GGC ATG GTT CTG GTC ATG AGT CTG TGG
1398 1413 1428

ASP ASP
GAT GAT GTGAGTTTGA TGGACAAACA TGCGCGTTGA CAAAGAGTCA
1444 1454 HincII 1474

TYR TYR ALA ASN MET LEU TRP
AGCAGCTGAC TGAGATGTTA CAG TAC TAC GCC AAC ATG CTG TGG
PvuII 1494 1512

LEU ASP SER THR TYR PRO THR ASN GLU THR SER SER THR PRO
CTG GAC TCC ACC TAC CCG ACA AAC GAG ACC TCC TCC ACA CCC
1527 1542 1557
 
GLY ALA VAL ARG GLY SER CYS SER THR SER SER GLY VAL PRO
GGT GCC GTG CGC GGA AGC TGC TCC ACC AGC TCC GGT GTC CCT
1572 1587 1602

ALA GLN VAL GLU SER GLN SER PRO ASN ALA LYS VAL THR PHE
GCT CAG GTC GAA TCT CAG TCT CCC AAC GCC AAG GTC ACC TTC
1617 1632 BstEII

SER ASN ILE LYS PHE GLY PRO ILE GLY SER THR GLY ASN PRO
TCC AAC ATC AAG TTC GGA CCC ATT GGC AGC ACC GGC AAC CCT
1647 1662 1677

SER GLY GLY ASN PRO PRO GLY GLY ASN ARG GLY THR THR THR
AGC GGC GGC AAC CCT CCC GGC GGA AAC CGT GGC ACC ACC ACC
1692 1707 1722

THR ARG ARG PRO ALA THR THR THR GLY SER SER PRO GLY PRO
ACC CGC CGC CCA GCC ACT ACC ACT GGA AGC TCT CCC GGA CCT
1737 1752 1767

THR GLN SER HIS TYR GLY GLN CYS GLY GLY ILE GLY TYR SER
ACC CAG TCT CAC TAC GGC CAG TGC GGC GGT ATT GGC TAC AGC
1782 1797 1812

GLY PRO THR VAL CYS ALA SER GLY THR THR CYS GLN VAL LEU
GGC CCC ACG GTC TGC GCC AGC GGC ACA ACT TGC CAG GTC CTG
1827 1842

ASN PRO TYR TYR SER GLN CYS LEU
AAC CCT TAC TAC TCT CAG TGC CTG TAAAGCTCCG TGCGAAAGCC
1857 1872 1888 1898

TGACGCACCG GTAGATTCTT GGTGAGCCCG TATCATGACG GCGGCGGGAG
1908 1918 1928 1938 1948
 or
(d) a DNA sequence encoding an amino acid sequence being substantially equivalent to the amino acid sequence given in claim 1 (a), wherein said amino acid sequence represents a protein capable of cleaving cellulose into cellobiose units from the non-reducing ends of cellulose polymer chains, said protein being preceded by a leader peptide, and wherein said DNA sequence is detectable with the DNA sequence obtained by the method of claim 1 (a).

3. The method according to claim 1, wherein said DNA sequence is (e) the DNA sequence contained in the following sequence data: 
GTTACTCTGT GTTGCAAATC CAACTTATAA AGACAACAAC CGCAAACTTT GTCTTGTCGC CATCAGATTG TTCGCCAAGC ACCCTCCCCC CCCCCTATCT TAGTCCTCTC TGTGTCCCAA ATG
MET
GCG CCC TCA GTT ACA CTG CCG TTG ACC ACG GCC ATC CTG GCC ATT GCC CGG CTC GTC GCC GCC CAG CAA CCG GGT ACC AGC ACC CCC GAG GTC CAT CCC AAG
ALA PRO SER VAL THR LEU PRO LEU THR THR ALA ILE LEU ALA ILE ALA ARG LEU VAL ALA ALA Gln Gln Pro Gly Thr Ser Thr Pro Glu Val His Pro Lys
TTG ACA ACC TAC AAG TGT ACA AAG TCC GGG GGG TGC GTG GCC CAG GAC ACC TCG GTG GTC CTT GAC TGG AAC TAC CGC TGG ATG CAC GAC GCA AAC TAC AAC
Leu Thr Thr Tyr Lys Cys Thr Lys Ser Gly Gly Cys Val Ala Gln Asp Thr Ser Val Val Leu Asp Trp Asn Tyr Arg Trp MET His Asp Ala Asn Tyr Asn
TCG TGC ACC GTC AAC GGC GGC GTC AAC ACC ACG CTC TGC CCT GAC GAG GCG ACC TGT GGC AAG AAC TGC TTC ATC GAG GGC GTC GAC TAC GCC GCC TCG GGC
Ser Cys Thr Val Asn Gly Gly Val Asn Thr Thr Leu Cys Pro Asp Glu Ala Thr Cys Gly Lys Asn Cys Phe Ile Glu Gly Val Asp Tyr Ala Ala Ser Gly
GTC ACG ACC TCG GGC AGC AGC CTC ACC ATG AAC CAG TAC ATG CCC AGC AGC TCT GGC GGC TAC AGC AGC GTC TCT CCT CGG CTG TAT CTC CTG GAC TCT GAC
Val Thr Thr Ser Gly Ser Ser Leu Thr MET Asn Gln Tyr MET Pro Ser Ser Ser Gly Gly Tyr Ser Ser Val Ser Pro Arg Leu Tyr Leu Leu Asp Ser Asp
GGT GAG TAC GTG ATG CTG AAG CTC AAC GGC CAG GAG CTG AGC TTC GAC GTC GAC CTC TCT GCT CTG CCG TGT GGA GAG AAC GGC TCG CTC TAC CTG TCT CAG
Gly Glu Tyr Val MET Leu Lys Leu Asn Gly Gln Glu Leu Ser Phe Asp Val Asp Leu Ser Ala Leu Pro Cys Gly Glu Asn Gly Ser Leu Tyr Leu Ser Gln
ATG GAC GAG AAC GGG GGC GCC AAC CAG TAT AAC ACG GCC GGT GCC AAC TAC GGG AGC GGC TAC TGC GAT GCT CAG TGC CCC GTC CAG ACA TGG AGG AAC GGC
MET Asp Glu Asn Gly Gly Ala Asn Gln Tyr Asn Thr Ala Gly Ala Asn Tyr Gly Ser Gly Tyr Cys Asp Ala Gln Cys Pro Val Gln Thr Trp Arg Asn Gly
ACC CTC AAC ACT AGC CAC CAG GGC TTC TGC TGC AAC GAG ATG GAT ATC CTG GAG GGC AAC TCG AGG GCG AAT GCC TTG ACC CCT CAC TCT TGC ACG GCC ACG
Thr Leu Asn Thr Ser His Gln Gly Phe Cys Cys Asn Glu MET Asp Ile Leu Glu Gly Asn Ser Arg Ala Asn Ala Leu Thr Pro His Ser Cys Thr Ala Thr
 
GCC TGC GAC TCT GCC GGT TGC GGC TTC AAC CCC TAT GGC AGC GGC TAC AAA AGg tgagcctgat gccactacta cccctttcct ggcgctctcg cggctttcca tgctgacat
Ala Cys Asp Ser Ala Gly Cys Gly Phe Asn Pro Tyr Gly Ser Gly Thr Lys Ser

ggtttccagC TAC TAC GGC CCC GGA GAT ACC GTT GAC ACC TCC AAG ACC TTC ACC ATC ATC ACC CAG TTC AAC ACG GAC AAC GGC TCG CCC TCG GGC AAC CTT
Tyr Tyr Gly Pro Gly Asp Thr Val Asp Thr Ser Lys Thr Phe Thr Ile Ile Thr Gln Phe Asn Thr Asp Asn Gly Ser Pro Ser Gly Asn Leu

GTG AGC ATC ACC CGC AAG TAC CAG CAA AAC GGC GTC GAC ATC CCC AGC GCC CAG CCC GGC GGC GAC ACC ATC TCG TCC TGC CCG TCC GCC TCA GCC TAC GGC
Val Ser Ile Thr Arg Lys Tyr Gln Gln Asn Gly Val Asp Ile Pro Ser Ala Gln Pro Gly Gly Asp Thr Ile Ser Ser Cys Pro Ser Ala Ser Ala Tyr Gly

GGC CTC GCC ACC ATG GGC AAG GCC CTG AGC AGC GGC ATG GTG CTC GTG TTC AGC ATT TGG AAC GAC AAC AGC CAG TAC ATG AAC TGG CTC GAC AGC GGC AAC
Gly Leu Ala Thr MET Gly Lys Ala Leu Ser Ser Gly MET Val Leu Val Phe Ser Ile Trp Asn Asp Asn Ser Gln Tyr MET Asn Trp Leu Asp Ser Gly Asn

GCC GGC CCC TGC AGC ACC GAG GGC AAC CCA TCC AAC ATC CTG GCC AAC AAC CCC AAC ACG CAC GTC GTC TTC TCC AAC ATC CGC TGG GGA GAC ATT GGG
Ala Gly Pro Cys Ser Thr Glu Gly Asn Pro Ser Asn Ile Leu Ala Asn Asn Pro Asn Thr His Val Val Phe Ser Asn Ile Arg Trp Gly Asp Ile Gly

TCT ACT ACG AAC TCG ACT GCG CCC CCG CCC CCG CCT GCG TCC AGC ACG ACG TTT TCG ACT ACA CGG AGG AGC TCG ACG ACT TCG AGC AGC CCG AGC TGC ACG
Ser Thr Thr Asn Ser Thr Ala Pro Pro Pro Pro Pro Ala Ser Ser Thr Thr Phe Ser Thr Thr Arg Arg Ser Ser Thr Thr Ser Ser Ser Pro Ser Cys Thr

CAG ACT CAC TGG GGG CAG TGC GGT GGC ATT GGG TAC AGC GGG TGC AAG ACG TGC ACG TCG GGC ACT ACG TGC CAG TAT AGC AAC GAC tgttcgtatc cccatgcct
Gln Thr His Trp Gly Gln Cys Gly Gly Ile Gly Tyr Ser Gly Cys Lys Thr Cys Thr Ser Gly Thr Thr Cys Gln Tyr Ser Asn Asp

gacggggagt gatttgagag tgctaaccgc taaaataca gAC TAC TCG CAA TGC CTT TAGAGCGTTG ACTTGCCTCT GGTCTGTCCA GACGGGGGAC GATAGAATGC GGGCACGCAG GGAGCTCGTA
Tyr Tyr Ser Gln Cys Leu
 or
(f) a DNA sequence encoding an amino acid sequence being substantially equivalent to the amino acid sequence given in claim 1 (b), wherein said amino acid sequence represents a protein, which is capable of cleaving cellulose at internal glycosidic sites, said protein being preceded by a leader peptide and wherein said DNA sequence is detectable with the DNA sequence of claim 1 (b).

4. The method according to any one of claims 1 to 3, which additionally comprises isolating the fragment of the DNA sequence in any one of claims 1 to 3 which encodes the leader peptide of said fungal cellulase.

5. The method according to any one of claims 1 to 4, wherein said DNA sequence is free of introns.

6. The method according to any one of claims 1, 2(d), 3(f) or 4, wherein said DNA sequence contains introns.

7. The method according to any one of claims 1 to 6, wherein said species of fungus is a species of Trichoderma.

8. The method according to claim 7, wherein the species of fungus is T. reesei.

9. The method according to any one of claims 1 (a), 2, 4, 5 or 6, wherein the cellobiohydrolase is the T.reesei cellobiohydrolase-I.

10. A method for producing a recombinant DNA molecule which comprises inserting a DNA sequence obtainable according to the method of any one of claims 1 to 9 into a vector molecule.

11. A method for the preparation of an expression vector for expressing a mature fungal cellulase in yeast as a host, said method comprising inserting into a vector molecule in a 5'- to 3'-direction the following elements:
(a) a yeast promoter sequence; and
(b) a DNA sequence prepared by a method according to any one of claims 1 to 3 or 5 to 9, said DNA sequence being linked operatively to said promoter sequence.

12. The method according to claim 11, wherein said expression vector further contains a yeast terminator sequence operatively joined to the 3' end of said coding region.

13. The method according to claim 11 or 12, wherein said promoter sequence contains a yeast enolase-1 promoter.

14. The method according to any one of claims 11 to 13, wherein said expression vector comprises additional control sequences compatible with said host operatively linked to said DNA sequences.

15. A method for the preparation of transformed yeast cells comprising transforming yeast cells with an expression vector, wherein said expression vector is obtained by a method according to any one of claims 11 to 14.

16. A method for the preparation of a mature, glycosylated, functionally active fungal endoglucanase-I, comprising the transformation ofSaccharomyces cerevisiae with a yeast expression vector prepared by a method according to any one of claims 11 to 14.

17. A method of treating cellulose comprising:
(a) transforming yeast with an expression vector prepared by a method according to any one of claims 11 to 14 to produce the mature, glycosylated, functionally active fungal cellulase; and
(b) enzymatically reacting the cellulose with the produced cellulase.

18. The method of claim 17, wherein the transformed yeast is capable of secreting the cellulase.

19. The method of claim 17 or 18, wherein said reacting is carried out in the presence of said yeast.

20. The method of claim 17 or 18, which further comprises separating the produced cellulase from the yeast prior to said reacting.

21. The method according to any one of claims 17 to 20, wherein the produced cellulase is a cellobiohydrolase and said reacting further includes reacting the cellulose with {3-glucosidase and/or an edoglucanase.

22. The method according to any one of claims 17 to 20, wherein the produced cellulase is an endoglucanase and said reacting further includes reacting the cellulose with a cellobiohydrolase and/or a β-glucosidase.

23. A method of hydrolysing cellulose which comprises treating cellulose with the yeast cells obtained by the method of claim 15.

24. A method for the production of a mature, functionally active fungal cellulase comprising the steps of
(a) cultivating yeast cells obtained by the method of claim 14 under suitable conditions so as to permit the secretion of said cellulase into the medium;and
(b) recovering said cellulase from the medium.

## Revendications (Revendications pour l'(les) Etat(s) contractant(s) suivant(s) : BE, CH, DE, FR, GB, IT, LI, NL, SE)

1. Séquence d'ADN codant pour une séquence d'amino-acides d'une cellulase fongique contenant un peptide leader, ladite séquence d'amino-acides étant
(a) la séquence d'amino-acides 
MET TYR ARG LYS LEU ALA VAL ILE SER ALA PHE
LEU ALA THR ALA ARG ALA GLN SER ALA CYS THR
LEU GLN SER GLU THR HIS PRO PRO LEU THR TRP
GLN LYS CYS SER SER GLY GLY THR CYS THR GLN
GLN THR GLY SER VAL VAL ILE ASP ALA ASN TRP
ARG TRP THR HIS ALA THR ASN SER SER THR ASN
CYS TYR ASP GLY ASN THR TRP SER SER THR LEU
CYS PRO ASP ASN GLU THR CYS ALA LYS ASN CYS
CYS LEU ASP GLY ALA ALA TYR ALA SER THR TYR
GLY VAL THR THR SER GLY ASN SER LEU SER ILE
GLY PHE VAL THR GLN SER ALA GLN LYS ASN VAL
GLY ALA ARG LEU TYR LEU MET ALA SER ASP THR
THR TYR GLN GLU PHE THR LEU LEU GLY ASN GLU
PHE SER PHE ASP VAL ASP VAL SER GLN LEU PRO
CYS GLY LEU ASN GLY ALA LEU TYR PHE VAL SER
MET ASP ALA ASP GLY GLY VAL SER LYS TYR PRO
THR ASN THR ALA GLY ALA LYS TYR GLY THR GLY
TYR CYS ASP SER GLN CYS PRO ARG ASP LEU LYS
PHE ILE ASN GLY GLN ALA ASN VAL GLU GLY TRP
GLU PRO SER SER ASN ASN ALA ASN THR GLY ILE
GLY GLY HIS GLY SER CYS CYS SER GLU MET ASP
ILE TRP GLU ALA ASN SER ILE SER GLU ALA LEU
THR PRO HIS PRO CYS THR THR VAL GLY GLN GLU
ILE CYS GLU GLY ASP GLY CYS GLY GLY THR TYR
SER ASP ASN ARG TYR GLY GLY THR CYS ASP PRO
ASP GLY CYS ASP TRP ASN PRO TYR ARG LEU GLY
ASN THR SER PHE TYR GLY PRO GLY SER SER PHE
THR LEU ASP THR THR LYS LYS LEU THR VAL VAL
 
THR GLN PHE GLU THR SER GLY ALA ILE ASN ARG
TYR TYR VAL GLN ASN GLY VAL THR PHE GLN GLN
PRO ASN ALA GLU LEU GLY SER TYR SER GLY ASN
GLU LEU ASN ASP ASP TYR CYS THR ALA GLU GLU
ALA GLU PHE GLY GLY SER SER PHE SER ASP LYS
GLY GLY LEU THR GLN PHE LYS LYS ALA THR SER
GLY GLY MET VAL LEU VAL MET SER LEU TRP ASP
ASP TYR TYR ALA ASN MET LEU TRP LEU ASP SER
THR TYR PRO THR ASN GLU THR SER SER THR PRO
GLY ALA VAL ARG GLY SER CYS SER THR SER SER
GLY VAL PRO ALA GLN VAL GLU SER GLN SER PRO
ASN ALA LYS VAL THR PHE SER ASN ILE LYS PHE
GLY PRO ILE GLY SER THR GLY ASN PRO SER GLY
GLY ASN PRO PRO GLY GLY ASN ARG GLY THR THR
THR THR ARG ARG PRO ALA THR THR THR GLY SER
SER PRO GLY PRO THR GLN SER HIS TYR GLY GLN
CYS GLY GLY ILE GLY TYR SER GLY PRO THR VAL
CYS ALA SER GLY THR THR CYS GLN VAL LEU ASN
PRO TYR TYR SER GLN CYS LEU
ladite séquence d'amino-acides représentant une cellobiohydrolase I ; ou
(b) la séquence d'amino-acides 
MET ALA PRO SER VAL THR LEU PRO LEU THR THR
ALA ILE LEU ALA ILE ALA ARG LEU VAL ALA ALA
Gln Gln Pro Gly Thr Ser Thr Pro Glu Val His
Pro Lys Leu Thr Thr Tyr Lys Cys Thr Lys Ser
Gly Gly Cys Val Ala Gln Asp Thr Ser Val Val
Leu Asp Trp Asn Tyr Arg Trp MET His Asp Ala
Asn Tyr Asn Ser Cys Thr Val Asn Gly Gly Val
Asn Thr Thr Leu Cys Pro Asp Glu Ala Thr Cys
Gly Lys Asn Cys Phe Ile Glu Gly Val Asp Tyr
Ala Ala Ser Gly Val Thr Thr Ser Gly Ser Ser
Leu Thr MET Asn Gln Tyr MET Pro Ser Ser Ser
Gly Gly Tyr Ser Ser Val Ser Pro Arg Leu Tyr
 
Leu Leu Asp Ser Asp Gly Glu Tyr Val MET Leu
Lys Leu Asn Gly Gln Glu Leu Ser Phe Asp Val
Asp Leu Ser Ala Leu Pro Cys Gly Glu Asn Gly
Ser Leu Tyr Leu Ser Gln MET Asp Glu Asn Gly
Gly Ala Asn Gln Tyr Asn Thr Ala Gly Ala Asn
Tyr Gly Ser Gly Tyr Cys Asp Ala Gln Cys Pro
Val Gln Thr Trp Arg Asn Gly Thr Leu Asn Thr
Ser His Gln Gly Phe Cys Cys Asn Glu MET Asp
Ile Leu Glu Gly Asn Ser Arg Ala Asn Ala Leu
Thr Pro His Ser Cys Thr Ala Thr Ala Cys Asp
Ser Ala Gly Cys Gly Phe Asn Pro Tyr Gly Ser
Gly Thr Lys Ser Tyr Tyr Gly Pro Gly Asp Thr
Val Asp Thr Ser Lys Thr Phe Thr Ile Ile Thr
Gln Phe Asn Thr Asp Asn Gly Ser Pro Ser Gly
Asn Leu Val Ser Ile Thr Arg Lys Tyr Gln Gln
Asn Gly Val Asp Ile Pro Ser Ala Gln Pro Gly
Gly Asp Thr Ile Ser Ser Cys Pro Ser Ala Ser
Ala Tyr Gly Gly Leu Ala Thr MET Gly Lys Ala
Leu Ser Ser Gly MET Val Leu Val Phe Ser Ile
Trp Asn Asp Asn Ser Gln Tyr MET Asn Trp Leu
Asp Ser Gly Asn Ala Gly Pro Cys Ser Ser Thr
Glu Gly Asn Pro Ser Asn Ile Leu Ala Asn Asn
Pro Asn Thr His Val Val Phe Ser Asn Ile Arg
Trp Gly Asp Ile Gly Ser Thr Thr Asn Ser Thr
Ala Pro Pro Pro Pro Pro Ala Ser Ser Thr Thr
Phe Ser Thr Thr Arg Arg Ser Ser Thr Thr Ser
Ser Ser Pro Ser Cys Thr Gln Thr His Trp Gly
Gln Cys Gly Gly Ile Gly Tyr Ser Gly Cys Lys
Thr Cys Thr Ser Gly Thr Thr Cys Gln Tyr Ser
Asn Asp Tyr Tyr Ser Gln Cys Leu
 ladite séquence d'amino-acides représentant une endoglucanase I.

2. Séquence d'ADN selon la revendication 1(a) qui est
(c) la séquence d'ADN contenue dans les indications suivantes relatives à la séquence : 
AAGCTTAGCC AAGAACAATA GCCGATAAAG ATAGCCTCAT TAAACGGAAT
1 HindIII 20 30 40 50

GAGCTAGTAG GCAAAGTCAG CGAATGTGTA TATATAAAGG TTCGAGGTCC
60 70 80 90 100

GTGCCTCCCT CATGCTCTCC CCATCTACTC ATCAACTCAG ATCCTCCAGG
110 120 130 140 150

AGACTTGTAC ACCATCTTTT GAGGCACAGA AACCCAATAG TCAACCGCGG
160 170 180 HincII SacII

MET TYR ARG LYS LEU ALA VAL ILE SER ALA PHE
ACTGGCATC ATG TAT CGG AAG TTG GCC GTC ATC TCG GCC TTC
209 224 BglI

LEU ALA THR ALA ARG ALA GLN SER ALA CYS THR LEU GLN SER
TTG GCC ACA GCT CGT GCT CAG TCG GCC TGC ACT CTC CAA TCG
254 269 284

GLU THR HIS PRO PRO LEU THR TRP GLN LYS CYS SER SER GLY
GAG ACT CAC CCG CCT CTG ACA TGG CAG AAA TGC TCG TCT GGT
299 314

GLY THR CYS THR GLN GLN THR GLY SER VAL VAL ILE ASP ALA
GGC ACT TGC ACT CAA CAG ACA GGC TCC GTG GTC ATC GAC GCC
329 344 359

ASN TRP ARG TRP THR HIS ALA THR ASN SER SER THR ASN CYS
AAC TGG CGC TGG ACT CAC GCT ACG AAC AGC AGC ACG AAC TGC
HaeII 389 404
 
TYR ASP GLY ASN THR TRP SER SER THR LEU CYS PRO ASP ASN
TAC GAT GGC AAC ACT TGG AGC TCG ACC CTA TGT CCT GAC AAC
419 434 449

GLU THR CYS ALA LYS ASN CYS CYS LEU ASP GLY ALA ALA TYR
GAG ACC TGC GCG AAG AAC TGC TGT CTG GAC GGT GCC GCC TAC
464 479 494

ALA SER THR TYR GLY VAL THR THR SER GLY ASN SER LEU SER
GCG TCC ACG TAC GGA GTT ACC ACG AGC GGT AAC AGC CTC TCC
509 524

ILE GLY PHE VAL THR GLN SER ALA GLN LYS ASN VAL GLY ALA
ATT GGC TTT GTC ACC CAG TCT GCG CAG AAG AAC GTT GGC GCT
539 554 MstI 569

ARG LEU TYR LEU MET ALA SER ASP THR THR TYR GLN GLU PHE
CGC CTT TAC CTT ATG GCG AGC GAC ACG ACC TAC CAG GAA TTC
584 599 614 EcoRI

THR LEU LEU GLY ASN GLU PHE SER PHE ASP VAL ASP VAL SER
ACC CTG CTT GGC AAC GAG TTC TCT TTC GAT GTT GAT GTT TCG
629 644 659

GLN LEU PRO
CAG CTG CCG TAAGTGACTT ACCATGAACC CCTGACGTAT CTTCTTGTGG
PvuII 681 691 701 711

CYS GLY LEU ASN GLY ALA
GCTCCCAGCT GACTGGCCAA TTTAAGG TGC GGC TTG AAC GGA GCT
PvuII 731 753

LEU TYR PHE VAL SER MET ASP ALA ASP GLY GLY VAL SER LYS
CTC TAC TTC GTG TCC ATG GAC GCG GAT GGT GGC GTG AGC AAG
768 NcoI 783 798
 
TYR PRO THR ASN THR ALA GLY ALA LYS TYR GLY THR GLY TYR
TAT CCC ACC AAC ACC GCT GGC GCC AAG TAC GGC ACG GGG TAC
813 HaeII 829

CYS ASP SER GLN CYS PRO ARG ASP LEU LYS PHE ILE ASN GLY
TGT GAC AGC CAG TGT CCC CGC GAT CTG AAG TTC ATC AAT GGC
843 858 873

GLN ALA ASN VAL GLU GLY TRP GLU PRO SER SER ASN ASN ALA
CAG GCC AAC GTT GAG GGC TGG GAG CCG TCA TCC AAC AAC GCA
888 903 918

ASN THR GLY ILE GLY GLY HIS GLY SER CYS CYS SER GLU MET
AAC ACG GGC ATT GGA GGA CAC GGA AGC TGC TGC TCT GAG ATG
933 948 963

ASP ILE TRP GLU ALA ASN SER ILE SER GLU ALA LEU THR PRO
GAT ATC TGG GAG GCC AAC TCC ATC TCC GAG GCT CTT ACC CCC
978 993 1008

HIS PRO CYS THR THR VAL GLY GLN GLU ILE CYS GLU GLY ASP
CAC CCT TGC ACG ACT GTC GGC CAG GAG ATC TGC GAG GGT GAT
1023 BglII

GLY CYS GLY GLY THR TYR SER ASP ASN ARG TYR GLY GLY THR
GGG TGC GGC GGA ACT TAC TCC GAT AAC AGA TAT GGC GGC ACT
1068 1083

CYS ASP PRO ASP GLY CYS ASP TRP ASN PRO TYR ARG LEU GLY
TGC GAT CCC GAT GGC TGC GAC TGG AAC CCA TAC CGC CTG GGC
1098 1113 1128

ASN THR SER PHE TYR GLY PRO GLY SER SER PHE THR LEU ASP
AAC ACC AGC TTC TAC GGC CCT GGC TCA AGC TTT ACC CTC GAT
1143 1158 HindIII 1173
 
THR THR LYS LYS LEU THR VAL VAL THR GLN PHE GLU THR SER
ACC ACC AAG AAA TTG ACC GTT GTC ACC CAG TTC GAG ACG TCG
1188 1203 1218

GLY ALA ILE ASN ARG TYR TYR VAL GLN ASN GLY VAL THR PHE
GGT GCC ATC AAC CGA TAC TAT GTC CAG AAT GGC GTC ACT TTC
1233 1248

GLN GLN PRO ASN ALA GLU LEU GLY SER TYR SER GLY ASN GLU
CAG CAG CCC AAC GCC GAG CTT GGT AGT TAC TCT GGC AAC GAG
1263 1278 1293

LEU ASN ASP ASP TYR CYS THR ALA GLU GLU ALA GLU PHE GLY
CTC AAC GAT GAT TAC TGC ACA GCT GAG GAG GCA GAA TTC GGC
PvuII EcoRI

GLY SER SER PHE SER ASP LYS GLY GLY LEU THR GLN PHE LYS
GGA TCC TCT TTC TCA GAC AAG GGC GGC CTG ACT CAG TTC AAG
BamHI 1353 1368 1383

LYS ALA THR SER GLY GLY MET VAL LEU VAL MET SER LEU TRP
AAG GCT ACC TCT GGC GGC ATG GTT CTG GTC ATG AGT CTG TGG
1398 1413 1428

ASP ASP
GAT GAT GTGAGTTTGA TGGACAAACA TGCGCGTTGA CAAAGAGTCA
1444 1454 HincII 1474

TYR TYR ALA ASN MET LEU TRP
AGCAGCTGAC TGAGATGTTA CAG TAC TAC GCC AAC ATG CTG TGG
PvuII 1494 1512

LEU ASP SER THR TYR PRO THR ASN GLU THR SER SER THR PRO
CTG GAC TCC ACC TAC CCG ACA AAC GAG ACC TCC TCC ACA CCC
1527 1542 1557
 
GLY ALA VAL ARG GLY SER CYS SER THR SER SER GLY VAL PRO
GGT GCC GTG CGC GGA AGC TGC TCC ACC AGC TCC GGT GTC CCT
1572 1587 1602

ALA GLN VAL GLU SER GLN SER PRO ASN ALA LYS VAL THR PHE
GCT CAG GTC GAA TCT CAG TCT CCC AAC GCC AAG GTC ACC TTC
1617 1632 BstEII

SER ASN ILE LYS PHE GLY PRO ILE GLY SER THR GLY ASN PRO
TCC AAC ATC AAG TTC GGA CCC ATT GGC AGC ACC GGC AAC CCT
1647 1662 1677

SER GLY GLY ASN PRO PRO GLY GLY ASN ARG GLY THR THR THR
AGC GGC GGC AAC CCT CCC GGC GGA AAC CGT GGC ACC ACC ACC
1692 1707 1722

THR ARG ARG PRO ALA THR THR THR GLY SER SER PRO GLY PRO
ACC CGC CGC CCA GCC ACT ACC ACT GGA AGC TCT CCC GGA CCT
1737 1752 1767

THR GLN SER HIS TYR GLY GLN CYS GLY GLY ILE GLY TYR SER
ACC CAG TCT CAC TAC GGC CAG TGC GGC GGT ATT GGC TAC AGC
1782 1797 1812

GLY PRO THR VAL CYS ALA SER GLY THR THR CYS GLN VAL LEU
GGC CCC ACG GTC TGC GCC AGC GGC ACA ACT TGC CAG GTC CTG
1827 1842

ASN PRO TYR TYR SER GLN CYS LEU
AAC CCT TAC TAC TCT CAG TGC CTG TAAAGCTCCG TGCGAAAGCC
1857 1872 1888 1898

TGACGCACCG GTAGATTCTT GGTGAGCCCG TATCATGACG GCGGCGGGAG
1908 1918 1928 1938 1948
 ou
(d) une séquence d'ADN codant pour une séquence d'amino-acides qui est sensiblement équivalente à la séquence d'amino-acides indiquée dans la revendication 1 (a), ladite séquence d'aminoacides représentant une protéine capable de cliver la cellulose en motifs de cellobiose à partir des extrémités non réductrices des chaînes polymères de la cellulose, ladite protéine étant précédée d'un peptide leader, et ladite séquence d'ADN étant détectable avec la séquence d'ADN de la revendication 1 (a).

3. Séquence d'ADN selon la revendication 1 (b) qui est
(e) la séquence d'ADN contenue dans les indications suivantes relatives à la séquence : 
GTTACTCTGT GTTGCAAATC CAACTTATAA AGACAACAAC CGCAAACTTT GTCTTGTGGC CATCAGATTG TTCGCCAAGC ACCCTCCCCC CCCCCTATCT TAGTCCTCTC TGTGTCCAAA ATG
MET

GCG CCC TCA GTT ACA CTG CCG TTG ACC ACG GCC ATC CTG GCC ATT GCC CGG CTC GTC GCC GCC CAG CAA CCG GGT ACC AGC ACC CCC GAG GTC CAT CCC AAG
ALA PRO SER VAL THR LEU PRO LEU THR THR ALA ILE LEU ALA ILE ALA ARG LEU VAL ALA ALA Gln Gln Pro Gly Thr Ser Thr Pro Glu Val His Pro Lys

TTG ACA ACC TAC AAG TGT ACA AAG TCC GGG GGG TGC GTG GCC CAG GAC ACC TCG GTG GTC CTT GAC TGG AAC TAC CGC TGG ATG CAC GAC GCA AAC TAC AAC
Leu Thr Thr Tyr Lys Cys Thr Lys Ser Gly Gly Cys Val Ala Gln Asp Thr Ser Val Val Leu Asp Trp Asn Tyr Arg Trp MET His Asp Ala Asn Tyr Asn

TCG TGC ACC GTC AAC GGC GGC GTC AAC ACC ACG CTC TGC CCT GAC GAG GCG ACC TGT GGC AAG AAC TGC TTC ATC GAG GGC GTC GAC TAC GCC GCC TCG GGC
Ser Cys Thr Val Asn Gly Gly Val Asn Thr Thr Leu Cys Pro Asp Glu Ala Thr Cys Gly Lys Asn Cys Phe Ile Glu Gly Val Asp Tyr Ala Ala Ser Gly

GTC ACG ACC TCG GGC AGC AGC CTC ACC ATG AAC CAG TAC ATG CCC AGC AGC TCT GGC GGC TAC AGC AGC GTC TCT CCT CGG CTG TAT CTC CTG GAC TCT GAC
Val Thr Thr Ser Gly Ser Ser Leu Thr MET Asn Gln Tyr MET Pro Ser Ser Ser Gly Gly Tyr Ser Ser Val Ser Pro Arg Leu Tyr Leu Leu Asp Ser Asp

GGT GAG TAC GTG ATG CTG AAG CTC AAC GGC CAG GAG CTG AGC TTC GAC GTC GAC CTC TCT GCT CTG CCG TGT GGA GAG AAC GGC TCG CTC TAC CTG TCT CAG
Gly Glu Tyr Val MET Leu Lys Leu Asn Gly Gln Glu Leu Ser Phe Asp Val Asp Leu Ser Ala Leu Pro Cys Gly Glu Asn Gly Ser Leu Tyr Leu Ser Gln

ATG GAC GAG AAC GGG GGC GCC AAC CAG TAT AAC ACG GCC GGT GCC AAC TAC GGG AGC GGC TAC TGC GAT GCT CAG TGC CCC GTC CAG ACA TGG AGG AAC GGC
MET Asp Glu Asn Gly Gly Ala Asn Gln Tyr Asn Thr Ala Gly Ala Asn Tyr Gly Ser Gly Tyr Cys Asp Ala Gln Cys Pro Val Gln Thr Trp Arg Asn Gly

ACC CTC AAC ACT AGC CAC CAG GGC TTC TGC TGC AAC GAG ATG GAT ATC CTG GAG GGC AAC TCG AGG GCG AAT GCC TTG ACC CCT CAC TCT TGC ACG GCC AC[?]
Thr Leu Asn Thr Ser His Gln Gly Phe Cys Cys Asn Glu MET Asp Ile Leu Glu Gly Asn Ser Arg Ala Asn Ala Leu Thr Pro His Ser Cys Thr Ala Thr
 
GCC TGC GAC TCT GCC GGT TGC GGC TTC AAC CCC TAT GGC AGC GGC TAC AAA AGg tgagcctgat gccactacta cccctttcct ggcgctctcg cggttttcca tgctgacat
Ala Cys Asp Ser Ala Gly Cys Gly Phe Asn Pro Tyr Gly Ser Gly Thr Lys Ser

ggttttccagC TAC TAC GGC CCC GGA GAT ACC GTT GAC ACC TCC AAG ACC TTC ACC ATC ATC ACC CAG TTC AAC ACG GAC AAC GGC TCG CCC TCG GGC AAC CTT
Tyr Tyr Gly Pro Gly Asp Thr Val Asp Thr Ser Lys Thr Phe Thr Ile Ile Thr Gln Phe Asn Thr Asp Asn Gly Ser Pro Ser Gly Asn Leu

GTG AGC ATC ACC CGC AAG TAC CAG CAA AAC GGC GTC GAC ATC CCC AGC GCC CAG CCC GGC GGC GAC ACC ATC TCG TCC TGC CCG TCC GCC TCA GCC TAC GGC
Val Ser Ile Thr Arg Lys Tyr Gln Gln Asn Gly Val Asp Ile Pro Ser Ala Gln Pro Gly Gly Asp Thr Ile Ser Ser Cys Pro Ser Ala Ser Ala Tyr Gly

GGC CTC GCC ACC ATG GGC AAG GCC CTG AGC AGC GGC ATG GTG CTC GTG TTC AGC ATT TGG AAC GAC AAC AGC CAG TAC ATG AAC TGG CTC GAC AGC GGC AAC
Gly Leu Ala Thr MET Gly Lys Ala Leu Ser Ser Gly MET Val Leu Val Phe Ser Ile Trp Asn Asp Asn Ser Gln Tyr MET Asn Trp Leu Asp Ser Gly Asn

GCC GGC CCC TGC AGC AGC ACC GAG GGC AAC CCA TCC AAC ATC CTG GCC AAC AAC CCC AAC ACG CAC GTC GTC TTC TCC AAC ATC CGC TGG GGA GAC ATT GGG
Ala Gly Pro Cys Ser Ser Thr Glu Gly Asn Pro Ser Asn Ile Leu Ala Asn Asn Pro Asn Thr His Val Val Phe Ser Asn Ile Arg Trp Gly Asp Ile Gly

TCT ACT ACG AAC TCG ACT GCG CCC CCG CCC CCG CCT GCG TCC AGC ACG ACG TTT TCG ACT ACA CGG AGG AGC TCG ACG ACT TCG AGC AGC CCG AGC TGC ACG
Ser Thr Thr Asn Ser Thr Ala Pro Pro Pro Pro Pro Ala Ser Ser Thr Thr Phe Ser Thr Thr Arg Arg Ser Ser Thr Thr Ser Ser Ser Pro Ser Cys Thr

CAG ACT CAC TGG GGG CAG TGC GGT GGC ATT GGG TAC AGC GGG TGC AAG ACG TGC ACG TCG GGC ACT ACG TGC CAG TAT AGC AAC GAC tgttcgtatc cccatgcct
Gln Thr His Trp Gly Gln Cys Gly Gly Ile Gly Tyr Ser Gly Cys Lys Thr Cys Thr Ser Gly Thr Thr Cys Gln Tyr Ser Asn Asp

gacgggagt gatttgagag tgctaaccgc taaaataca gAC TAC TCG CAA TGC CTT TAGAGCGTTG ACTTGCCTCT GGTCTGTCCA GACGGGGCAC GATAGAATGC GGGCACGCAG GGAGTCGTA
Tyr Tyr Ser Gln Cys Leu
 ou
(f) une séquence d'ADN codant pour une séquence d'amino-acides qui est sensiblement équivalente à la séquence d'amino-acides indiquée dans la revendication 1 (b), où ladite séquence d'aminoacides représente une protéine qui est capable de cliver la cellulose aux sites glycosidiques internes, ladite protéine étant précédée d'un peptide leader et ladite séquence d'ADN étant détectable avec la séquence d'ADN de la revendication 1 (b).

4. Fragment de la séquence d'ADN selon l'une quelconque des revendications 1 à 3, qui code pour le peptide leader de ladite cellulase fongique.

5. Séquence d'ADN selon l'une quelconque des revendications 1 à 4, qui est dépourvue d'introns.

6. Séquence d'ADN selon l'une quelconque des revendications 1, 2(d), 3(f) ou 4, qui contient des introns.

7. Séquence d'ADN selon l'une quelconque des revendications 1 à 6, dans laquelle l'espèce de champignon est une espèce de Trichoderma.

8. Séquence d'ADN selon la revendication 7, dans laquelle l'espèce de champignon est T. reesei.

9. Séquence d'ADN selon l'une quelconque des revendications 1 (a), 2, 4, 5 ou 6, où la cellobiohydrolase est la cellobiohydrolase-I de T. reesei.

10. Vecteur d'expression pour exprimer une cellulase fongique mature dans une levure servant d'hôte comprenant, dans une direction 5' à 3' :
(a) une séquence promoteur de levure ; et
(b) une séquence d'ADN selon l'une quelconque des revendications 1 à 3 ou 5 à 9 en liaison fonctionnelle avec ladite séquence promoteur.

11. Vecteur d'expression selon la revendication 10, qui de plus contient une séquence terminateur de levure en liaison fonctionnelle avec l'extrémité 3' de ladite région codante.

12. Vecteur selon la revendication 10 ou 11, dans lequel ladite séquence promoteur contient un promoteur énolase-1 de levure.

13. Vecteur selon l'une quelconque des revendications 10 à 12 comprenant des séquences régulatrices additionnelles compatibles avec ledit hôte en liaison fonctionnelle avec ladite séquence d'ADN.

14. Cellules de levure transformées avec un vecteur d'expression selon l'une quelconque des revendications 10 à 13.

15. Endoglucanase 1 fongique, mature, glycosylée, fonctionnellement active, produite par Saccharomyces cerevisiae transformé avec un vecteur d'expression dans une levure selon l'une quelconque des revendications 10 à 13.

16. Procédé pour le traitement de la cellulose comprenant :
(a) la transformation d'une levure avec un vecteur d'expression selon l'une quelconque des revendications 10 à 13 pour produire la cellulase fongique mature, glycosylée, fonctionnellement active ; et
(b) la réaction enzymatique de la cellulose avec la cellulase produite.

17. Procédé selon la revendication 16, dans lequel la levure transformée est capable de sécréter la cellulase.

18. Procédé selon la revendication 16 ou 17, dans lequel ladite réaction est effectuée en présence de ladite levure.

19. Procédé selon la revendication 16 ou 17, qui comprend de plus la séparation de la cellulase produite d'avec la levure avant ladite réaction.

20. Procédé selon l'une quelconque des revendications 16 à 19, dans lequel la cellulase produite est une cellobiohydrolase et ladite réaction comprend de plus la réaction de la cellulose avec une glucosidase et/ou une endoglucanase.

21. Procédé selon l'une quelconque des revendications 16 à 19, dans lequel la cellulase produite est une endoglucanase et ladite réaction comprend de plus la réaction de la cellulose avec une cellobiohydrolase et/ou une β-glucosidase.

22. Procédé d'hydrolyse de la cellulose qui comprend le traitement de la cellulose avec les cellules de levure de la revendication 14.

23. Procédé pour la production d'une cellulase fongique mature, fonctionnellement active, comprenant les étapes de :
(a) culture de cellules de levure selon la revendication 14 dans des conditions appropriées pour permettre la sécrétion de ladite cellulase dans le milieu ; et
(b) la récupération de ladite cellulase à partir du milieu.

## Revendications (Revendications pour l'(les) Etat(s) contractant(s) suivant(s) : AT)

1. Procédé pour préparer une séquence d'ADN codant pour une séquence d'amino-acides d'une cellulase fongique contenant un peptide leader, ladite séquence d'amino-acides étant
(a) la séquence d'amino-acides 
MET TYR ARG LYS LEU ALA VAL ILE SER ALA PHE LEU ALA THR
ALA ARG ALA GLN SER ALA CYS THR LEU GLN SER GLU THR HIS
PRO PRO LEU THR TRP GLN LYS CYS SER SER GLY GLY THR CYS
THR GLN GLN THR GLY SER VAL VAL ILE ASP ALA ASN TRP ARG
TRP THR HIS ALA THR ASN SER SER THR ASN CYS TYR ASP GLY
ASN THR TRP SER SER THR LEU CYS PRO ASP ASN GLU THR CYS
ALA LYS ASN CYS CYS LEU ASP GLY ALA ALA TYR ALA SER THR
TYR GLY VAL THR THR SER GLY ASN SER LEU SER ILE GLY PHE
VAL THR GLN SER ALA GLN LYS ASN VAL GLY ALA ARG LEU TYR
LEU MET ALA SER ASP THR THR TYR GLN GLU PHE THR LEU LEU
GLY ASN GLU PHE SER PHE ASP VAL ASP VAL SER GLN LEU PRO
CYS GLY LEU ASN GLY ALA LEU TYR PHE VAL SER MET ASP ALA
ASP GLY GLY VAL SER LYS TYR PRO THR ASN THR ALA GLY ALA
LYS TYR GLY THR GLY TYR CYS ASP SER GLN CYS PRO ARG ASP
LEU LYS PHE ILE ASN GLY GLN ALA ASN VAL GLU GLY TRP GLU
PRO SER SER ASN ASN ALA ASN THR GLY ILE GLY GLY HIS GLY
SER CYS CYS SER GLU MET ASP ILE TRP GLU ALA ASN SER ILE
SER GLU ALA LEU THR PRO HIS PRO CYS THR THR VAL GLY GLN
GLU ILE CYS GLU GLY ASP GLY CYS GLY GLY THR TYR SER ASP
ASN ARG TYR GLY GLY THR CYS ASP PRO ASP GLY CYS ASP TRP
ASN PRO TYR ARG LEU GLY ASN THR SER PHE TYR GLY PRO GLY
SER SER PHE THR LEU ASP THR THR LYS LYS LEU THR VAL VAL
THR GLN PHE GLU THR SER GLY ALA ILE ASN ARG TYR TYR VAL
GLN ASN GLY VAL THR PHE GLN GLN PRO ASN ALA GLU LEU GLY
SER TYR SER GLY ASN GLU LEU ASN ASP ASP TYR CYS THR ALA
GLU GLU ALA GLU PHE GLY GLY SER SER PHE SER ASP LYS GLY
GLY LEU THR GLN PHE LYS LYS ALA THR SER GLY GLY MET VAL
LEU VAL MET SER LEU TRP ASP ASP TYR TYR ALA ASN MET LEU
TRP LEU ASP SER THR TYR PRO THR ASN GLU THR SER SER THR
PRO GLY ALA VAL ARG GLY SER CYS SER THR SER SER GLY VAL
PRO ALA GLN VAL GLU SER GLN SER PRO ASN ALA LYS VAL THR
PHE SER ASN ILE LYS PHE GLY PRO ILE GLY SER THR GLY ASN
PRO SER GLY GLY ASN PRO PRO GLY GLY ASN ARG GLY THR THR
THR THR ARG ARG PRO ALA THR THR THR GLY SER SER PRO GLY
PRO THR GLN SER HIS TYR GLY GLN CYS GLY GLY ILE GLY TYR
SER GLY PRO THR VAL CYS ALA SER GLY THR THR CYS GLN VAL
LEU ASN PRO TYR TYR SER GLN CYS LEU
 ladite séquence d'amino-acides représentant une cellobiohydrolase I ; ou
(b) la séquence d'amino-acides 
MET ALA PRO SER VAL THR LEU PRO LEU THR THR ALA ILE LEU
ALA ILE ALA ARG LEU VAL ALA ALA GLN GLN PRO GLY THR SER
THR PRO GLU VAL HIS PRO LYS LEU THR THR TYR LYS CYS THR
LYS SER GLY GLY CYS VAL ALA GLN ASP THR SER VAL VAL LEU
ASP TRP ASN TYR ARG TRP MET HIS ASP ALA ASN TYR ASN SER
CYS THR VAL ASN GLY GLY VAL ASN THR THR LEU CYS PRO ASP
GLU ALA THR CYS GLY LYS ASN CYS PHE ILE GLU GLY VAL ASP
TYR ALA ALA SER GLY VAL THR THR SER GLY SER SER LEU THR
MET ASN GLN TYR MET PRO SER SER SER GLY GLY TYR SER SER
VAL SER PRO ARG LEU TYR LEU LEU ASP SER ASP GLY GLU TYR
VAL MET LEU LYS LEU ASN GLY GLN GLU LEU SER PHE ASP VAL
ASP LEU SER ALA LEU PRO CYS GLY GLU ASN GLY SER LEU TYR
LEU SER GLN MET ASP GLU ASN GLY GLY ALA ASN GLN TYR ASN
THR ALA GLY ALA ASN TYR GLY SER GLY TYR CYS ASP ALA GLN
CYS PRO VAL GLN THR TRP ARG ASN GLY THR LEU ASN THR SER
HIS GLN GLY PHE CYS CYS ASN GLU MET ASP ILE LEU GLU GLY
ASN SER ARG ALA ASN ALA LEU THR PRO HIS SER CYS THR ALA
THR ALA CYS ASP SER ALA GLY CYS GLY PHE ASN PRO TYR GLY
SER GLY THR LYS SER TYR TYR GLY PRO GLY ASP THR VAL ASP
THR SER LYS THR PHE THR ILE ILE THR GLN PHE ASN THR ASP
ASN GLY SER PRO SER GLY ASN LEU VAL SER ILE THR ARG LYS
TYR GLN GLN ASN GLY VAL ASP ILE PRO SER ALA GLN PRO GLY
GLY ASP THR ILE SER SER CYS PRO SER ALA SER ALA TYR GLY
GLY LEU ALA THR MET GLY LYS ALA LEU SER SER GLY MET VAL
LEU VAL PHE SER ILE TRP ASN ASP ASN SER GLN TYR MET ASN
TRP LEU ASP SER GLY ASN ALA GLY PRO CYS SER SER THR GLU
GLY ASN PRO SER ASN ILE LEU ALA ASN ASN PRO ASN THR HIS
VAL VAL PHE SER ASN ILE ARG TRP GLY ASP ILE GLY SER THR
THR ASN SER THR ALA PRO PRO PRO PRO PRO ALA SER SER THR
THR PHE SER THR THR ARG ARG SER SER THR THR SER SER SER
PRO SER CYS THR GLN THR HIS TRP GLY GLN CYS GLY GLY ILE
GLY TYR SER GLY CYS LYS THR CYS THR SER GLY THR THR CYS
GLN TYR SER ASN ASP TYR TYR SER GLN CYS LEU
 ladite séquence d'amino-acides représentant une endoglucanase I, ledit procédé comprenant l'isolement de ladite séquence d'ADN d'une banque dérivée d'un champignon producteur de cellulase.

2. Procédé selon la revendication 1 (a), dans lequel ladite séquence d'ADN est :
(c) la séquence d'ADN contenue dans les indications suivantes relatives à la séquence : 
AAGCTTAGCC AAGAACAATA GCCGATAAAG ATAGCCTCAT TAAACGGAAT
1 HindIII 20 30 40 50

GAGCTAGTAG GCAAAGTCAG CGAATGTGTA TATATAAAGG TTCGAGGTCC
60 70 80 90 100

GTGCCTCCCT CATGCTCTCC CCATCTACTC ATCAACTCAG ATCCTCCAGG
110 120 130 140 150

AGACTTGTAC ACCATCTTTT GAGGCACAGA AACCCAATAG TCAACCGCGG
160 170 180 HincII SacII

MET TYR ARG LYS LEU ALA VAL ILE SER ALA PHE
ACTGGCATC ATG TAT CGG AAG TTG GCC GTC ATC TCG GCC TTC
209 224 BglI

LEU ALA THR ALA ARG ALA GLN SER ALA CYS THR LEU GLN SER
TTG GCC ACA GCT CGT GCT CAG TCG GCC TGC ACT CTC CAA TCG
254 269 284

GLU THR HIS PRO PRO LEU THR TRP GLN LYS CYS SER SER GLY
GAG ACT CAC CCG CCT CTG ACA TGG CAG AAA TGC TCG TCT GGT
299 314

GLY THR CYS THR GLN GLN THR GLY SER VAL VAL ILE ASP ALA
GGC ACT TGC ACT CAA CAG ACA GGC TCC GTG GTC ATC GAC GCC
329 344 359

ASN TRP ARG TRP THR HIS ALA THR ASN SER SER THR ASN CYS
AAC TGG CGC TGG ACT CAC GCT ACG AAC AGC AGC ACG AAC TGC
HaeII 389 404
 
TYR ASP GLY ASN THR TRP SER SER THR LEU CYS PRO ASP ASN
TAC GAT GGC AAC ACT TGG AGC TCG ACC CTA TGT CCT GAC AAC
419 434 449

GLU THR CYS ALA LYS ASN CYS CYS LEU ASP GLY ALA ALA TYR
GAG ACC TGC GCG AAG AAC TGC TGT CTG GAC GGT GCC GCC TAC
464 479 494

ALA SER THR TYR GLY VAL THR THR SER GLY ASN SER LEU SER
GCG TCC ACG TAC GGA GTT ACC ACG AGC GGT AAC AGC CTC TCC
509 524

ILE GLY PHE VAL THR GLN SER ALA GLN LYS ASN VAL GLY ALA
ATT GGC TTT GTC ACC CAG TCT GCG CAG AAG AAC GTT GGC GCT
539 554 MstI 569

ARG LEU TYR LEU MET ALA SER ASP THR THR TYR GLN GLU PHE
CGC CTT TAC CTT ATG GCG AGC GAC ACG ACC TAC CAG GAA TTC
584 599 614 EcoRI

THR LEU LEU GLY ASN GLU PHE SER PHE ASP VAL ASP VAL SER
ACC CTG CTT GGC AAC GAG TTC TCT TTC GAT GTT GAT GTT TCG
629 644 659

GLN LEU PRO
CAG CTG CCG TAAGTGACTT ACCATGAACC CCTGACGTAT CTTCTTGTGG
PvuII 681 691 701 711

CYS GLY LEU ASN GLY ALA
GCTCCCAGCT GACTGGCCAA TTTAAGG TGC GGC TTG AAC GGA GCT
PvuII 731 753

LEU TYR PHE VAL SER MET ASP ALA ASP GLY GLY VAL SER LYS
CTC TAC TTC GTG TCC ATG GAC GCG GAT GGT GGC GTG AGC AAG
768 NcoI 783 798
 
TYR PRO THR ASN THR ALA GLY ALA LYS TYR GLY THR GLY TYR
TAT CCC ACC AAC ACC GCT GGC GCC AAG TAC GGC ACG GGG TAC
813 HaeII 829

CYS ASP SER GLN CYS PRO ARG ASP LEU LYS PHE ILE ASN GLY
TGT GAC AGC CAG TGT CCC CGC GAT CTG AAG TTC ATC AAT GGC
843 858 873

GLN ALA ASN VAL GLU GLY TRP GLU PRO SER SER ASN ASN ALA
CAG GCC AAC GTT GAG GGC TGG GAG CCG TCA TCC AAC AAC GCA
888 903 918

ASN THR GLY ILE GLY GLY HIS GLY SER CYS CYS SER GLU MET
AAC ACG GGC ATT GGA GGA CAC GGA AGC TGC TGC TCT GAG ATG
933 948 963

ASP ILE TRP GLU ALA ASN SER ILE SER GLU ALA LEU THR PRO
GAT ATC TGG GAG GCC AAC TCC ATC TCC GAG GCT CTT ACC CCC
978 993 1008

HIS PRO CYS THR THR VAL GLY GLN GLU ILE CYS GLU GLY ASP
CAC CCT TGC ACG ACT GTC GGC CAG GAG ATC TGC GAG GGT GAT
1023 BglII

GLY CYS GLY GLY THR TYR SER ASP ASN ARG TYR GLY GLY THR
GGG TGC GGC GGA ACT TAC TCC GAT AAC AGA TAT GGC GGC ACT
1068 1083

CYS ASP PRO ASP GLY CYS ASP TRP ASN PRO TYR ARG LEU GLY
TGC GAT CCC GAT GGC TGC GAC TGG AAC CCA TAC CGC CTG GGC
1098 1113 1128

ASN THR SER PHE TYR GLY PRO GLY SER SER PHE THR LEU ASP
AAC ACC AGC TTC TAC GGC CCT GGC TCA AGC TTT ACC CTC GAT
1143 1158 HindIII 1173
 
THR THR LYS LYS LEU THR VAL VAL THR GLN PHE GLU THR SER
ACC ACC AAG AAA TTG ACC GTT GTC ACC CAG TTC GAG ACG TCG
1188 1203 1218

GLY ALA ILE ASN ARG TYR TYR VAL GLN ASN GLY VAL THR PHE
GGT GCC ATC AAC CGA TAC TAT GTC CAG AAT GGC GTC ACT TTC
1233 1248

GLN GLN PRO ASN ALA GLU LEU GLY SER TYR SER GLY ASN GLU
CAG CAG CCC AAC GCC GAG CTT GGT AGT TAC TCT GGC AAC GAG
1263 1278 1293

LEU ASN ASP ASP TYR CYS THR ALA GLU GLU ALA GLU PHE GLY
CTC AAC GAT GAT TAC TGC ACA GCT GAG GAG GCA GAA TTC GGC
PvuII EcoRI

GLY SER SER PHE SER ASP LYS GLY GLY LEU THR GLN PHE LYS
GGA TCC TCT TTC TCA GAC AAG GGC GGC CTG ACT CAG TTC AAG
BamHI 1353 1368 1383

LYS ALA THR SER GLY GLY MET VAL LEU VAL MET SER LEU TRP
AAG GCT ACC TCT GGC GGC ATG GTT CTG GTC ATG AGT CTG TGG
1398 1413 1428

ASP ASP
GAT GAT GTGAGTTTGA TGGACAAACA TGCGCGTTGA CAAAGAGTCA
1444 1454 HincII 1474

TYR TYR ALA ASN MET LEU TRP
AGCAGCTGAC TGAGATGTTA CAG TAC TAC GCC AAC ATG CTG TGG
PvuII 1494 1512

LEU ASP SER THR TYR PRO THR ASN GLU THR SER SER THR PRO
CTG GAC TCC ACC TAC CCG ACA AAC GAG ACC TCC TCC ACA CCC
1527 1542 1557
 
GLY ALA VAL ARG GLY SER CYS SER THR SER SER GLY VAL PRO
GGT GCC GTG CGC GGA AGC TGC TCC ACC AGC TCC GGT GTC CCT
1572 1587 1602

ALA GLN VAL GLU SER GLN SER PRO ASN ALA LYS VAL THR PHE
GCT CAG GTC GAA TCT CAG TCT CCC AAC GCC AAG GTC ACC TTC
1617 1632 BstEII

SER ASN ILE LYS PHE GLY PRO ILE GLY SER THR GLY ASN PRO
TCC AAC ATC AAG TTC GGA CCC ATT GGC AGC ACC GGC AAC CCT
1647 1662 1677

SER GLY GLY ASN PRO PRO GLY GLY ASN ARG GLY THR THR THR
AGC GGC GGC AAC CCT CCC GGC GGA AAC CGT GGC ACC ACC ACC
1692 1707 1722

THR ARG ARG PRO ALA THR THR THR GLY SER SER PRO GLY PRO
ACC CGC CGC CCA GCC ACT ACC ACT GGA AGC TCT CCC GGA CCT
1737 1752 1767

THR GLN SER HIS TYR GLY GLN CYS GLY GLY ILE GLY TYR SER
ACC CAG TCT CAC TAC GGC CAG TGC GGC GGT ATT GGC TAC AGC
1782 1797 1812

GLY PRO THR VAL CYS ALA SER GLY THR THR CYS GLN VAL LEU
GGC CCC ACG GTC TGC GCC AGC GGC ACA ACT TGC CAG GTC CTG
1827 1842

ASN PRO TYR TYR SER GLN CYS LEU
AAC CCT TAC TAC TCT CAG TGC CTG TAAAGCTCCG TGCGAAAGCC
1857 1872 1888 1898

TGACGCACCG GTAGATTCTT GGTGAGCCCG TATCATGACG GCGGCGGGAG,
1908 1918 1928 1938 1948
 ou
(d) une séquence d'ADN codant pour une séquence d'amino-acides qui est sensiblement équivalente à la séquence d'amino-acides indiquée dans la revendication 1 (a), ladite séquence d'aminoacides représentant une protéine capable de cliver la cellulose en motifs de cellobiose à partir des extrémités non réductrices des chaînes polymères de la cellulose, ladite protéine étant précédée d'un peptide leader, et ladite séquence d'ADN étant détectable avec la séquence d'ADN obtenue selon le procédé de la revendication 1 (a).

3. Procédé selon la revendication 1, dans lequel ladite séquence d'ADN est :
(e) la séquence d'ADN contenue dans les indications suivantes relatives à la séquence : 
GTTACTCTGT GTTGCAAATC CAACTATAAA AGACAACAAC CGCAAACTTT GTCTTGTCGC CATCAGATTG TTCGCCAAGC ACCCTCCCCC CCCCCTATCT TAGTCCTCTT TGTGTCCCAAA ATG
MET
GCG CCC TCA GTT ACA CTG CCG TTG ACC ACG GCC ATC CTG GCC ATT GCC CGG CTC GTC GCC GCC CAG CAA CCG GGT ACC AGC ACC CCC GAG GTC CAT CCC AAG
ALA PRO SER VAL THR LEU PRO LEU THR THR ALA ILE LEU ALA ILE ALA ARG LEU VAL ALA ALA Gln Gln Pro Gly Thr Ser Thr Pro Glu Val His Pro Lys
TTG ACA ACC TAC AAG TGT ACA AAG TCC GGG GGG TGC GTG GCC CAG GAC ACC TCG GTG GTC CTT GAC TGG AAC TAC CGC TGG ATG CAC GAC GCA AAC TAC AAC
Leu Thr Thr Tyr Lys Cys Thr Lys Ser Gly Gly Cys Val Ala Gln Asp Thr Ser Val Val Leu Asp Trp Asn Tyr Arg Trp MET His Asp Ala Asn Tyr Asn
TCG TGC ACC GTC AAC GGC GGC GTC AAC ACC ACG CTC TGC CCT GAC GAG GCG ACC TGT GGC AAG AAC TGC TTC ATC GAG GGC GTC GAC TAC GCC GCC TCG GGC
Ser Cys Thr Val Asn Gly Gly Val Asn Thr Thr Leu Cys Pro Asp Glu Ala Thr Cys Gly Lys Asn Cys Phe Ile Glu Gly Val Asp Tyr Ala Ala Ser Gly
GTC ACG ACC TCG GGC AGC AGC CTC ACC ATG AAC CAG TAC ATG CCC AGC AGC TCT GGC GGC TAC AGC AGC GTC TCT CCT CGG CTG TAT CTC CTG GAC TCT GAC
Val Thr Thr Ser Gly Ser Ser Leu Thr MET Asn Gln Tyr MET Pro Ser Ser Ser Gly Gly Tyr Ser Ser Val Ser Pro Arg Leu Tyr Leu Leu Asp Ser Asp
GGT GAG TAC GTG ATG CTG AAG CTC AAC GGC CAG GAG CTG AGC TTC GAC GTC GAC CTC TCT GCT CTG CCG TGT GGA GAG AAC GGC TCG CTC TAC CTG TCT CAG
Gly Glu Tyr Val MET Leu Lys Leu Asn Gly Gln Glu Leu Ser Phe Asp Val Asp Leu Ser Ala Leu Pro Cys Gly Glu Asn Gly Ser Leu Tyr Leu Ser Gln
ATG GAC GAG AAC GGG GGC GCC AAC CAG TAT AAC ACG GCC GGT GCC AAC TAC GGG AGC GGC TAC TGC GAT GCT CAG TGC CCC GTC CAG ACA TGG AGG AAC GGC
MET Asp Glu Asn Gly Gly Ala Asn Gln Tyr Asn Thr Ala Gly Ala Asn Tyr Gly Ser Gly Tyr Cys Asp Ala Gln Cys Pro Val Gln Thr Trp Arg Asn Gly
ACC CTC AAC ACT AGC CAC CAG GGC TTC TGC TGC AAC GAG ATG GAT ATC CTG GAG GGC AAC TCG AGG GCG AAT GCC TTG ACC CCT CAC TCT TGC ACG GCC AC[?]
Thr Leu Asn Thr Ser His Gln Gly Phe Cys Cys Asn Glu MET Asp Ile Leu Glu Gly Asn Ser Arg Ala Asn Ala Leu Thr Pro His Ser Cys Thr Ala Thr
 
GCC TGC GAC TCT GCC GGT TGC GGC TTC AAC CCC TAT GGC AGC GGC TAC AAA AGg tgagcctgat gccactacta cccctttcct gggcgctctcg cggtttccca tgctgacat
Ala Cys Asp Ser Ala Gly Cys Gly Phe Asn Pro Tyr Gly Ser Gly Thr Lys Ser

ggtttccagC TAC GGC CCC GGA GAT ACC GTT GAC ACC TCC AAG ACC TTC ACC ATC ATC ACC CAG TTC AAC ACG GAC AAC GGC TCG CCC TCG GGC AAC CTT
Tyr Gly Pro Gly Asp Thr Val Asp Thr Ser Lys Thr Phe Thr Ile Ile Thr Gln Phe Asn Thr Asp Asn Gly Ser Pro Ser Gly Asn Leu

GTG AGC ATC ACC CGC AAG TAC CAG CAA AAC GGC GTC GAC ATC CCC AGC GCC CAG CCC GGC GGC GAC ACC ATC TCG TCC TGC CCG TCC GCC TCA GCC TAC GGC
Val Ser Ile Thr Arg Lys Tyr Gln Gln Asn Gly Val Asp Ile Pro Ser Ala Gln Pro Gly Gly Asp Thr Ile Ser Ser Cys Pro Ser Ala Ser Ala Tyr Gly

GGC CTC GCC ACC ATG GGC AAG GCC CTG AGC AGC GGC ATG GTG CTC GTG TTC AGC ATT TGG AAC GAC AAC AGC CAG TAC ATG AAC TGG CTC GAC AGC GGC AAC
Gly Leu Ala Thr MET Gly Lys Ala Leu Ser Ser Gly MET Val Leu Val Phe Ser Ile Trp Asn Asp Asn Ser Gln Tyr MET Asn Trp Leu Asp Ser Gly Asn

GCC GGC CCC TGC AGC AGC ACC GAG GGC AAC CCA TCC AAC ATC CTG GCC AAC AAC CCC AAC ACG CAC GTC GTC TTC TCC AAC ATC CGC TGG GGA GAC ATT GGG
Ala Gly Pro Cys Ser Ser Thr Glu Gly Asn Pro Ser Asn Ile Leu Ala Asn Asn Pro Asn Thr His Val Val Phe Ser Asn Ile Arg Trp Gly Asp Ile Gly

TCT ACT ACG AAC TCG ACT GCG CCC CCG CCC CCT GCG TCC AGC ACG TTT TCG ACT ACA CGG AGG AGC TCG ACG ACT TCG AGC AGC CCG AGC TGC ACG
Ser Thr Thr Asn Ser Thr Ala Pro Pro Pro Pro Ala Ser Ser Thr Phe Ser Thr Thr Arg Arg Ser Ser Thr Thr Ser Ser Ser Pro Ser Cys Thr

CAG ACT CAC TGG GGG CAG TGC GGT GGC ATT GGG TAC AGC TGC AAG ACG TGC ACG TCG GGC ACT ACG TGC CAG TAT AGC AAC GAC tgctcgtatc cccatgcct
Gln Thr His Trp Gly Gln Cys Gly Gly Ile Gly Tyr Ser Cys Lys Thr Cys Thr Ser Gly Thr Thr Cys Gln Tyr Ser Asn Asp

gacggggagt gatttgaga tgctaaccgc taaaataca gAC TAC TCG CAA TGC CTT TAGAGGCGTTG ACTTGCCTCT GGTCTGTCCA GACGGGGCAC GATAGAATGC GGGCACGCAG GGAGTTCGTA
Tyr Tyr Ser Gln Cys Leu
 ou
(f) une séquence d'ADN codant pour une séquence d'amino-acides qui est sensiblement équivalente à la séquence d'amino-acides indiquée dans la revendication 1 (b), où ladite séquence d'aminoacides représente une protéine qui est capable de cliver la cellulose aux sites glycosidiques internes, ladite protéine étant précédée d'un peptide leader et ladite séquence d'ADN étant détectable avec la séquence d'ADN de la revendication 1 (b).

4. Procédé selon l'une quelconque des revendications 1 à 3, qui comprend de plus l'isolement du fragment de la séquence d'ADN de l'une quelconque des revendications 1 à 3 qui code pour le peptide leader de ladite cellulase fongique.

5. Procédé selon l'une quelconque des revendications 1 à 4, dans lequel ladite séquence d'ADN est dépourvue d'introns.

6. Procédé selon l'une quelconque des revendications 1, 2(d), 3(f) ou 4, dans lequel ladite séquence d'ADN contient des introns.

7. Procédé selon l'une quelconque des revendications 1 à 6, dans lequel ladite espèce de champignon est une espèce de Trichoderma.

8. Procédé selon la revendication 7, dans lequel l'espèce de champignon est T. reesei.

9. Procédé selon l'une quelconque des revendications 1 (a), 2, 4, 5 ou 6, dans lequel la cellobiohydrolase est la cellobiohydrolase-I de T. reesei.

10. Procédé pour produire une molécule d'ADN recombinant qui comprend l'insertion d'une séquence d'ADN pouvant être obtenue selon le procédé de l'une quelconque des revendications 1 à 9 dans une molécule vecteur.

11. Procédé pour la préparation d'un vecteur d'expression pour l'expression d'une cellulase fongique mature dans une levure servant d'hôte, ledit procédé comprenant l'insertion dans une molécule vecteur dans la direction 5' à 3' des éléments suivants :
(a) une séquence promoteur de levure ; et
(b) une séquence d'ADN préparée selon le procédé de l'une quelconque des revendications 1 à 3 ou 5 à 9, ladite séquence d'ADN étant en liaison fonctionnelle avec ladite séquence promoteur.

12. Procédé selon la revendication 11, dans lequel ledit vecteur d'expression contient de plus une séquence terminateur de levure en liaison fonctionnelle avec l'extrémité 3' de ladite région codante.

13. Procédé selon la revendication 11 ou 12, dans lequel ladite séquence promoteur contient un promoteur énolase-1 de levure.

14. Procédé selon l'une quelconque des revendications 11 à 13, dans lequel ledit vecteur d'expression comprend des séquences régulatrices additionnelles compatibles avec ledit hôte en liaison fonctionnelle avec lesdites séquences d'ADN.

15. Procédé pour la préparation de cellules de levure transformées comprenant la transformation des cellules de levure avec un vecteur d'expression, ledit vecteur d'expression étant obtenu selon le procédé de l'une quelconque des revendications 11 à 14.

16. Procédé pour la préparation d'une endoglucanase-I fongique, mature, glycosylée, fonctionnellement active, comprenant la transformation de Saccharomyces cerevisiae avec un vecteur d'expression dans les levures, préparé selon le procédé de l'une quelconque des revendications 11 à 14.

17. Procédé pour le traitement de la cellulose comprenant :
(a) la transformation d'une levure avec un vecteur d'expression préparé selon le procédé de l'une quelconque des revendications 11 à 14 pour produire la cellulase fongique mature, glycosylée, fonctionnellement active ; et
(b) la réaction enzymatique de la cellulose avec la cellulase produite.

18. Procédé selon la revendication 17, dans lequel la levure transformée est capable de sécréter la cellulase.

19. Procédé selon la revendication 17 ou 18, dans lequel ladite réaction est effectuée en présence de ladite levure.

20. Procédé selon la revendication 17 ou 18, qui comprend de plus la séparation de la cellulase produite d'avec la levure avant ladite réaction.

21. Procédé selon l'une quelconque des revendications 17 à 20, dans lequel la cellulase produite est une cellobiohydrolase et ladite réaction comprend de plus la réaction de la cellulose avec une glucosidase et/ou une endoglucanase.

22. Procédé selon l'une quelconque des revendications 17 à 20, dans lequel la cellulase produite est une endoglucanase et ladite réaction comprend de plus la réaction de la cellulose avec une cellobiohydrolase et/ou une Q-glucosidase.

23. Procédé d'hydrolyse de la cellulose qui comprend le traitement de la cellulose avec les cellules de levure de la revendication 15.

24. Procédé pour la production d'une cellulase fongique mature, fonctionnellement active, comprenant les étapes de :
(a) culture de cellules de levure obtenues selon le procédé de la revendication 14 dans des conditions appropriées pour permettre la sécrétion de ladite cellulase dans le milieu ; et
(b) la récupération de ladite cellulase à partir du milieu.

## Patentansprüche (Patentansprüche für folgende(n) Vertragsstaat(en) : BE, CH, DE, FR, GB, IT, LI, NL, SE)

1. DNA-Sequenz, codierend eine Aminosäuresequenz einer ein Signalpeptid enthaltenden Pilzcellulase, wobei die Aminosäuresequenz
(a) die Aminosäuresequenz 
MET TYR ARG LYS LEU ALA VAL ILE SER ALA PHE LEU ALA THR
ALA ARG ALA GLN SER ALA CYS THR LEU GLN SER GLU THR HIS
PRO PRO LEU THR TRP GLN LYS CYS SER SER GLY GLY THR CYS
THR GLN GLN THR GLY SER VAL VAL ILE ASP ALA ASN TRP ARG
TRP THR HIS ALA THR ASN SER SER THR ASN CYS TYR ASP GLY
ASN THR TRP SER SER THR LEU CYS PRO ASP ASN GLU THR CYS
ALA LYS ASN CYS CYS LEU ASP GLY ALA ALA TYR ALA SER THR
TYR GLY VAL THR THR SER GLY ASN SER LEU SER ILE GLY PHE
VAL THR GLN SER ALA GLN LYS ASN VAL GLY ALA ARG LEU TYR
LEU MET ALA SER ASP THR THR TYR GLN GLU PHE THR LEU LEU
GLY ASN GLU PHE SER PHE ASP VAL ASP VAL SER GLN LEU PRO
CYS GLY LEU ASN GLY ALA LEU TYR PHE VAL SER MET ASP ALA
ASP GLY GLY VAL SER LYS TYR PRO THR ASN THR ALA GLY ALA
LYS TYR GLY THR GLY TYR CYS ASP SER GLN CYS PRO ARG ASP
LEU LYS PHE ILE ASN GLY GLN ALA ASN VAL GLU GLY TRP GLU
PRO SER SER ASN ASN ALA ASN THR GLY ILE GLY GLY HIS GLY
SER CYS CYS SER GLU MET ASP ILE TRP GLU ALA ASN SER ILE
SER GLU ALA LEU THR PRO HIS PRO CYS THR THR VAL GLY GLN
GLU ILE CYS GLU GLY ASP GLY CYS GLY GLY THR TYR SER ASP
ASN ARG TYR GLY GLY THR CYS ASP PRO ASP GLY CYS ASP TRP
ASN PRO TYR ARG LEU GLY ASN THR SER PHE TYR GLY PRO GLY
SER SER PHE THR LEU ASP THR THR LYS LYS LEU THR VAL VAL
THR GLN PHE GLU THR SER GLY ALA ILE ASN ARG TYR TYR VAL
GLN ASN GLY VAL THR PHE GLN GLN PRO ASN ALA GLU LEU GLY
SER TYR SER GLY ASN GLU LEU ASN ASP ASP TYR CYS THR ALA
GLU GLU ALA GLU PHE GLY GLY SER SER PHE SER ASP LYS GLY
GLY LEU THR GLN PHE LYS LYS ALA THR SER GLY GLY MET VAL
LEU VAL MET SER LEU TRP ASP ASP TYR TYR ALA ASN MET LEU
TRP LEU ASP SER THR TYR PRO THR ASN GLU THR SER SER THR
PRO GLY ALA VAL ARG GLY SER CYS SER THR SER SER GLY VAL
PRO ALA GLN VAL GLU SER GLN SER PRO ASN ALA LYS VAL THR
PHE SER ASN ILE LYS PHE GLY PRO ILE GLY SER THR GLY ASN
PRO SER GLY GLY ASN PRO PRO GLY GLY ASN ARG GLY THR THR
THR THR ARG ARG PRO ALA THR THR THR GLY SER SER PRO GLY
PRO THR GLN SER HIS TYR GLY GLN CYS GLY GLY ILE GLY TYR
SER GLY PRO THR VAL CYS ALA SER GLY THR THR CYS GLN VAL
LEU ASN PRO TYR TYR SER GLN CYS LEU
 ist, die eine Cellobiohydrolase I verkörpert oder
(b) die Aminosäuresequenz 
MET ALA PRO SER VAL THR LEU PRO LEU THR THR ALA ILE LEU
ALA ILE ALA ARG LEU VAL ALA ALA GLN GLN PRO GLY THR SER
THR PRO GLU VAL HIS PRO LYS LEU THR THR TYR LYS CYS THR
LYS SER GLY GLY CYS VAL ALA GLN ASP THR SER VAL VAL LEU
ASP TRP ASN TYR ARG TRP MET HIS ASP ALA ASN TYR ASN SER
CYS THR VAL ASN GLY GLY VAL ASN THR THR LEU CYS PRO ASP
GLU ALA THR CYS GLY LYS ASN CYS PHE ILE GLU GLY VAL ASP
TYR ALA ALA SER GLY VAL THR THR SER GLY SER SER LEU THR
MET ASN GLN TYR MET PRO SER SER SER GLY GLY TYR SER SER
VAL SER PRO ARG LEU TYR LEU LEU ASP SER ASP GLY GLU TYR
VAL MET LEU LYS LEU ASN GLY GLN GLU LEU SER PHE ASP VAL
ASP LEU SER ALA LEU PRO CYS GLY GLU ASN GLY SER LEU TYR
LEU SER GLN MET ASP GLU ASN GLY GLY ALA ASN GLN TYR ASN
THR ALA GLY ALA ASN TYR GLY SER GLY TYR CYS ASP ALA GLN
CYS PRO VAL GLN THR TRP ARG ASN GLY THR LEU ASN THR SER
HIS GLN GLY PHE CYS CYS ASN GLU MET ASP ILE LEU GLU GLY
ASN SER ARG ALA ASN ALA LEU THR PRO HIS SER CYS THR ALA
THR ALA CYS ASP SER ALA GLY CYS GLY PHE ASN PRO TYR GLY
SER GLY THR LYS SER TYR TYR GLY PRO GLY ASP THR VAL ASP
THR SER LYS THR PHE THR ILE ILE THR GLN PHE ASN THR ASP
ASN GLY SER PRO SER GLY ASN LEU VAL SER ILE THR ARG LYS
TYR GLN GLN ASN GLY VAL ASP ILE PRO SER ALA GLN PRO GLY
GLY ASP THR ILE SER SER CYS PRO SER ALA SER ALA TYR GLY
GLY LEU ALA THR MET GLY LYS ALA LEU SER SER GLY MET VAL
LEU VAL PHE SER ILE TRP ASN ASP ASN SER GLN TYR MET ASN
TRP LEU ASP SER GLY ASN ALA GLY PRO CYS SER SER THR GLU
GLY ASN PRO SER ASN ILE LEU ALA ASN ASN PRO ASN THR HIS
VAL VAL PHE SER ASN ILE ARG TRP GLY ASP ILE GLY SER THR
THR ASN SER THR ALA PRO PRO PRO PRO PRO ALA SER SER THR
THR PHE SER THR THR ARG ARG SER SER THR THR SER SER SER
PRO SER CYS THR GLN THR HIS TRP GLY GLN CYS GLY GLY ILE
GLY TYR SER GLY CYS LYS THR CYS THR SER GLY THR THR CYS
GLN TYR SER ASN ASP TYR TYR SER GLN CYS LEU
 ist, die eine Endoglucanase I verkörpert

2. DNA-Sequenz, nach Anspruch 1 (a), die
(c) die in den folgenden Sequenzdaten enthaltene DNA-Sequenz ist: 
AAGCTTAGCC AAGAACAATA GCCGATAAAG ATAGCCTCAT TAAACGGAAT
1 HindIII 20 30 40 50

GAGCTAGTAG GCAAAGTCAG CGAATGTGTA TATATAAAGG TTCGAGGTCC
60 70 80 90 100

GTGCCTCCCT CATGCTCTCC CCATCTACTC ATCAACTCAG ATCCTCCAGG
110 120 130 140 150

AGACTTGTAC ACCATCTTTT GAGGCACAGA AACCCAATAG TCAACCGCGG
160 170 180 HincII SacII

MET TYR ARG LYS LEU ALA VAL ILE SER ALA PHE
ACTGGCATC ATG TAT CGG AAG TTG GCC GTC ATC TCG GCC TTC
209 224 BglI

LEU ALA THR ALA ARG ALA GLN SER ALA CYS THR LEU GLN SER
TTG GCC ACA GCT CGT GCT CAG TCG GCC TGC ACT CTC CAA TCG
254 269 284

GLU THR HIS PRO PRO LEU THR TRP GLN LYS CYS SER SER GLY
GAG ACT CAC CCG CCT CTG ACA TGG CAG AAA TGC TCG TCT GGT
299 314

GLY THR CYS THR GLN GLN THR GLY SER VAL VAL ILE ASP ALA
GGC ACT TGC ACT CAA CAG ACA GGC TCC GTG GTC ATC GAC GCC
329 344 359

ASN TRP ARG TRP THR HIS ALA THR ASN SER SER THR ASN CYS
AAC TGG CGC TGG ACT CAC GCT ACG AAC AGC AGC ACG AAC TGC
HaeII 389 404
 
TYR ASP GLY ASN THR TRP SER SER THR LEU CYS PRO ASP ASN
TAC GAT GGC AAC ACT TGG AGC TCG ACC CTA TGT CCT GAC AAC
419 434 449

GLU THR CYS ALA LYS ASN CYS CYS LEU ASP GLY ALA ALA TYR
GAG ACC TGC GCG AAG AAC TGC TGT CTG GAC GGT GCC GCC TAC
464 479 494

ALA SER THR TYR GLY VAL THR THR SER GLY ASN SER LEU SER
GCG TCC ACG TAC GGA GTT ACC ACG AGC GGT AAC AGC CTC TCC
509 524

ILE GLY PHE VAL THR GLN SER ALA GLN LYS ASN VAL GLY ALA
ATT GGC TTT GTC ACC CAG TCT GCG CAG AAG AAC GTT GGC GCT
539 554 MstI 569

ARG LEU TYR LEU MET ALA SER ASP THR THR TYR GLN GLU PHE
CGC CTT TAC CTT ATG GCG AGC GAC ACG ACC TAC CAG GAA TTC
584 599 614 EcoRI

THR LEU LEU GLY ASN GLU PHE SER PHE ASP VAL ASP VAL SER
ACC CTG CTT GGC AAC GAG TTC TCT TTC GAT GTT GAT GTT TCG
629 644 659

GLN LEU PRO
CAG CTG CCG TAAGTGACTT ACCATGAACC CCTGACGTAT CTTCTTGTGG
PvuII 681 691 701 711

CYS GLY LEU ASN GLY ALA
GCTCCCAGCT GACTGGCCAA TTTAAGG TGC GGC TTG AAC GGA GCT
PvuII 731 753

LEU TYR PHE VAL SER MET ASP ALA ASP GLY GLY VAL SER LYS
CTC TAC TTC GTG TCC ATG GAC GCG GAT GGT GGC GTG AGC AAG
768 NcoI 783 798
 
TYR PRO THR ASN THR ALA GLY ALA LYS TYR GLY THR GLY TYR
TAT CCC ACC AAC ACC GCT GGC GCC AAG TAC GGC ACG GGG TAC
813 HaeII 829

CYS ASP SER GLN CYS PRO ARG ASP LEU LYS PHE ILE ASN GLY
TGT GAC AGC CAG TGT CCC CGC GAT CTG AAG TTC ATC AAT GGC
843 858 873

GLN ALA ASN VAL GLU GLY TRP GLU PRO SER SER ASN ASN ALA
CAG GCC AAC GTT GAG GGC TGG GAG CCG TCA TCC AAC AAC GCA
888 903 918

ASN THR GLY ILE GLY GLY HIS GLY SER CYS CYS SER GLU MET
AAC ACG GGC ATT GGA GGA CAC GGA AGC TGC TGC TCT GAG ATG
933 948 963

ASP ILE TRP GLU ALA ASN SER ILE SER GLU ALA LEU THR PRO
GAT ATC TGG GAG GCC AAC TCC ATC TCC GAG GCT CTT ACC CCC
978 993 1008

HIS PRO CYS THR THR VAL GLY GLN GLU ILE CYS GLU GLY ASP
CAC CCT TGC ACG ACT GTC GGC CAG GAG ATC TGC GAG GGT GAT
1023 BglII

GLY CYS GLY GLY THR TYR SER ASP ASN ARG TYR GLY GLY THR
GGG TGC GGC GGA ACT TAC TCC GAT AAC AGA TAT GGC GGC ACT
1068 1083

CYS ASP PRO ASP GLY CYS ASP TRP ASN PRO TYR ARG LEU GLY
TGC GAT CCC GAT GGC TGC GAC TGG AAC CCA TAC CGC CTG GGC
1098 1113 1128

ASN THR SER PHE TYR GLY PRO GLY SER SER PHE THR LEU ASP
AAC ACC AGC TTC TAC GGC CCT GGC TCA AGC TTT ACC CTC GAT
1143 1158 HindIII 1173
 
THR THR LYS LYS LEU THR VAL VAL THR GLN PHE GLU THR SER
ACC ACC AAG AAA TTG ACC GTT GTC ACC CAG TTC GAG ACG TCG
1188 1203 1218

GLY ALA ILE ASN ARG TYR TYR VAL GLN ASN GLY VAL THR PHE
GGT GCC ATC AAC CGA TAC TAT GTC CAG AAT GGC GTC ACT TTC
1233 1248

GLN GLN PRO ASN ALA GLU LEU GLY SER TYR SER GLY ASN GLU
CAG CAG CCC AAC GCC GAG CTT GGT AGT TAC TCT GGC AAC GAG
1263 1278 1293

LEU ASN ASP ASP TYR CYS THR ALA GLU GLU ALA GLU PHE GLY
CTC AAC GAT GAT TAC TGC ACA GCT GAG GAG GCA GAA TTC GGC
PvuII EcoRI

GLY SER SER PHE SER ASP LYS GLY GLY LEU THR GLN PHE LYS
GGA TCC TCT TTC TCA GAC AAG GGC GGC CTG ACT CAG TTC AAG
BamHI 1353 1368 1383

LYS ALA THR SER GLY GLY MET VAL LEU VAL MET SER LEU TRP
AAG GCT ACC TCT GGC GGC ATG GTT CTG GTC ATG AGT CTG TGG
1398 1413 1428

ASP ASP
GAT GAT GTGAGTTTGA TGGACAAACA TGCGCGTTGA CAAAGAGTCA
1444 1454 HincII 1474

TYR TYR ALA ASN MET LEU TRP
AGCAGCTGAC TGAGATGTTA CAG TAC TAC GCC AAC ATG CTG TGG
PvuII 1494 1512

LEU ASP SER THR TYR PRO THR ASN GLU THR SER SER THR PRO
CTG GAC TCC ACC TAC CCG ACA AAC GAG ACC TCC TCC ACA CCC
1527 1542 1557
 
GLY ALA VAL ARG GLY SER CYS SER THR SER SER GLY VAL PRO
GGT GCC GTG CGC GGA AGC TGC TCC ACC AGC TCC GGT GTC CCT
1572 1587 1602

ALA GLN VAL GLU SER GLN SER PRO ASN ALA LYS VAL THR PHE
GCT CAG GTC GAA TCT CAG TCT CCC AAC GCC AAG GTC ACC TTC
1617 1632 BstEII

SER ASN ILE LYS PHE GLY PRO ILE GLY SER THR GLY ASN PRO
TCC AAC ATC AAG TTC GGA CCC ATT GGC AGC ACC GGC AAC CCT
1647 1662 1677

SER GLY GLY ASN PRO PRO GLY GLY ASN ARG GLY THR THR THR
AGC GGC GGC AAC CCT CCC GGC GGA AAC CGT GGC ACC ACC ACC
1692 1707 1722

THR ARG ARG PRO ALA THR THR THR GLY SER SER PRO GLY PRO
ACC CGC CGC CCA GCC ACT ACC ACT GGA AGC TCT CCC GGA CCT
1737 1752 1767

THR GLN SER HIS TYR GLY GLN CYS GLY GLY ILE GLY TYR SER
ACC CAG TCT CAC TAC GGC CAG TGC GGC GGT ATT GGC TAC AGC
1782 1797 1812

GLY PRO THR VAL CYS ALA SER GLY THR THR CYS GLN VAL LEU
GGC CCC ACG GTC TGC GCC AGC GGC ACA ACT TGC CAG GTC CTG
1827 1842

ASN PRO TYR TYR SER GLN CYS LEU
AAC CCT TAC TAC TCT CAG TGC CTG TAAAGCTCCG TGCGAAAGCC
1857 1872 1888 1898

TGACGCACCG GTAGATTCTT GGTGAGCCCG TATCATGACG GCGGCGGGAG
1908 1918 1928 1938 1948
 oder
(d) eine DNA-Sequenz ist, die eine zur in Anspruch 1 (a) angegebenen Aminosäuresequenz im wesentlichen äquivalente Aminosäuresequenz codiert, wobei die Aminosäuresequenz ein Protein verkörpert, das in der Lage ist, von den nicht-reduzierenden Enden der Cellulose-Polymerketten Cellulose in Cellubioseeinheiten zu spalten, wobei dem Protein ein Signalpeptid vorangeht und wobei die DNA-Sequenz mit der DNA-Sequenz von Anspruch 1 (a) nachweisbar ist.

3. DNA-Sequenz nach Anspruch 1 (b), die
(e) die DNA-Sequenz ist, die in den folgenden Sequenzdaten enthalten ist: 
GTTACCCTGT GTTTGCAAATC CAACTTATAA ACACAACAAC CGCAAACTTT GTCTTGTCGTC CATCAGATTG TTCGCCAAGC ACCCTCCCCC CCCCCTATCT TAGTCCTTCT TGTGTCCCAA ATG
MET

GCG CCC TCA GTT ACA CTG CCG TTG ACC ACG GCC ATC CTG GCC ATT GCC CGG CTC GTC GCC GCC CAG CAA CCG GGT ACC AGC ACC CCC GAG GTC CAT CCC AAG
ALA PRO SER VAL THR LEU PRO LEU THR THR ALA ILE LEU ALA ILE ALA ARG LEU VAL ALA ALA Gln Gln Pro Gly Thr Ser Thr Pro Glu Val His Pro Lys

TTG ACA ACC TAC AAG TGT ACA AAG TCC GGG GGG TGC GTG GCC CAG GAC ACC TCG GTG GTC CTT GAC TGG AAC TAC CGC TGG ATG CAC GAC GCA AAC TAC AAC
Leu Thr Thr Tyr Lys Cys Thr Lys Ser Gly Gly Cys Val Ala Gln Asp Thr Ser Val Val Leu Asp Trp Asn Tyr Arg Trp Met His Asp Ala Asn Tyr Asn

TCG TGC ACC GTC AAC GGC GGC GTC AAC ACC ACG CTC TGC CCT GAC GAG GCG ACC TGT GGC AAG AAC TGC TTC ATC GAG GGC GTC GAC TAC GCC GCC TCG GGC
Ser Cys Thr Val Asn Gly Gly Val Asn Thr Thr Leu Cys Pro Asp Glu Ala Thr Cys Gly Lys Asn Cys Phe Ile Glu Gly Val Asp Tyr Ala Ala Ser Gly

GTC ACG ACC TCG GGC AGC AGC CTC ACC ATG AAC CAG TAC ATG CCC AGC AGC TCT GGC GGC TAC AGC AGC GTC TCT CCT CGG CTG TAT CTC CTG GAC TCT GAC
Val Thr Thr Ser Gly Ser Ser Leu Thr Met Asn Gln Tyr Met Pro Ser Ser Ser Gly Gly Tyr Ser Ser Val Ser Pro Arg Leu Tyr Leu Leu Asp Ser Asp

GGT GAG TAC GTG ATG CTG AAG CTC AAC GGC CAG GAG CTG AGC TTC GAC GTC GAC CTC TCT GCT CTG CCG TGT GGA GAG AAC GGC TCG CTC TAC CTG TCT CAG
Gly Glu Tyr Val Met Leu Lys Leu Asn Gly Gln Glu Leu Ser Phe Asp Val Asp Leu Ser Ala Leu Pro Cys Gly Glu Asn Gly Ser Leu Tyr Leu Ser Gln

ATG GAC GAG AAC GGG GGC GCC AAC CAG TAT AAC ACG GCC GGT GCC AAC TAC GGG AGC GGC TAC TGC GAT GCT CAG TGC CCC GTC CAG ACA TGG AGG AAC GGC
Met Asp Glu Asn Gly Gly Ala Asn Gln Tyr Asn Thr Ala Gly Ala Asn Tyr Gly Ser Gly Tyr Cys Asp Ala Gln Cys Pro Val Gln Thr Trp Arg Asn Gly

ACC CTC AAC ACT AGC CAC CAG GGC TTC TGC TGC AAC GAG ATG GAT ATC CTG GAG GGC AAC TCG AGG GCG AAT GCC TTG ACC CCT CAC TCT TGC ACG GCC ACG
Thr Leu Asn Thr Ser His Gln Gly Phe Cys Cys Asn Glu Met Asp Ile Leu Glu Gly Asn Ser Arg Ala Asn Ala Leu Thr Pro His Ser Cys Thr Ala Thr
 
GCC TGC GAC TCT GCC GGT TGC GGC TTC AAC CCC TAT GGC AGC GGC TAC AAA AGg tgagcctgat gccactacta cccctttcct ggcgctctcg cggtttccca tgctgacat
Ala Cys Asp Ser Ala Gly Cys Gly Phe Asn Pro Tyr Gly Ser Gly Thr Lys Ser

ggttttccagC TAC TAC GGC CCC GGA GAT ACC GTT GAC ACT TCC AAG ACC TTC ACC ATC ATC ACC CAG TTC AAC ACG GAC AAC GGC TCG CCC TCG GGC AAC CTT
Tyr Tyr Gly Pro Gly Asp Thr Val Asp Thr Ser Lys Thr Phe Thr Ile Ile Thr Gln Phe Asn Thr Asp Asn Gly Ser Pro Ser Gly Asn Leu

GTG AGC ATC ACC CGC AAG TAC CAG CAA AAC GGC GTC GAC ATC CCC AGC GCC CAG CCC GGC GGC GAC ACC ATC TCG TCC TGC CCG TCC GCC TCA GCC TAC GGC
Val Ser Ile Thr Arg Lys Tyr Gln Gln Asn Gly Val Asp Ile Pro Ser Ala Gln Pro Gly Gly Asp Thr Ile Ser Ser Cys Pro Ser Ala Ser Ala Tyr Gly

GGC CTC GCC ACC ATG GGC AAG GCC CTG AGC AGC GGC ATG GTG CTC GTG TTC AGC ATT TGG AAC GAC AAC AGC CAG TAC ATG AAC TGG CTC GAC AGC GGC AAC
Gly Leu Ala Thr MET Gly Lys Ala Leu Ser Ser Gly MET Val Leu Val Phe Ser Ile Trp Asn Asp Asn Ser Gln Tyr MET Asn Trp Leu Asp Ser Gly Asn

GCC GGC CCC TGC AGC AGC ACC GAG GGC AAC CCA TCC AAC ATC CTG GCC AAC AAC CCC AAC ACG CAC GTC GTC TTC TCC AAC ATC CGC TGG GGA GAC ATT GGG
Ala Gly Pro Cys Ser Ser Thr Glu Gly Asn Pro Ser Asn Ile Leu Ala Asn Asn Pro Asn Thr His Val Val Phe Ser Asn Ile Arg Trp Gly Asp Ile Gly

TCT ACT ACG AAT TCG ACT GCG CCC CCG CCC CCT GCG TCC AGC ACG ACG TTT TCG ACT ACA CGG AGG AGC TCG ACG ACT TCG AGC AGC CCG AGC TGC ACG
Ser Thr Thr Asn Ser Thr Ala Pro Pro Pro Pro Ala Ser Ser Thr Thr Phe Ser Thr Thr Arg Arg Ser Ser Thr Thr Ser Ser Ser Pro Ser Cys Thr

CAG ACT CAC TGG GGG CAG TGC GGT GGC ATT GGG TAC AGC GGG TGC AAG ACG TGC ACG TCG GGC ACT ACG TGC CAG TAT AGC AAC GAC tgtcgtcatc ccccatgcct
Gln Thr His Trp Gly Gln Cys Gly Gly Ile Gly Tyr Ser Gly Cys Lys Thr Cys Thr Ser Gly Thr Thr Cys Gln Tyr Ser Asn Asp

gacgggagt gatttagagc tgctaaccgc taaaatacagAC TAC TCG CAA TGC CTT TAGAGGCGTG ACTTGCCTCT GGTCTGTCCA GACGGGGCAC GATAGAATGC GGGCACGCAG , GGAGCTCGTA
Tyr Tyr Ser Gln Cys Leu
 oder
(f) eine DNA-Sequenz ist, die eine zur in Anspruch 1 (b) angegebenen Aminosäuresequenz im wesentlichen äquivalente Aminosäuresequenz codiert, wobei die Aminosäuresequenz ein Protein verkörpert, das in der Lage ist, Cellulose an internen glykosidischen Stellen zu spalten, wobei dem Protein ein Signalpeptid vorangeht, und wobei die DNA-Sequenz mit der DNA-Sequenz von Anspruch 1 (b) nachweisbar ist.

4. Fragment der DNA-Sequenz gemäß einem der Ansprüche 1 bis 3, das das Signalpeptid der Pilzcellulase codiert.

5. DNA-Sequenz nach einem der Ansprüche 1 bis 4, die frei von Introns ist.

6. DNA-Sequenz nach einem der Ansprüche 1, 2(d), 3(f) oder 4, die Introns enthält.

7. DNA-Sequence nach einem der Ansprüche 1 bis 6, wobei die Pilzart eine Art von Trichoderma ist.

8. DNA-Sequenz nach Anspruch 7, wobei die Pilzart T. reesei ist.

9. DNA-Sequenz nach einem der Ansprüche 1 (a), 2, 4, 5 oder 6, wobei die Cellobiohydrolase die T. reesei-Cellobiohydrolase-I ist.

10. Expressionsvektor zur Expression einer reifen Pilzcellulase in Hefe als Wirt, der in einer 5'- nach -3'-Richtung
(a) eine Hefepromotor-Sequenz; und
(b) eine mit der Promotorsequenz in funktionell verknüpfte DNA-Sequenz gemäß einem der Ansprüche 1 bis 3 oder 5 bis 8 umfaßt.

11. Expressionsvektor nach Anspruch 10, der außerdem eine mit dem 3'-Ende des codierenden Bereiches funktionell verbundene Hefe-Terminator-Sequenz enthält.

12. Vektor nach Anspruch 10 oder 11, wobei die Promotorsequenz einen Hefe-Enolase-1-Promotor enthält.

13. Vektor nach einem der Ansprüche 10 bis 12, der zusätzliche mit dem Wirt kompatible Kontrollsequenzen umfaßt, die mit der DNA-Sequenz in funktionsfähiger Weise verknüpft sind.

14. Hefezellen, die mit einem Expressionsvektor nach einem der Ansprüche 10 bis 13 transformiert sind.

15. Reife, glykosylierte funktionell aktive Pilzendoglucanase-I, die durch mit einem Hefeexpressionsvektor nach einem der Ansprüche 10 bis 13 transformierte Saccharomyces cerivisiae produziert wird.

16. Verfahren zur Behandlung von Cellulose, umfassend:
(a) Transformieren von Hefe mit einem Expressionsvektor nach einem der Ansprüche 10 bis 13, um die reife, glykosylierte, funktionell aktive Pilzcellulase zu produzieren; und
(b) die enzymatische Umsetzung der Cellulose mit der produzierte Cellulase.

17. Verfahren nach Anspruch 16, wobei die transformierte Hefe in der Lage ist, Cellulase zu sekretieren.

18. Verfahren nach Anspruch 16 oder 17, wobei die Umsetzung in Anwesenheit der Hefe ausgeführt wird.

19. Verfahren nach Anspruch 16 oder 17, das außerdem vor der Umsetzung das Abtrennen der produzierten Cellulase von der Hefe umfaßt.

20. Verfahren nach einem der Ansprüche 16 bis 19, wobei die hergestellte Cellulase eine Cellobiohydrolase ist, und wobei die Umsetzung ferner eine Umsetzung der Cellulase mit einer β-Glucosidase und/oder einer Endoglucanase einschließt.

21. Verfahren nach einem der Ansprüche 16 bis 19, wobei die produzierte Cellulase eine Endoglucanase ist, und die Umsetzung ferner eine Umsetzung der Cellulose mit einer Cellobiohydrolase und/oder einer β-Glucosidase einschließt.

22. Verfahren zur Hydrolyse von Cellulose, das eine Behandlung der Cellulose mit den Hefezellen gemäß Anspruch 14 umfaßt.

23. Verfahren zur Herstellung einer reifen, funktionell aktiven Pilzcellulase, das die Schritte
(a) Züchten der Hefezellen gemäß Anspruch 14 unter geeigneten Bedingungen, die die Sekretion der Cellulase in das Medium erlauben; und
(b) Gewinnung der Cellulase aus dem Medium, umfaßt.

## Patentansprüche (Patentansprüche für folgende(n) Vertragsstaat(en) : AT)

1. Verfahren zur Herstellung einer DNA-Sequenz, die eine Aminosäuresequenz einer ein Signalpeptid enthaltenden Pilzcellulase codiert, wobei die Aminosäuresequenz
(a) die Aminosäuresequenz 
MET TYR ARG LYS LEU ALA VAL ILE SER ALA PHE LEU ALA THR
ALA ARG ALA GLN SER ALA CYS THR LEU GLN SER GLU THR HIS
PRO PRO LEU THR TRP GLN LYS CYS SER SER GLY GLY THR CYS
THR GLN GLN THR GLY SER VAL VAL ILE ASP ALA ASN TRP ARG
TRP THR HIS ALA THR ASN SER SER THR ASN CYS TYR ASP GLY
ASN THR TRP SER SER THR LEU CYS PRO ASP ASN GLU THR CYS
ALA LYS ASN CYS CYS LEU ASP GLY ALA ALA TYR ALA SER THR
TYR GLY VAL THR THR SER GLY ASN SER LEU SER ILE GLY PHE
VAL THR GLN SER ALA GLN LYS ASN VAL GLY ALA ARG LEU TYR
LEU MET ALA SER ASP THR THR TYR GLN GLU PHE THR LEU LEU
GLY ASN GLU PHE SER PHE ASP VAL ASP VAL SER GLN LEU PRO
CYS GLY LEU ASN GLY ALA LEU TYR PHE VAL SER MET ASP ALA
ASP GLY GLY VAL SER LYS TYR PRO THR ASN THR ALA GLY ALA
LYS TYR GLY THR GLY TYR CYS ASP SER GLN CYS PRO ARG ASP
LEU LYS PHE ILE ASN GLY GLN ALA ASN VAL GLU GLY TRP GLU
PRO SER SER ASN ASN ALA ASN THR GLY ILE GLY GLY HIS GLY
SER CYS CYS SER GLU MET ASP ILE TRP GLU ALA ASN SER ILE
SER GLU ALA LEU THR PRO HIS PRO CYS THR THR VAL GLY GLN
GLU ILE CYS GLU GLY ASP GLY CYS GLY GLY THR TYR SER ASP
ASN ARG TYR GLY GLY THR CYS ASP PRO ASP GLY CYS ASP TRP
ASN PRO TYR ARG LEU GLY ASN THR SER PHE TYR GLY PRO GLY
SER SER PHE THR LEU ASP THR THR LYS LYS LEU THR VAL VAL
THR GLN PHE GLU THR SER GLY ALA ILE ASN ARG TYR TYR VAL
GLN ASN GLY VAL THR PHE GLN GLN PRO ASN ALA GLU LEU GLY
SER TYR SER GLY ASN GLU LEU ASN ASP ASP TYR CYS THR ALA
GLU GLU ALA GLU PHE GLY GLY SER SER PHE SER ASP LYS GLY
GLY LEU THR GLN PHE LYS LYS ALA THR SER GLY GLY MET VAL
LEU VAL MET SER LEU TRP ASP ASP TYR TYR ALA ASN MET LEU
TRP LEU ASP SER THR TYR PRO THR ASN GLU THR SER SER THR
PRO GLY ALA VAL ARG GLY SER CYS SER THR SER SER GLY VAL

PRO ALA GLN VAL GLU SER GLN SER PRO ASN ALA LYS VAL THR
PHE SER ASN ILE LYS PHE GLY PRO ILE GLY SER THR GLY ASN
PRO SER GLY GLY ASN PRO PRO GLY GLY ASN ARG GLY THR THR
THR THR ARG ARG PRO ALA THR THR THR GLY SER SER PRO GLY
PRO THR GLN SER HIS TYR GLY GLN CYS GLY GLY ILE GLY TYR
SER GLY PRO THR VAL CYS ALA SER GLY THR THR CYS GLN VAL
LEU ASN PRO TYR TYR SER GLN CYS LEU
 ist, die eine Cellobiohydrolase I verkörpert; oder
(b) die Aminosäuresequenz 
MET ALA PRO SER VAL THR LEU PRO LEU THR THR ALA ILE LEU
ALA ILE ALA ARG LEU VAL ALA ALA GLN GLN PRO GLY THR SER
THR PRO GLU VAL HIS PRO LYS LEU THR THR TYR LYS CYS THR
LYS SER GLY GLY CYS VAL ALA GLN ASP THR SER VAL VAL LEU
ASP TRP ASN TYR ARG TRP MET HIS ASP ALA ASN TYR ASN SER
CYS THR VAL ASN GLY GLY VAL ASN THR THR LEU CYS PRO ASP
GLU ALA THR CYS GLY LYS ASN CYS PHE ILE GLU GLY VAL ASP
TYR ALA ALA SER GLY VAL THR THR SER GLY SER SER LEU THR
MET ASN GLN TYR MET PRO SER SER SER GLY GLY TYR SER SER
VAL SER PRO ARG LEU TYR LEU LEU ASP SER ASP GLY GLU TYR
VAL MET LEU LYS LEU ASN GLY GLN GLU LEU SER PHE ASP VAL
ASP LEU SER ALA LEU PRO CYS GLY GLU ASN GLY SER LEU TYR
LEU SER GLN MET ASP GLU ASN GLY GLY ALA ASN GLN TYR ASN
THR ALA GLY ALA ASN TYR GLY SER GLY TYR CYS ASP ALA GLN
CYS PRO VAL GLN THR TRP ARG ASN GLY THR LEU ASN THR SER
HIS GLN GLY PHE CYS CYS ASN GLU MET ASP ILE LEU GLU GLY
ASN SER ARG ALA ASN ALA LEU THR PRO HIS SER CYS THR ALA
THR ALA CYS ASP SER ALA GLY CYS GLY PHE ASN PRO TYR GLY
SER GLY THR LYS SER TYR TYR GLY PRO GLY ASP THR VAL ASP
THR SER LYS THR PHE THR ILE ILE THR GLN PHE ASN THR ASP
ASN GLY SER PRO SER GLY ASN LEU VAL SER ILE THR ARG LYS
TYR GLN GLN ASN GLY VAL ASP ILE PRO SER ALA GLN PRO GLY
GLY ASP THR ILE SER SER CYS PRO SER ALA SER ALA TYR GLY
GLY LEU ALA THR MET GLY LYS ALA LEU SER SER GLY MET VAL
LEU VAL PHE SER ILE TRP ASN ASP ASN SER GLN TYR MET ASN
TRP LEU ASP SER GLY ASN ALA GLY PRO CYS SER SER THR GLU
GLY ASN PRO SER ASN ILE LEU ALA ASN ASN PRO ASN THR HIS
VAL VAL PHE SER ASN ILE ARG TRP GLY ASP ILE GLY SER THR
THR ASN SER THR ALA PRO PRO PRO PRO PRO ALA SER SER THR
THR PHE SER THR THR ARG ARG SER SER THR THR SER SER SER
PRO SER CYS THR GLN THR HIS TRP GLY GLN CYS GLY GLY ILE
GLY TYR SER GLY CYS LYS THR CYS THR SER GLY THR THR CYS
GLN TYR SER ASN ASP TYR TYR SER GLN CYS LEU
 ist, die eine Endoglucanase I verkörpert, wobei das Verfahren das Isolieren der DNA-Sequenz aus einer von einem Cellulase produzierenden Pilz abgeleiteten Bank umfaßt.

2. Verfahren nach Anspruch 1 (a), wobei die DNA-Sequenz
(c) die in den folgenden Sequenzdaten enthaltene DNA-Sequenz ist:
(c) die in den folgenden Sequenzdaten enthaltene DNA-Sequenz ist: 
AAGCTTAGCC AAGAACAATA GCCGATAAAG ATAGCCTCAT TAAACGGAAT
1 HindIII 20 30 40 50

GAGCTAGTAG GCAAAGTCAG CGAATGTGTA TATATAAAGG TTCGAGGTCC
60 70 80 90 100

GTGCCTCCCT CATGCTCTCC CCATCTACTC ATCAACTCAG ATCCTCCAGG
110 120 130 140 150

AGACTTGTAC ACCATCTTTT GAGGCACAGA AACCCAATAG TCAACCGCGG
160 170 180 HincII SacII

MET TYR ARG LYS LEU ALA VAL ILE SER ALA PHE
ACTGGCATC ATG TAT CGG AAG TTG GCC GTC ATC TCG GCC TTC
209 224 BglI

LEU ALA THR ALA ARG ALA GLN SER ALA CYS THR LEU GLN SER
TTG GCC ACA GCT CGT GCT CAG TCG GCC TGC ACT CTC CAA TCG
254 269 284

GLU THR HIS PRO PRO LEU THR TRP GLN LYS CYS SER SER GLY
GAG ACT CAC CCG CCT CTG ACA TGG CAG AAA TGC TCG TCT GGT
299 314

GLY THR CYS THR GLN GLN THR GLY SER VAL VAL ILE ASP ALA
GGC ACT TGC ACT CAA CAG ACA GGC TCC GTG GTC ATC GAC GCC
329 344 359

ASN TRP ARG TRP THR HIS ALA THR ASN SER SER THR ASN CYS
AAC TGG CGC TGG ACT CAC GCT ACG AAC AGC AGC ACG AAC TGC
HaeII 389 404
 
TYR ASP GLY ASN THR TRP SER SER THR LEU CYS PRO ASP ASN
TAC GAT GGC AAC ACT TGG AGC TCG ACC CTA TGT CCT GAC AAC
419 434 449

GLU THR CYS ALA LYS ASN CYS CYS LEU ASP GLY ALA ALA TYR
GAG ACC TGC GCG AAG AAC TGC TGT CTG GAC GGT GCC GCC TAC
464 479 494

ALA SER THR TYR GLY VAL THR THR SER GLY ASN SER LEU SER
GCG TCC ACG TAC GGA GTT ACC ACG AGC GGT AAC AGC CTC TCC
509 524

ILE GLY PHE VAL THR GLN SER ALA GLN LYS ASN VAL GLY ALA
ATT GGC TTT GTC ACC CAG TCT GCG CAG AAG AAC GTT GGC GCT
539 554 MstI 569

ARG LEU TYR LEU MET ALA SER ASP THR THR TYR GLN GLU PHE
CGC CTT TAC CTT ATG GCG AGC GAC ACG ACC TAC CAG GAA TTC
584 599 614 EcoRI

THR LEU LEU GLY ASN GLU PHE SER PHE ASP VAL ASP VAL SER
ACC CTG CTT GGC AAC GAG TTC TCT TTC GAT GTT GAT GTT TCG
629 644 659

GLN LEU PRO
CAG CTG CCG TAAGTGACTT ACCATGAACC CCTGACGTAT CTTCTTGTGG
PvuII 681 691 701 711

CYS GLY LEU ASN GLY ALA
GCTCCCAGCT GACTGGCCAA TTTAAGG TGC GGC TTG AAC GGA GCT
PvuII 731 753

LEU TYR PHE VAL SER MET ASP ALA ASP GLY GLY VAL SER LYS
CTC TAC TTC GTG TCC ATG GAC GCG GAT GGT GGC GTG AGC AAG
768 NcoI 783 798
 
TYR PRO THR ASN THR ALA GLY ALA LYS TYR GLY THR GLY TYR
TAT CCC ACC AAC ACC GCT GGC GCC AAG TAC GGC ACG GGG TAC
813 HaeII 829

CYS ASP SER GLN CYS PRO ARG ASP LEU LYS PHE ILE ASN GLY
TGT GAC AGC CAG TGT CCC CGC GAT CTG AAG TTC ATC AAT GGC
843 858 873

GLN ALA ASN VAL GLU GLY TRP GLU PRO SER SER ASN ASN ALA
CAG GCC AAC GTT GAG GGC TGG GAG CCG TCA TCC AAC AAC GCA
888 903 918

ASN THR GLY ILE GLY GLY HIS GLY SER CYS CYS SER GLU MET
AAC ACG GGC ATT GGA GGA CAC GGA AGC TGC TGC TCT GAG ATG
933 948 963

ASP ILE TRP GLU ALA ASN SER ILE SER GLU ALA LEU THR PRO
GAT ATC TGG GAG GCC AAC TCC ATC TCC GAG GCT CTT ACC CCC
978 993 1008

HIS PRO CYS THR THR VAL GLY GLN GLU ILE CYS GLU GLY ASP
CAC CCT TGC ACG ACT GTC GGC CAG GAG ATC TGC GAG GGT GAT
1023 BglII

GLY CYS GLY GLY THR TYR SER ASP ASN ARG TYR GLY GLY THR
GGG TGC GGC GGA ACT TAC TCC GAT AAC AGA TAT GGC GGC ACT
1068 1083

CYS ASP PRO ASP GLY CYS ASP TRP ASN PRO TYR ARG LEU GLY
TGC GAT CCC GAT GGC TGC GAC TGG AAC CCA TAC CGC CTG GGC
1098 1113 1128

ASN THR SER PHE TYR GLY PRO GLY SER SER PHE THR LEU ASP
AAC ACC AGC TTC TAC GGC CCT GGC TCA AGC TTT ACC CTC GAT
1143 1158 HindIII 1173
 
THR THR LYS LYS LEU THR VAL VAL THR GLN PHE GLU THR SER
ACC ACC AAG AAA TTG ACC GTT GTC ACC CAG TTC GAG ACG TCG
1188 1203 1218

GLY ALA ILE ASN ARG TYR TYR VAL GLN ASN GLY VAL THR PHE
GGT GCC ATC AAC CGA TAC TAT GTC CAG AAT GGC GTC ACT TTC
1233 1248

GLN GLN PRO ASN ALA GLU LEU GLY SER TYR SER GLY ASN GLU
CAG CAG CCC AAC GCC GAG CTT GGT AGT TAC TCT GGC AAC GAG
1263 1278 1293

LEU ASN ASP ASP TYR CYS THR ALA GLU GLU ALA GLU PHE GLY
CTC AAC GAT GAT TAC TGC ACA GCT GAG GAG GCA GAA TTC GGC
PvuII EcoRI

GLY SER SER PHE SER ASP LYS GLY GLY LEU THR GLN PHE LYS
GGA TCC TCT TTC TCA GAC AAG GGC GGC CTG ACT CAG TTC AAG
BamHI 1353 1368 1383

LYS ALA THR SER GLY GLY MET VAL LEU VAL MET SER LEU TRP
AAG GCT ACC TCT GGC GGC ATG GTT CTG GTC ATG AGT CTG TGG
1398 1413 1428

ASP ASP
GAT GAT GTGAGTTTGA TGGACAAACA TGCGCGTTGA CAAAGAGTCA
1444 1454 HincII 1474

TYR TYR ALA ASN MET LEU TRP
AGCAGCTGAC TGAGATGTTA CAG TAC TAC GCC AAC ATG CTG TGG
PvuII 1494 1512

LEU ASP SER THR TYR PRO THR ASN GLU THR SER SER THR PRO
CTG GAC TCC ACC TAC CCG ACA AAC GAG ACC TCC TCC ACA CCC
1527 1542 1557
 
GLY ALA VAL ARG GLY SER CYS SER THR SER SER GLY VAL PRO
GGT GCC GTG CGC GGA AGC TGC TCC ACC AGC TCC GGT GTC CCT
1572 1587 1602

ALA GLN VAL GLU SER GLN SER PRO ASN ALA LYS VAL THR PHE
GCT CAG GTC GAA TCT CAG TCT CCC AAC GCC AAG GTC ACC TTC
1617 1632 BstEII

SER ASN ILE LYS PHE GLY PRO ILE GLY SER THR GLY ASN PRO
TCC AAC ATC AAG TTC GGA CCC ATT GGC AGC ACC GGC AAC CCT
1647 1662 1677

SER GLY GLY ASN PRO PRO GLY GLY ASN ARG GLY THR THR THR
AGC GGC GGC AAC CCT CCC GGC GGA AAC CGT GGC ACC ACC ACC
1692 1707 1722

THR ARG ARG PRO ALA THR THR THR GLY SER SER PRO GLY PRO
ACC CGC CGC CCA GCC ACT ACC ACT GGA AGC TCT CCC GGA CCT
1737 1752 1767

THR GLN SER HIS TYR GLY GLN CYS GLY GLY ILE GLY TYR SER
ACC CAG TCT CAC TAC GGC CAG TGC GGC GGT ATT GGC TAC AGC
1782 1797 1812

GLY PRO THR VAL CYS ALA SER GLY THR THR CYS GLN VAL LEU
GGC CCC ACG GTC TGC GCC AGC GGC ACA ACT TGC CAG GTC CTG
1827 1842

ASN PRO TYR TYR SER GLN CYS LEU
AAC CCT TAC TAC TCT CAG TGC CTG TAAAGCTCCG TGCGAAAGCC
1857 1872 1888 1898

TGACGCACCG GTAGATTCTT GGTGAGCCCG TATCATGACG GCGGCGGGAG,
1908 1918 1928 1938 1948
 oder
(d) eine DNA-Sequenz ist, die eine der in Anspruch 1 (a) angegebenen Aminosäuresequenz äquivalente Aminosäuresequenz codiert, wobei die Aminosäuresequenz ein Protein verkörpert, das in der Lage ist, von den nicht-reduzierenden Enden der Cellulose-Polymerketten Cellulose in Cellubioseeinheiten zu spalten, wobei dem Protein ein Signalpeptid vorangeht und wobei die DNA-Sequenz mit der nach dem Verfahren gemäß Anspruch 1(a) erhaltenen DNA-Sequenz nachweisbar ist.

3. Verfahren nach Anspruch 1, wobei die DNA-Sequenz
(e) die in den folgenden Sequenzdaten enthaltene DNA-Sequenz ist: 
GTTACTCTGT GTTGCAAATC CAACTTATAA AGACAACAAC CGCAAACTTT GTCTTGTGTC CATCAGATTG TTGTCCAAGC ACCCTCCCCC CCCCCTATCT TAGTCCTTCT TGTTGTCCAA ATG
MET
GCG CCC TCA GTT ACA CTG CCG TTG ACC ACG GCC ATC CTG GCC ATT GCC CGG CTC GTC GCC GCC CAG CAA CCG GGT ACC AGC ACC CCC GAG GTC CAT CCC AAG
ALA PRO SER VAL THR LEU PRO LEU THR THR ALA ILE LEU ALA ILE ALA ARG LEU VAL ALA ALA Gln Gln Pro Gly Thr Ser Thr Pro Glu Val His Pro Lys
TTG ACA ACC TAC AAG TGT ACA AAG TCC GGG GGG TGC GTG GCC CAG GAC ACC TCG GTG GTC CTT GAC TGG AAC TAC CGC TGG ATG CAC GAC GCA AAC TAC AAC
Leu Thr Thr Tyr Lys Cys Thr Lys Ser Gly Gly Cys Val Ala Gln Asp Thr Ser Val Val Leu Asp Trp Asn Tyr Arg Trp MET His Asp Ala Asn Tyr Asn
TCG TGC ACC GTC AAC GGC GGC GTC AAC ACC CTC TGC CCT GAC GAG GCG ACC TGT GGC AAG AAC TGC TTC ATC GAG GGC GTC GAC TAC GCC GCC TCG GGC
Ser Cys Thr Val Asn Gly Gly Val Asn Thr Leu Cys Pro Asp Glu Ala Thr Cys Gly Lys Asn Cys Phe Ile Glu Gly Val Asp Tyr Ala Ala Ser Gly
GTC ACG ACC TCG GGC AGC AGC CTC ACC ATG AAC CAG TAC ATG CCC AGC AGC TCT GGC GGC TAC AGC AGC GTC TCT CCT CGG CTG TAT CTC CTG GAC TCT GAC
Val Thr Thr Ser Gly Ser Ser Leu Thr MET Asn Gln Tyr MET Pro Ser Ser Ser Gly Gly Tyr Ser Ser Val Ser Pro Arg Leu Tyr Leu Leu Asp Ser Asp
GGT GAG TAC GTG ATG CTG AAG CTC AAC GGC CAG GAG CTG AGC TTC GAC GTC GAC CTC TCT GCT CTG CCG TGT GGA GAG AAC GGC TCG CTC TAC CTG TCT CAG
Gly Glu Tyr Val MET Leu Lys Leu Asn Gly Gln Glu Leu Ser Phe Asp Val Asp Leu Ser Ala Leu Pro Cys Gly Glu Asn Gly Ser Leu Tyr Leu Ser Gln
ATG GAC GAG AAC GGG GGC GCC AAC CAG TAT AAC ACG GCC GGT GCC AAC TAC GGG AGC GGC TAC TGC GAT GCT CAG TGC CCC GTC CAG ACA TGG AGG AAC GGC
MET Asp Glu Asn Gly Gly Ala Asn Gln Tyr Asn Thr Ala Gly Ala Asn Tyr Gly Ser Gly Tyr Cys Asp Ala Gln Cys Pro Val Gln Thr Trp Arg Asn Gly
ACC CTC AAC ACT AGC CAC CAG GGC TTC TGC TGC AAC GAG ATG GAT ATC CTG GAG GGC AAC TCG AGG GCG AAT GCC TTG ACC CCT CAC TCT TGC ACG GCC ACG
Thr Leu Asn Thr Ser His Gln Gly Phe Cys Cys Asn Glu MET Asp Ile Leu Glu Gly Asn Ser Arg Ala Asn Ala Leu Thr Pro His Ser Cys Thr Ala Thr
 
ggcgctctcg  cggttttcca  tgctggacat
AAC GGC TCG CCC TCG GGC AAC CTT
Asn Gly Ser Pro Ser Gly Asn Leu
TGC CCG TCC GCC TCA GCC TAC GGC
Cys Pro Ser Ala Ser Ala Tyr Gly
ATG AAC TGG CTC GAC AGC GGC AAC
MET Asn Trp Leu Asp Ser Gly Asn
AAC ATC CGC TGG GGA GAC ATT GGG
Asn Ile Arg Trp Gly Asp Ile Gly
ACT TCG AGC AGC CCG AGC TGC ACG
Thr Ser Ser Ser Pro Ser Cys Thr
AGC AAC GAC  tgttcgtctc  cccatgccct
Ser Asn Asp
C  GATAGAATGC  GCGGCAGCGCAG  GCGAGCTGCGTA
AAA AGg  tgagcctgat  gcccactacta  ccccttttcct
Lys Ser
TTC ACC ATC ATC ACC CAG TTC AAC ACG GAC
Phe Thr Ile Ile Thr Gln Phe Asn Thr Asp
GCC CAG CCC GGC GGC GAC ACC ATC TCG TCC
Ala Gln Pro Gly Gly Asp Thr Ile Ser Ser
TTC AGC ATT TGG AAC GAC AAC AGC CAG TAC
Phe Ser Ile Trp Asn Asp Asn Ser Gln Tyr
AAC AAC CCC AAC ATG CAC GTC GTC TTC TCC
Asn Asn Pro Asn Thr His Val Val Phe Ser
ACG TTT TCG ACT ACA CGG AGG AGC TCG ACG
Thr Phe Ser Thr Thr Arg Arg Ser Ser Thr
ACG TGC ACG TCG GGC ACT ACG TGC CAG TAT
Thr Cys Thr Ser Gly Thr Thr Cys Gln Tyr
TAGAGCGTTTG  ACTTGCCTCT  GGTCTGGTCCA  GACGGGGCA
TGC GGC TTC AAC CCC TAT GGC AGC GGC TAC
Cys Gly Phe Asn Pro Tyr Gly Ser Gly Thr
CCC GGA GAT ACC GTT GAC ACC TCC AAG ACC
Pro Gly Asp Thr Val Asp Thr Ser Lys Thr
TAC CAG CAA AAC GGC GTC GAC ATC CCC AGC
Tyr Gln Gln Asn Gly Val Asp Ile Pro Ser
AAG GCC CTG AGC AGC GGC ATG GTG CTC GTG
Lys Ala Leu Ser Ser Gly MET Val Leu Val
ACC GAG GGC AAC CCA TCC AAC ATC CTG GCC
Thr Glu Gly Asn Pro Ser Asn Ile Leu Ala
GCG CCC CCG CCC CCG CCT GCG TCC AGC ACG
Ala Pro Pro Pro Pro Pro Ala Ser Ser Thr
TGC GGT GGC ATT GGG TAC AGC GGG TGC AAG
Cys Gly Gly Ile Gly Tyr Ser Gly Cys Lys
aaccgc  taaaataca  gAC TAC TTG CAA TGC CTT
Tyr Tyr Ser Gln Cys Leu
GCC TGC GAC TCT GCC GGT
Ala Cys Asp Ser Ala Gly
ggttttccagC  TAC TAC GGC
Tyr Tyr Gly
GTG AGC ATC ACC CGC AAG
Val Ser Ile Thr Arg Lys
GGC CTC GCC ACC ATG GGC
Gly Leu Ala Thr MET Gly
GCC GGC CCC TGC AGC AGC
Ala Gly Pro Cys Ser Ser
TCT ACT ACG AAC TCG ACT
Ser Thr Thr Asn Ser Thr
CAG ACT CAC TGG GGG CAG
Gln Thr His Trp Gly Gln
gacgggcagt  gatttgagag  tgcct
 oder
(f) eine DNA-Sequenz ist, die eine der in Anspruch 1 (b) angegebenen Aminosäuresequenz im wesentlichen äquivalente Aminosäuresequenz codiert, wobei die Aminosäuresequenz ein Protein verkörpert, das in der Lage ist, Cellulose an internen glykosidischen Stellen zu spalten, wobei dem Protein ein Signalpeptid vorangeht, und wobei die DNA-Sequenz mit der DNA-Sequenz gemäß Anspruch 1 (b) nachweisbar ist.

4. Verfahren nach einem der Ansprüche 1 bis 3, das außerdem das Isolieren eines das Signalpeptid der Pilzcellulase codierenden Fragmentes der DNA-Sequenz in einem der Ansprüche 1 bis 3 umfaßt.

5. Verfahren nach einem der Ansprüche 1 bis 4, wobei die DNA-Sequenz frei von Introns ist.

6. Verfahren nach einem der Ansprüche 1, 2(d), 3(f) oder 4, wobei die DNA-Sequenz Introns enthält.

7. Verfahren nach einem der Ansprüche 1 bis 6, wobei die Pilzart eine Art von Trichoderma ist.

8. Verfahren nach Anspruch 7, wobei die Pilzart T. reesei ist.

9. Verfahren nach einem der Ansprüche 1 (a), 2, 4, 5 oder 6, wobei die Cellobiohydrolase die Cellobiohydrolase-I von T. reesei ist.

10. Verfahren zur Herstellung eines rekombinanten DNA-Moleküls, das das Inserieren einer DNA-Sequenz, die nach dem Verfahren nach einem der Ansprüche 1 bis 9 erhältlich ist, in ein Vektormolekül umfaßt.

11. Verfahren zur Herstellung eines Expressionsvektor zur Expression einer reifen Pilzcellulase in Hefe als Wirt, wobei das Verfahren das Inserieren der folgenden Elemente in ein Vektormolekül in einer 5'- nach -3'-Richtung umfaßt:
(a) eine Hefepromotor-Sequenz; und
(b) eine nach einem der Ansprüche 1 bis 3 oder 5 bis 9 hergestellte DNA-Sequenz, wobei die DNA-Sequenz mit der Promotorsequenz funktionell verknüpft ist.

12. Verfahren nach Anspruch 11, wobei der Expressionsvektor außerdem eine Hefe-Terminator-Sequenz, die funktionell mit dem 3'-Ende des codierenden Bereichs verbunden ist, enthält.

13. Verfahren nach Anspruch 11 oder 12, wobei die Promotorsequenz einen Hefe-Enolase-1-Promotor enthält.

14. Verfahren nach einem der Ansprüche 11 bis 13, wobei der Expressionsvektor außerdem zusätzliche mit dem Wirt kompatible Kontrollsequenzen umfaßt, die mit den DNA-Sequenzen in funktionsfähiger Weise verknüpft sind.

15. Verfahren zur Herstellung transformierter Hefezellen, das das Transformieren von Hefezellen mit einem Expressionsvektor umfaßt, wobei der Expressionsvektor durch ein Verfahren nach einem der Ansprüche 11 bis 14 erhältlich ist.

16. Verfahren zur Herstellung einer reifen, glykosylierten funktionell aktiven Pilzendoglucanase-I, das das Transformieren von Saccharomyces cerivisiae mit einem Hefeexpressionsvektor umfaßt, der nach einem Verfahren nach einem der Ansprüche 11 bis 14 hergestellt ist.

17. Verfahren zur Behandlung von Cellulose, umfassend:
(a) Transformieren von Hefe mit einem nach einem Verfahren nach einem der Ansprüche 11 bis 14 hergestellten Expressionsvektor, um die reife, glykosylierte, funktionell aktive Pilzcellulase zu produzieren; und
(b) die enzymatische Umsetzung der Cellulose mit der produzierten Cellulase.

18. Verfahren nach Anspruch 17, wobei die transformierte Hefe in der Lage ist, Cellulase zu sekretieren.

19. Verfahren nach Anspruch 17 oder 18, wobei die Umsetzung in Anwesenheit der Hefe ausgeführt wird.

20. Verfahren nach Anspruch 17 oder 18, das außerdem vor der Umsetzung das Abtrennen der produzierten Cellulase von der Hefe umfaßt.

21. Verfahren nach einem der Ansprüche 17 bis 20, wobei die hergestellte Cellulase eine Cellobiohydrolase ist, und die Umsetzung ferner eine Umsetzung der Cellulase mit einer β-Glucosidase und/oder einer Endoglucanase einschließt.

22. Verfahren nach einem der Ansprüche 17 bis 20, wobei die produzierte Cellulase eine Endoglucanase ist und die Umsetzung ferner eine Umsetzung der Cellulose mit einer Cellobiohydrolase und/oder einer β-Glucosidase einschließt.

23. Verfahren zur Hydrolyse von Cellulose, das eine Behandlung der Cellulose mit den Hefezellen umfaßt, die nach dem Verfahren nach Anspruch 15 erhalten werden.

24. Verfahren zur Herstellung einer reifen, funktionell aktiven Pilzcellulase, das die Schritte
(a) Züchten der nach dem Verfahren nach Anspruch 14 erhaltenen Hefezellen, unter geeigneten Bedingungen, die die Sekretion der Cellulase in das Medium erlauen; und
(b) Gewinnung der Cellulase aus dem Medium, umfaßt.
